(19) Europäisches Patentamt
European Patent Office
Office européen des brevets

(11) **EP 3 851 456 A1**

(12) **EUROPEAN PATENT APPLICATION**
published in accordance with Art. 153(4) EPC

(43) Date of publication:
**21.07.2021 Bulletin 2021/29**

(21) Application number: **19859106.7**

(22) Date of filing: **10.09.2019**

(51) Int Cl.:
*C07K 16/28* (2006.01)   *C12N 15/13* (2006.01)
*C12N 15/63* (2006.01)   *C12N 5/10* (2006.01)
*G01N 33/53* (2006.01)   *A61K 39/395* (2006.01)
*A61P 35/00* (2006.01)   *A61P 37/00* (2006.01)

(86) International application number:
**PCT/CN2019/105119**

(87) International publication number:
**WO 2020/052546 (19.03.2020 Gazette 2020/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **11.09.2018   CN 201811060341**

(71) Applicants:
• **Jiangsu Hengrui Medicine Co., Ltd.
Lianyungang, Jiangsu 222047 (CN)**
• **Shanghai Hengrui Pharmaceutical Co., Ltd.
Shanghai 200245 (CN)**

(72) Inventors:
• **YE, Xin
Shanghai 200245 (CN)**
• **SUN, Le
Shanghai 200245 (CN)**
• **SONG, Mingjuan
Shanghai 200245 (CN)**
• **FU, Beibei
Shanghai 200245 (CN)**
• **WANG, Xiaohua
Shanghai 200245 (CN)**
• **ZHANG, Lei
Shanghai 200245 (CN)**
• **TAO, Weikang
Shanghai 200245 (CN)**

(74) Representative: **Regimbeau
20, rue de Chazelles
75847 Paris Cedex 17 (FR)**

(54) **ANTI-CD38 ANTIBODY, ANTIGEN-BINDING FRAGMENT THEREOF, AND PHARMACEUTICAL USE**

(57)   The present application provides an anti-CD38 antibody, an antigen-binding fragment thereof, and pharmaceutical use. Specifically, the present application provides a murine-derived antibody, a chimeric antibody or a humanized antibody comprising a CDR region of the anti-CD38 antibdoy, a pharmaceutical composition comprising the anti-CD38 antibody or the antigen-binding fragment thereof, and an application thereof as a drug. In particular, the present application provides an application of a humanized anti-CD38 antibody in preparing a drug for treating a CD38 positive disease or disorder.

EP 3 851 456 A1

**Description**

**FIELD OF THE INVENTION**

The present disclosure belongs to the field of biotechnology. More specifically, the present disclosure relates to therapeutic uses of anti-CD38 antibodies and compositions thereof and methods for producing the antibody molecules.

**BACKGROUND OF THE INVENTION**

The descriptions herein only provide background information about the present disclosure, and do not necessarily constitute prior art.

Multiple myeloma (MM) is a malignant plasma cell disease, in which tumor cells are derived from plasma cells in bone marrow, whereas the plasma cells are cells at the final functional stage developed from B lymphocytes. Therefore, multiple myeloma can also be classified as B lymphocyte lymphoma.

Multiple myeloma is characterized by abnormal proliferation of bone marrow plasma cells, accompanied by excessive production of monoclonal immunoglobulin or light chain (M protein); whereas non-secretory MM that does not produce M protein in few patients. Multiple myeloma is often accompanied by multiple osteolytic damage, hypercalcemia, anemia, and kidney damage. Various bacterial infections are easily to be found due to the inhibition of the production of normal immunoglobulin.

The domestic morbidity in China is estimated to be 2 to 3 per 100,000 persons, and the male to female ratio is 1.6:1. Most patients are older than 40 years old, and most are older than 60 years old. At present, there are estimated to be about 70,000 patients in China, and there are about 80,000 new patients worldwide each year. Multiple myeloma accounts for about 10% of various hematopoietic tumors. The number of patients has a tendency to further increase with the increased aging.

At present, multiple myeloma is an incurable hematological malignant tumor. The currently main treatments include: bone marrow transplantation, multiple small molecule chemotherapies, especially protease inhibitors represented by carfilzomib, and immunomodulators represented by lenalidomide, they greatly prolong the survival of MM patients. However, the disease almost always recurs in the end, and the average survival after relapse is only about 9 months. Daratumumab (Dara) available from Janssen Company alone showed good results in patients with relapsed or refractory multiple myeloma (RRMM), so FDA regarded it as a breakthrough, and Daratumumab was approved for marketing in November 2015 for the treatment of RRMM patients. Currently, several clinical trials with Dara in combination with small molecules have been carried out. Dara in combination with small molecule drugs can greatly improve the clinical efficacy.

CD38 is a type II transmembrane multifunctional protein with an extracellular domain of 256 amino acids. In one aspect, CD38 has been indirectly demonstrated to play a role in lateral signaling through the ligand CD31, which can cause cell adhesion and play a role in lymphocyte activation and B cell differentiation. However, there is no direct biochemical evidence for this function. On the other hand, CD38 has functions as cyclase and hydrolase, and it can not only promote the conversion of NAD (Nicotinamide Adenine Dinucleotide) into cADPR (cyclic Adenosine Diphosphate Ribose) by the action of cyclase, but also promote the conversion of cADPR into ADPR (Adenosine Diphosphate Ribose) by the action of cADPR hydrolase. CD38 can regulate $Ca^{2+}$ flow, and it can also promote the production of adenosine in cells to suppress immunity. Changes in $Ca^{2+}$ flow may affect the secretion of insulin. The mice with CD38 gene being knocked out showed normal survival, but exhibited various symptoms such as decreased humoral immune response, decreased insulin, and heart/pulmonary muscle defects. CD38 in humans is expressed in hematopoietic cells, immune cells, and various normal tissues such as brain, pancreas, kidney, muscle, and the like. It was found highly expressed in prostate and thymus on RNA level, whereas the expression level was significantly increased in a variety of hematologic tumor cells, especially in multiple myeloma cells. The clinical data of Daratumumab also verified the efficacy and safety of CD38 antibody in the field of multiple myeloma, suggesting the potential value of CD38 target development. Various studies in literatures have shown that the mechanism underlying the treatment of MM with anti-CD38 antibodies mainly includes:

    1) ADCC, CDC, ADCP, mechanism mainly related to epitopes and IgG1-Fc;
    2) direct killing effect caused by apoptosis;
    3) affecting tumor cell survival by inhibiting CD38 enzyme activity;
    4) potential clearance for Treg cells expressing CD38.

The CD38 target has become a hot spot in the treatment of multiple myeloma. Another important CD38-targeting antibody is Isartuximab available from Sanofi, which has also been used in a number of clinical trials for single use and in combination with small molecule drugs. From the existing data, Isartuximab has shown the same efficacy and safety as Daratumumab. At present, antibodies such as MOR202 (Morphasys) and TAK-079 (Takeda) have entered the clinic, and there are also CD38/CD3 bispecific antibodies and CD38-CAR-T being in the preclinical research phase. Other patents related to antibodies against CD38 as target can be found in, for example, WO2006099875, WO2007042309, WO2008047242, WO2012092612, WO2016164656, WO2017149122, etc.

Currently, there is still an urgent need to continue to develop antibodies with high selectivity, high affinity and favorable efficacy.

## SUMMARY OF THE INVENTION

The present disclosure provides a series of CD38 antibodies with higher affinity, better anti-tumor activity *in vivo,* and favorable metabolic activity in *vivo.* Specifically, the present disclosure provides a monoclonal antibody or an antigen-binding fragment that specifically binds to human CD38.

In some embodiments, the present disclosure provides an anti-CD38 antibody or an antigen-binding fragment thereof, the antibody specifically binding to human CD38, and the antibody or the antigen-binding fragment thereof comprises CDRs as shown below:

(i) heavy chain HCDR1, HCDR2, HCDR3 as shown in SEQ ID Nos: 9, 10 and 11 respectively, or HCDR variants having 3, 2 or 1 amino acid(s) difference(s) when compared with HCDR1, HCDR2, and HCDR3 as shown in SEQ ID Nos: 9, 10, and 11 respectively; and light chain LCDR1, LCDR2, LCDR3 as shown in amino acid sequence SEQ ID Nos: 12, 13 and 14 respectively, or LCDR variants having 3, 2 or 1 amino acid(s) difference(s) when compared with LCDR1, LCDR2, LCDR3 as shown in SEQ ID Nos: 12, 13 and 14 respectively; or

(ii) heavy chain HCDR1, HCDR2, HCDR3 as shown in SEQ ID Nos: 15, 16 and 17 respectively, or HCDR variants having 3, 2 or 1 amino acid(s) difference(s) when compared with HCDR1, HCDR2, and HCDR3 as shown in SEQ ID Nos: 15, 16, and 17 respectively; and light chain LCDR1, LCDR2, LCDR3 as shown in amino acid sequence SEQ ID Nos: 18, 19 and 20 respectively, or LCDR variants having 3, 2 or 1 amino acid(s) difference(s) when compared with LCDR1, LCDR2, LCDR3 as shown in SEQ ID Nos: 18, 19 and 20 respectively; or

(iii) heavy chain HCDR1, HCDR2, HCDR3 as shown in SEQ ID Nos: 15, 21 and 17 respectively, or HCDR variants having 3, 2 or 1 amino acid(s) difference(s) when compared with HCDR1, HCDR2, and HCDR3 as shown in SEQ ID Nos: 15, 21, and 17 respectively; and light chain LCDR1, LCDR2, LCDR3 as shown in amino acid sequence SEQ ID Nos: 22, 19 and 23 respectively, or LCDR variants having 3, 2 or 1 amino acid(s) difference(s) when compared with LCDR1, LCDR2, LCDR3 as shown in SEQ ID Nos: 22, 19 and 23 respectively.

In some embodiments, the CDR variants having 3, 2 or 1 amino acid(s) difference(s) of the CDR (including 3 heavy chain CDRs and 3 light chain CDRs) of the anti-CD38 antibody or the antigen-binding fragment are CDR variants with 3, 2 or 1 amino acid difference(s) obtained by screening by affinity maturation methods.

In some embodiments, the affinity (KD) of the anti-CD38 antibody or the antigen-binding fragment thereof to human CD38 is less than $10^{-8}$ M, less than $10^{-9}$M, less than $10^{-10}$ M, or less than $10^{-11}$ M.

In some embodiments, the anti-CD38 antibody is a murine, a chimeric or a humanized antibody, preferable is a huminazied antibody.

In some embodiments, the antibody is a murine antibody or a chimeric antibody, and the amino acid sequence of the heavy chain variable region of the antibody is as shown in SEQ ID Nos: 3, 5, 7, or having at least 95%, 96%, 97%, 98%, 99% or 100% sequence identity to SEQ ID Nos: 3, 5, 7; and/or the amino acid sequence of the light chain variable region of the antibody is as shown in SEQ ID Nos: 4, 6, 8, or having at least 95%, 96%, 97%, 98%, 99% or 100% sequence identity to SEQ ID Nos: 4, 6, 8.

In some embodiments, the anti-CD38 antibody or the antigen-binding fragment thereof comprises a heavy chain variable region and a light chain variable region as shown below:

(a):

a heavy chain variable region, the amino acid sequence thereof is as shown in SEQ ID No: 3 or has at least 95%, 96%, 97%, 98%, 99%, or 100% sequence identity to SEQ ID No: 3, and
a light chain variable region, the amino acid sequence thereof is as shown in SEQ ID No: 4 or has at least 95%, 96%, 97%, 98%, 99% or 100% sequence identity to SEQ ID No: 4;

(b):

a heavy chain variable region, the amino acid sequence thereof is as shown in SEQ ID No: 5 or has at least 95%, 96%, 97%, 98%, 99%, or 100% sequence identity to SEQ ID No: 5, and
a light chain variable region, the amino acid sequence thereof is as shown in SEQ ID No: 6 or has at least 95%, 96%, 97%, 98%, 99% or 100% sequence identity to SEQ ID No: 6;

(c):

a heavy chain variable region, the amino acid sequence thereof is as shown in SEQ ID No: 7 or has at least 95%, 96%, 97%, 98%, 99%, or 100% sequence identity to SEQ ID No: 7, and
a light chain variable region, the amino acid sequence thereof is as shown in SEQ ID No: 8 or has at least 95%, 96%, 97%, 98%, 99% or 100% sequence identity to SEQ ID No: 8.

In some embodiments, the antibody is a humanized antibody, and the humanized antibody comprises framework (FR) regions or framework region variants derived from human germline, and the framework region variants have up to 10 (for example, 10, 9, 8, 7, 6, 5, 4, 3, 2, or 1) amino acid back-mutations on light chain framework regions and/or heavy chain framework regions of the human antibody, respectively. In some embodiments, the humanized antibody comprises any one selected from the following (d) to (f):

(d) a heavy chain variable region, wherein a heavy chain variable region comprising heavy chain HCDR1, HCDR2, HCDR3 and heavy chain framework region(s), wherein:

the amino acid sequence of the HCDR1 is as shown in SEQ ID No: 9 or has 3, 2 or 1 amino acid(s) difference(s) when compared with SEQ ID No: 9,
the amino acid sequence of the HCDR2 is as shown in SEQ ID No: 10 or has 3, 2 or 1 amino acid(s) difference(s) when compared with the sequence of SEQ ID No: 10,
the amino acid sequence of the HCDR3 is as shown in SEQ ID No: 11 or has 3, 2 or 1 amino acid(s) difference(s) when compared with the sequence of SEQ ID No: 11,
the heavy chain framework region(s) has/have one or more back-mutations selected from group consisting of 2F, 38K, 44S, 48I, 67A, 66K, 69L, 71V and 73Q; and/or
a light chain variable region, wherein a light chain variable region comprising light chain LCDR1, LCDR2, LCDR3 and light chain framework region(s), wherein:

the amino acid sequence of the LCDR1 is as shown in SEQ ID No: 12 or has 3, 2 or 1 amino acid(s) difference(s) when compared with SEQ ID No: 12,
the amino acid sequence of the LCDR2 is as shown in SEQ ID No: 13 or has 3, 2 or 1 amino acid(s) difference(s) when compared with the sequence of SEQ ID No: 13, the amino acid sequence of the LCDR3 is as shown in SEQ ID No: 14 or has 3, 2 or 1 amino acid(s) difference(s) when compared with the sequence of SEQ ID No: 14,
the light chain framework region(s) has/have one or more back-mutations selected from group consisting of 2F, 43S, 49K and 87F;

(e) a heavy chain variable region, wherein a heavy chain variable region comprising heavy chain HCDR1, HCDR2, HCDR3 and heavy chain framework region(s), wherein:

the amino acid sequence of the HCDR1 is as shown in SEQ ID No: 15 or has 3, 2 or 1 amino acid(s) difference(s) when compared with SEQ ID No: 15,
the amino acid sequence of the HCDR2 is as shown in SEQ ID No: 16 or has 3, 2 or 1 amino acid(s) difference(s) when compared with the sequence of SEQ ID No: 16,
the amino acid sequence of the HCDR3 is as shown in SEQ ID No: 17 or has 3, 2 or 1 amino acid(s) difference(s) when compared with the sequence of SEQ ID No: 17,
the heavy chain framework region(s) has/have one or more back-mutations selected from group consisting of 79F, 82A T, 91S and 76S; and/or
a light chain variable region, wherein a light chain variable region comprising light chain LCDR1, LCDR2, LCDR3 and light chain framework region(s), wherein:

the amino acid sequence of the LCDR1 is as shown in SEQ ID No: 18 or has 3, 2 or 1 amino acid(s) difference(s) when compared with SEQ ID No: 18,
the amino acid sequence of the LCDR2 is as shown in SEQ ID No: 19 or has 3, 2 or 1 amino acid(s) difference(s) when compared with the sequence of SEQ ID No: 19,
the amino acid sequence of the LCDR3 is as shown in SEQ ID No: 20 or has 3, 2 or 1 amino acid(s) difference(s) when compared with the sequence of SEQ ID No: 20,

the light chain framework region(s) has/have one or more back-mutations selected from group consisting of 581, 68R and 85T;

(f) a heavy chain variable region, wherein a heavy chain variable region comprising heavy chain HCDR1, HCDR2, HCDR3 and heavy chain framework region(s), wherein:

the amino acid sequence of the HCDR1 is as shown in SEQ ID No: 15 or has 3, 2 or 1 amino acid(s) difference(s) when compared with SEQ ID No: 15,
the amino acid sequence of the HCDR2 is as shown in SEQ ID No: 21 or has 3, 2 or 1 amino acid(s) difference(s) when compared with the sequence of SEQ ID No: 21,
the amino acid sequence of the HCDR3 is as shown in SEQ ID No: 17 or has 3, 2 or 1 amino acid(s) difference(s) when compared with the sequence of SEQ ID No: 17,
the heavy chain framework region(s) has/have one or more back-mutations selected from group consisting of 481, 77T and 82A T; and/or
a light chain variable region, wherein a light chain variable region comprising light chain LCDR1, LCDR2, LCDR3 and light chain framework region(s), wherein:

The amino acid sequence of the LCDR1 is as shown in SEQ ID No: 22 or has 3, 2 or 1 amino acid(s) difference(s) when compared with SEQ ID No: 22,
the amino acid sequence of the LCDR2 is as shown in SEQ ID No: 19 or has 3, 2 or 1 amino acid(s) difference(s) when compared with the sequence of SEQ ID No: 19, the amino acid sequence of the LCDR3 is as shown in SEQ ID No: 23 or has 3, 2 or 1 amino acid(s) difference(s) when compared with the sequence of SEQ ID No: 23,
the light chain framework region(s) has/have one or more back-mutations selected from group consisting of 4L, 9A, 22S, 581, 60A and 68R;
wherein, the back-mutation sites described above are numbered according to Kabat Numbering Criteria, and the mutation described above such as "2F" refers to that the amino acid at position 2 (numbered according to the Kabat Numbering Criteria) has been back-mutated to phenylalanine (Phe or F), "82A T" refers to that the amino acid at position 82A (numbered according to the Kabat Numbering Criteria) has been back-mutated to threonine (Thr or T).

In some embodiments, the antibody heavy chain FR regions are selected from the combination of human germline IGHV1-3*01 and hJH4.1 or have at least 95%, 96%, 97%, 98%, 99% or 100% sequence identity thereto.
In some embodiments, the light chain FR regions are selected from the combination of human germline IGKV3-11*01 and hJK4.1 or have at least 95%, 96%, 97%, 98%, 99% or 100% sequence identity thereto.
In some embodiments, the heavy chain FR regions are selected from the combination of human germline IGHV3-7*01 and hJH6.1 FR4 or have at least 95%, 96%, 97%, 98%, 99% or 100% sequence identity thereto.
In some embodiments, the light chain FR regions are selected from the combination of human germline IGKV4-1*01 and hJK4.1 or have at least 95%, 96%, 97%, 98%, 99% or 100% sequence identity thereto.
In some embodiments, the humanized antibody comprises a heavy chain variable region as shown in SEQ ID Nos: 24, 32, or 37 or a variant thereof; wherein the variant has 1-10 (for example, 10, 9, 8, 7, 6, 5, 4, 3, 2, or 1) amino acid mutations on the framework regions of the above heavy chain variable region. In some embodiments, the amino acid mutation is selected from any of the following:

(g) one or more amino acid back-mutation(s) selected from the group consisting of 2F, 38K, 44S, 481, 67A, 66K, 69L, 71V and 73Q on the framework regions of the heavy chain variable region as shown in SEQ ID No: 24;
(h) one or more amino acid back-mutation(s) selected from the the group consisting of 79F and 91S on the framework regions of the heavy chain variable region as shown in SEQ ID No: 32;
(i) back-mutation of 481 on the framework regions of the heavy chain variable region as shown in SEQ ID No: 37.

In some embodiments, the humanized antibody comprises:
the heavy chain variable region as shown in SEQ ID Nos: 26, 27, 28, 29, 34 or 39, or the heavy chain variable region having at least 95%, 96%, 97%, 98%, 99% or 100% sequence identity to SEQ ID Nos: 26, 27, 28, 29, 34 or 39.
In some embodiments, the humanized antibody comprises the light chain variable region as shown in SEQ ID Nos: 25, 33, or 38 or a variant thereof; the variant has 1-10 (e.g., 10, 9, 8, 7, 6, 5, 4, 3, 2 or 1) amino acid mutation(s) in the light chain variable region as shown in any of SEQ ID Nos: 25, 33, or 38.
In some embodiments, the amino acid mutation is selected from any one of the following (j) to (1):

(j) one or more amino acid back-mutation(s) selected from the the group consisting of 2F, 43 S, 49K and 87F on the framework regions of the light chain variable region as shown in SEQ ID No: 25;
(k) one or more amino acid back-mutation(s) selected from the the group consisting of 581, 68R and 85T on the framework regions of the light chain variable region as shown in SEQ ID No: 33;
(1) one or more amino acid back-mutation(s) selected from the the group consisting of 4L, 9A, 22S, 581, 60A and 68R on the framework regions of the light chain variable region as shown in SEQ ID No: 38;
wherein, the back-mutation sites are numbered according to Kabat numbering system.

In some embodiments, the humanized antibody comprises a light chain variable region as shown in SEQ ID Nos: 30, 31, 35, 36, 40, 41 or 42, or having at least 95%, 96%, 97%, 98%, 99%, or 100% sequence identity to SEQ ID Nos: 30, 31, 35, 36, 40, 41, or 42.

In some embodiments, the humanized antibody comprises:

(m) a heavy chain variable region as shown in SEQ ID Nos: 24, 26, 27, 28 or 29, and a light chain variable region as shown in SEQ ID No:25, 30 or 31; or
(n) a heavy chain variable region as shown in SEQ ID Nos: 32 or 34, and a light chain variable region as shown in SEQ ID No:33, 35 or 36; or
(o) a heavy chain variable region as shown in SEQ ID Nos: 37 or 39, and a light chain variable region as shown in SEQ ID No:38, 40, 41 or 42;

In some embodiments, the humanized antibody comprises:

(p) a heavy chain variable region as shown in SEQ ID No:26, and a light chain variable region as shown in SEQ ID No:30; or
(q) a heavy chain variable region as shown in SEQ ID No:32, and a light chain variable region as shown in SEQ ID No:33; or
(r) a heavy chain variable region as shown in SEQ ID No:37, and a light chain variable region as shown in SEQ ID No:38.

In some embodiments, the antibody further comprises a constant region; preferably, the antibody is a chimeric antibody or humanized antibody, the heavy chain constant region of which is derived from human antibody IgG1, IgG2, IgG3 or IgG4, or conventional variants of IgG1, IgG2, IgG3 or IgG4; the light chain constant region of which is derived from human antibody kappa, lambda chains or conventional variants thereof. In some specific embodiments, the amino acid sequence of the heavy chain constant region is as shown in SEQ ID Nos: 43 or 44, or has at least 95%, 96%, 97%, 98%, 99% or 100% sequence identity thereto; the amino acid sequence of the light chain constant region is as shown in SEQ ID Nos: 45 or has at least 95%, 96%, 97%, 98%, 99%, or 100% sequence identity thereto.

In some embodiments, the heavy chain is as shown in SEQ ID Nos: 46, 48, 49, 51, 52, or 54, or has at least 85%, 86%, 87%, 88%, 89%, 90%, 91%, 92%, 93%, 94%, 95%, 96%, 97%, 98%, 99%, or 100% sequence identity thereto; and/or
the light chain is as shown in SEQ ID Nos: 47, 50 or 53, or has at least 85%, 86%, 87%, 88%, 89%, 90%, 91%, 92%, 93%, 94%, 95%, 96%, 97%, 98%, 99% or 100% sequence identity thereto.

In some embodiments, the anti-CD38 antibody comprises:

(i) a heavy chain as shown in SEQ ID Nos: 46 or 48, and a light chain as shown in SEQ ID No: 47; or
(ii) a heavy chain as shown in SEQ ID Nos: 49 or 51, and a light chain as shown in SEQ ID No: 50; or
(iii) a heavy chain as shown in SEQ ID Nos: 52 or 54, and a light chain as shown in SEQ ID No: 53.

In some specific embodiments, the anti-CD38 antibody comprises:

(iv) a heavy chain as shown in SEQ ID No: 48 and a light chain as shown in SEQ ID No: 47; or
(v) a heavy chain as shown in SEQ ID No: 51 and a light chain as shown in SEQ ID No: 50; or
(vi) a heavy chain as shown in SEQ ID No: 54 and a light chain as shown in SEQ ID No: 53.

In some embodiments, the anti-CD38 antibody or the antigen-binding fragment thereof has enhanced ADCC activity, which is achieved by modifying the affinity of the Fc region of the antibody or the antigen-binding fragment thereof to FcγIIIa. For example, mutations F243L, R292P, Y300L and combinations thereof mentioned in Patent WO2008140603 of MacroGenics, mutations S239D, I332E or combination thereof on the IgG1 Fc region mentioned in Patent US20080260731 of Xencor, and other mutations capable of enhancing ADCC function disclosed in the art.

In some embodiments, the antigen-binding fragment is selected from the group consisting of Fab, Fab', F(ab')2, scFv, diabody, and dsFv.

The present disclosure also provides an anti-CD38 antibody competing with the antibody or the antigen-binding fragment thereof described above for binding to human CD38, or competing with the antibody or the antigen-binding fragment thereof described above for binding to the same epitope of CD38 antigen.

The present disclosure also provides a pharmaceutical composition, comprising a therapeutically effective amount of the anti-CD38 antibody or the antigen-binding fragment thereof described above, and one or more pharmaceutically acceptable carriers, diluents, buffers, or excipients. Preferably, the pharmaceutical composition may contain 0.01 to 99% by weight of the anti-CD38 antibody or the antigen-binding fragment thereof in unit dosage; or the pharmaceutical composition may contain, preferably 0.1-2000 mg, more preferably 1-1000 mg of the antibody or the antigen-binding fragment thereof in unit dosage.

The present disclosure also provides an isolated nucleic acid molecule, which encodes the anti-CD38 antibody or the antigen-binding fragment thereof described above.

The present disclosure also provides a vector, which comprises the nucleic acid molecule described above.

The present disclosure also provides a host cell transformed (or transduced or transfected) with the vector described above. Also discloses a host cell, which contains the vector described above. The host cell is selected from prokaryotic cell and eukaryotic cell. In some embodiments, the host cell does not include any human cell capable of developing into a complete individual, such as human embryonic stem cell, fertilized egg, and germ cell; preferably, the host cell is a eukaryotic cell, more preferably a mammalian cell, wherein the mammalian cell includes but not limited to CHO, 293, NSO, and a mammalian cell in which gene editing is performed to change the glycosylation modification of the antibody or antigen-binding fragment thereof, thereby modifying the ADCC function of the antibody or antigen-binding fragments thereof, for example, knocking out genes such as FUT8 or GnT-III; in some embodiments, the mammalian cell do not include human cell.

The present disclosure also provides a method for preparing the anti-CD38 antibody or the antigen-binding fragments thereof described above, the method comprises the steps of cultivating the host cells described above, and then recovering the anti-CD38 antibody or the antigen-binding fragments thereof; optionally including the step of purifying the anti-CD38 antibody or the antigen-binding fragment thereof.

The present disclosure also provides a method for detecting or measuring human CD38, comprising contacting the anti-CD38 antibody or the antigen-binding fragment thereof described above with a sample to be tested.

The present disclosure also provides a reagent for detecting or measuring human CD38, the reagent comprises the anti-CD38 antibody or the antigen-binding fragment thereof described above.

The present disclosure also provides a diagnostic agent for diseases related to human CD38, the diagnostic agent comprises the anti-CD38 antibody or the antigen-binding fragment thereof described above.

The present disclosure also provides a method for diagnosing diseases related to human CD38, comprising detecting or measuring human CD38 or CD38 positive cells using the anti-CD38 antibody or the antigen-binding fragment thereof described above.

The present disclosure also provides the use of the anti-CD38 antibody or the antigen-binding fragment thereof described above in the preparation of a diagnostic agent for diseases related to human CD38.

The present disclosure also provides a method for treating or preventing a disease, comprising administering to a subject a therapeutically effective amount or a prophylactically effective amount of the anti-CD38 antibody or the antigen-binding fragment thereof described above, or a pharmaceutical composition comprising the same, or the nucleic acid molecular described above.

In some embodiments, the disease or disorder is tumor or immune disease.

In some embodiments, the disease or disorder is CD38 positive disease or disorder.

In some embodiments, the disease or disorder described above is tumor.

In some embodiments, the tumor described above is selected from the group consisting of leukemia, B cell lymphoma, plasma cell malignant tumor, T/NK cell lymphoma and myeloma. In some embodiments, the leukemia is selected from the group consisting of acute lymphocytic leukemia, acute lymphoblastic leukemia, acute promyelocytic leukemia, chronic lymphocytic leukemia, acute and chronic myeloid leukemia. In some embodiments, the myeloma is selected from the group consisting of multiple myeloma, anterior medullary tumor, light chain amyloidosis, and the like. In some embodiments, the lymphoma is non-Hodgkin's lymphoma or Hodgkin's lymphoma. In some embodiments, the tumor is B cell lymphoma/leukemia, for example selected from the group consisting of: mature B cell tumor, precursor B cell lymphoblastic leukemia/lymphoma, B cell non-Hodgkin's lymphoma and B cell Hodgkin's lymphoma. In some embodiments, the tumor is selected from the group consisting of B cell chronic lymphocytic leukemia (CLL), small lymphocytic leukemia (SLL), B cell acute lymphocytic leukemia, B cell prelymphocytic leukemia, lymphoplasmacytoid lymphoma, mantle cell lymphoma (MCL), low-grade/intermediate/high-grade follicular lymphoma (FL), cutaneous follicular central lymphoma, marginal zone B cell lymphoma (including MALT type, lymph node MZBL type, spleen MZBL type), hairy cell leukemia, diffuse large B cell lymphoma, Burkitt lymphoma, plasma cell tumor/plasma cell myeloma, plasma cell leukemia, post-transplant lymphoproliferative disease, Waldenstrom macroglobulinemia, plasma cell leukemia, anaplastic large cell lymphoma (ALCL) and hairy cell lymphoma.

In some embodiments, the tumor is B cell lymphoma or multiple myeloma.

In some embodiments, the tumor is multiple myeloma.

In some embodiments, the disease or disorder described above is an immune disease, such as an immune disease involving B cells, plasma cells, monocytes, and T cells that express CD38.

In some embodiments, the immune disease includes but not limited to: rheumatoid arthritis, psoriasis, ankylosing spondylitis, joint psoriasis, dermatitis, systemic scleroderma and sclerosis, inflammatory bowel disease (IBD), Crohn's disease, ulcerative Colitis, respiratory distress syndrome, meningitis, encephalitis, gastritis, uveitis, glomerulonephritis, eczema, asthma, arteriosclerosis, leukocyte adhesion deficiency, Raynaud syndrome, Sjogren syndrome, juvenile diabetes, Reiter disease, Behcet disease, immune complex nephritis, IgA nephropathy, IgM polyneuropathy, immune-mediated thrombocytopenia symptom (e.g. acute idiopathic thrombocytopenic purpura, chronic idiopathic thrombocytopenic purpura), hemolytic anemia, myasthenia gravis, lupus nephritis, systemic lupus erythematosus, rheumatoid arthritis (RA), atopic dermatitis, pemphigus, Graves disease, Hashimoto's thyroiditis, Wegener's granulomatosis, Omenn syndrome, chronic renal failure, acute infectious mononucleosis, multiple sclerosis, HIV and herpes virus-related diseases, severe acute respiratory syndrome and chorioretinitis, graft versus host disease, and immune disease caused by virus infection (such as disease caused or mediated by B cells infected with Ebola virus (EBV)).

In some embodiments, the immune disease is selected from the group consisting of: rheumatoid arthritis, systemic lupus erythematosus, asthma, inflammatory bowel disease, multiple sclerosis, Crohn's disease, gastritis, Hashimoto's thyroiditis, ankylosing spondylitis and graft versus host disease. In some embodiments, the disease or disorder is rheumatoid arthritis.

The present disclosure further provides the use of the anti-CD38 antibody or the antigen-binding fragment thereof described above or the pharmaceutical composition or the nucleic acid molecule described above in the preparation of a medicament for the treatment or prevention of diseases or disorders.

In some embodiments, the disease or disorder is tumor or immune disease.

In some embodiments, the disease or disorder is CD38 positive disease or disorder.

In some embodiments, the disease or disorder described above may be tumor, for example, the disease is characterized by the presence of CD38-expressing tumor cells.

In some embodiments, the tumor described above is selected from the group consisting of leukemia, B cell lymphoma, plasma cell malignant tumor, T/NK cell lymphoma and myeloma.

In some embodiments, the leukemia is selected from the group consisting of acute lymphocytic leukemia, acute lymphoblastic leukemia, acute promyelocytic leukemia, chronic lymphocytic leukemia, acute and chronic myeloid leukemia.

In some embodiments, the myeloma is selected from the group consisting of multiple myeloma, anterior medullary tumor, and light chain amyloidosis.

In some embodiments, the lymphoma is non-Hodgkin's lymphoma or Hodgkin's lymphoma.

In some embodiments, the tumor described above is B cell lymphoma/leukemia, for example is selected from mature B cell tumors or precursor B cell lymphoblastic leukemia/lymphoma, or selected from B cell non-Hodgkin's lymphoma or B cell Hodgkin's lymphoma.

In some embodiments, the tumor described above is selected from the group consisting of: B cell chronic lymphocytic leukemia (CLL), small lymphocytic leukemia (SLL), B cell acute lymphocytic leukemia, B cell prelymphocytic leukemia, lymphoplasmacytoid lymphoma, mantle cell lymphoma (MCL), follicular lymphoma (including low-grade, intermediate or high-grade FL), cutaneous follicular central lymphoma, marginal zone B cell lymphoma (including MALT type, lymph node MZBL type, spleen MZBL type), hairy cell leukemia, diffuse large B cell lymphoma, Burkitt lymphoma, plasma cell tumor, plasma cell myeloma, plasma cell leukemia, post-transplant lymphoproliferative disease, Waldenstrom macroglobulinemia, plasma cell leukemia, anaplastic large cell lymphoma (ALCL) and hairy cell lymphoma.

In some embodiments, the tumor is B cell lymphoma or multiple myeloma.

In some embodiments, the tumor is multiple myeloma.

In some embodiments, the disease or disorder is an immune disease, such as an immune disease involving B cells, plasma cells, monocytes, and T cells that express CD38.

In some embodiments, the immune disease may be selected from the group consisting of rheumatoid arthritis, psoriasis, ankylosing spondylitis, joint psoriasis, dermatitis, systemic scleroderma and sclerosis, inflammatory bowel disease (IBD), Crohn's disease, ulcerative colitis, respiratory distress syndrome, meningitis, encephalitis, gastritis, uveitis, glomerulonephritis, eczema, asthma, arteriosclerosis, leukocyte adhesion deficiency, Raynaud syndrome, Sjogren syndrome, juvenile diabetes, Reiter disease, Behcet disease, immune complex nephritis, IgA nephropathy, IgM polyneuropathy, immune-mediated thrombocytopenia symptom (e.g. acute idiopathic thrombocytopenic purpura, chronic idiopathic thrombocytopenic purpura), hemolytic anemia, myasthenia gravis, lupus nephritis, systemic lupus erythematosus, rheumatoid arthritis (RA), atopic dermatitis, pemphigus, Graves disease, Hashimoto's thyroiditis, Wegener's granulomatosis, Omenn syndrome, chronic renal failure, acute infectious mononucleosis, multiple sclerosis, HIV and herpes virus-related diseases, severe acute respiratory syndrome, chorioretinitis, graft versus host disease, and immune disease caused by virus infection (such as disease caused or mediated by B cells infected with Ebola virus (EBV)). In some embodiments, the immune disease is selected from the group consisting of: rheumatoid arthritis, systemic lupus erythematosus, asthma, inflammatory bowel disease, multiple sclerosis, Crohn's disease, gastritis, Hashimoto's thyroiditis, ankylosing spondylitis and graft versus host disease. In some embodiments, the immune disease or disorder is rheumatoid arthritis.

The present disclosure further provides an anti-CD38 antibody or an antigen-binding fragment thereof, or a pharmaceutical composition, or a nucleic acid molecule, for use in the treatment or prevention of the diseases or disorders described above.

In some embodiments, the disease or disorder is tumor or immune disease; in some embodiments, the disease or disorder is CD38 positive disease or disorder.

In some embodiments, the disease or disorder described above is tumor. For example, the disease is characterized by the presence of CD38-expressing tumor cells. In some embodiments, the tumor is selected from the group consisting of leukemia, B cell lymphoma, plasma cell malignant tumor, T/NK cell lymphoma and myeloma. In some embodiments, the leukemia is selected from the group consisting of acute lymphocytic leukemia, acute lymphoblastic leukemia, acute promyelocytic leukemia, chronic lymphocytic leukemia, acute and chronic myeloid leukemia. In some embodiments, the myeloma is selected from the group consisting of multiple myeloma, anterior medullary tumor, and light chain amyloidosis. In some embodiments, the lymphoma is non-Hodgkin's lymphoma or Hodgkin's lymphoma. In some embodiments, the tumor is B cell lymphoma/leukemia, for example selected from the group consisting of: mature B cell tumor, precursor B cell lymphoblastic leukemia/lymphoma, B cell non-Hodgkin's lymphoma and B cell Hodgkin's lymphoma. In some embodiments, the tumor is selected from the group consisting of: B cell chronic lymphocytic leukemia (CLL), small lymphocytic leukemia (SLL), B cell acute lymphocytic leukemia, B cell prelymphocytic leukemia, lymphoplasmacytoid lymphoma, mantle cell lymphoma (MCL), follicular lymphoma (including low-grade, intermediate or high-grade FL), cutaneous follicular central lymphoma, marginal zone B cell lymphoma (including MALT type, lymph node MZBL type, spleen MZBL type), hairy cell leukemia, diffuse large B cell lymphoma, Burkitt lymphoma, plasma cell tumor, plasma cell myeloma, plasma cell leukemia, post-transplant lymphoproliferative disease, Waldenstrom macroglobulinemia, plasma cell leukemia, anaplastic large cell lymphoma (ALCL) and hairy cell lymphoma. In some embodiments, the tumor is B cell lymphoma or multiple myeloma. In some embodiments, the tumor is multiple myeloma.

In some embodiments, the disease or disorder described above is immune disease, for example, immune disease involving B cells, plasma cells, monocytes, and T cells that express CD38.

In some embodiments, the immune disease may be selected from the group consisting of rheumatoid arthritis, psoriasis, joint psoriasis, dermatitis, systemic scleroderma and sclerosis, inflammatory bowel disease (IBD), Crohn's disease, ulcerative colitis, respiratory distress syndrome, meningitis, encephalitis, uveitis, glomerulonephritis, eczema, asthma, arteriosclerosis, leukocyte adhesion deficiency, multiple sclerosis, Raynaud syndrome, Sjogren syndrome, juvenile diabetes, Reiter disease, Behcet disease, immune complex nephritis, IgA nephropathy, IgM polyneuropathy, immune-mediated thrombocytopenia symptom (e.g. acute idiopathic thrombocytopenic purpura, chronic idiopathic thrombocytopenic purpura), hemolytic anemia, myasthenia gravis, lupus nephritis, systemic lupus erythematosus, rheumatoid arthritis (RA), atopic dermatitis, pemphigus, Graves disease, Hashimoto's thyroiditis, Wegener's granulomatosis, Omenn syndrome, chronic renal failure, acute infectious mononucleosis, multiple sclerosis, HIV and herpes virus-related diseases, severe acute respiratory syndrome, chorioretinitis, and immune disease caused by virus infection (such as disease caused or mediated by B cells infected with Ebola virus (EBV)). In some embodiments, the immune disease is selected from the group consisting of rheumatoid arthritis, systemic lupus erythematosus, asthma, inflammatory bowel disease, multiple sclerosis, Crohn's disease, gastritis, Hashimoto's thyroiditis, ankylosing spondylitis and graft versus host disease. In some embodiments, the immune disease is rheumatoid arthritis.

The anti-CD38 antibodies or the antigen-binding fragments thereof of the present disclosure exhibit favorable efficiency in both biochemical tests and *in vivo* pharmacodynamic assays. In the test for detecting the affinity of antibody-to-antigen, the antibody of the present disclosure hu9E showed KD value of 1.31 nM to human CD38, hu11E showed KD value of 0.568 nM to human CD38, and hu160E showed KD value of 0.0585 nM to human CD38, whereas the control antibody showed KD value of 2.35 nM, suggesting that the antibodies of the present disclosure have higher affinity (Table 18).

In the test of *in vivo* inhibition of tumor, it was found that both antibodies hu11E and hu160E of the present disclosure can effectively inhibit tumor growth in mice. The tumor-inhibition rate of hu11E at a dosage of 1mpk was up to 93.14%, and hu160E exhibited tumor-inhibition rate of 70.02%, both were significantly higher than that of control antibody Dara (the tumor-inhibition rate was 56.83%) (Table 20).

In addition, the anti-CD38 monoclonal antibodies or the antigen-binding fragments thereof of the present disclosure have favorable metabolic kinetic properties in rats, and show a longer half-life and higher bioavailability.

**DESCRIPTION OF THE DRAWINGS**

Figure 1A: *In vitro* ADCP test results of CD38 antibodies on Molp-8 cells.
Figure 1B: *In vitro* ADCP test results of CD38 antibodies on Daudi cells.
Figure 2: Tumor-inhibition effects of CD38 antibodies in mice.

**DETAILED DESCRIPTION OF THE INVENTION**

**Terminology**

In order to make the present disclosure be more easily understood, certain technical and scientific terms are specifically defined below. Unless otherwise defined explicitly herein, all other technical and scientific terms used herein have the meaning commonly understood by those skilled in the art to which this disclosure pertains.

Three-letter codes and one-letter codes for amino acids used in the present disclosure are as described in J. biol. chem, 243, p3558 (1968).

As used herein, "antibody" refers to immunoglobulin, a four-peptide chain structure connected together by disulfide bond between two identical heavy chains and two identical light chains. Different immunoglobulin heavy chain constant regions exhibit different amino acid compositions and sequences, hence present different antigenicity. Accordingly, immunoglobulins can be divided into five types (or immunoglobulin isotypes), namely IgM, IgD, IgG, IgA and IgE, corresponding to heavy chain $\mu$, $\delta$, $\gamma$, $\alpha$ and $\epsilon$, respectively According to its amino acid composition of hinge region and the number and location of heavy chain disulfide bonds, the same type of Ig can further be divided into different subtypes, for example, IgG can be divided into IgG1, IgG2, IgG3 and IgG4. Light chain can be divided into $\kappa$ or $\lambda$ chain based on different constant regions. Each of five types of Ig has $\kappa$ or $\lambda$ chain.

About 110 amino acid sequences adjacent to the N-terminus of the antibody heavy and light chains are highly variable, known as variable region (Fv region); the rest of amino acid sequences close to the C-terminus are relatively stable, known as constant region. The variable region includes three hypervariable regions (HVRs) and four relatively conserved framework regions (FRs). The three hypervariable regions which determine the specificity of the antibody are also known as complementarity determining regions (CDRs). Each light chain variable region (VL or LCVR) and each heavy chain variable region (VH or HCVR) consists of three CDR regions and four FR regions, with sequential order from the amino terminus to carboxyl terminus as the following: FR1, CDR1, FR2, CDR2, FR3, CDR3, and FR4. The three CDR regions of the light chain refer to LCDR1, LCDR2, and LCDR3, and the three CDR regions of the heavy chain refer to HCDR1, HCDR2, and HCDR3.

The antibodies of the present disclosure include murine antibodies, chimeric antibodies, and humanized antibodies, preferably humanized antibodies.

As used herein, the term "murine antibody" refers to anti-human CD38 monoclonal antibodies prepared according to the knowledge and skills in the art. During the preparation, test subject is injected with CD38 antigen, and then a hybridoma expressing the antibody which possesses desired sequence or functional characteristics is isolated. In a preferable embodiment of the present disclosure, the murine CD38 antibody or antigen-binding fragment thereof further comprises a light chain constant region of murine $\kappa$, $\lambda$ chain or variant thereof, or further comprises a heavy chain constant region of murine IgG1, IgG2, IgG3 or variant thereof.

The term "chimeric antibody" is an antibody obtained by fusing the variable region of a species (such as murine) antibody with the constant region of another species (such as human) antibody, and the chimeric antibody can alleviate the murine antibody-induced immune response. To prepare a chimeric antibody, a hybridoma secreting specific murine monoclonal antibody is firstly prepared and variable region gene is cloned from the murine hybridoma; then constant region gene is cloned from human antibody according to the need; the murine variable region gene is connected to the human constant region gene to form a chimeric gene, which can be subsequently inserted into an expression vector. Finally the chimeric antibody molecule will be expressed in eukaryotic or prokaryotic system. In a specific embodiment of the present disclosure, the antibody light chain of the CD38 chimeric antibody further comprises a light chain constant region of a human kappa, lambda chain or a variant thereof. The antibody heavy chain of the CD38 chimeric antibody further comprises a heavy chain constant region of human IgG1, IgG2, IgG3, IgG4 or a conventional variant thereof, preferably comprises a heavy chain constant region of human IgG1, and more preferably comprises an IgG1 heavy chain constant region with amino acid mutations (such as E333A mutation) which enhance the CDC function.

The term "humanized antibody" refers to an antibody generated by grafting murine CDR sequences into human antibody variable region frameworks, i.e., an antibody produced in different types of human germline antibody framework sequences. Humanized antibody can avoid heterologous responses induced by chimeric antibody which carries a large number of murine protein components. Such framework sequences can be obtained from public DNA database or published references covering sequences of germline antibody gene. For example, germline DNA sequences of human heavy and light chain variable region genes can be found in "VBase" human germline sequence database (www.mrccpe.com.ac.uk/vbase), as well as in Kabat, EA, et al. 1991 Sequences of Proteins of Immunological Interest, 5th Ed. To avoid a decrease in activity caused by the decreased immunogenicity, the framework sequences in human antibody variable region may be subjected to minimal reverse mutations or back mutations to maintain the activity. The humanized antibodies of the present disclosure also include humanized antibodies obtained after CDR affinity maturation by phage display or yeast display.

The grafting of CDR can result in the decrease of the affinity of the antibody or antigen-binding fragment thereof to the antigen, due to the change of the framework residues responsible for the contact with the antigen. Such interactions may be resulted from highly somatic mutations. Therefore, it is necessary to graft the donor framework amino acids to the humanized antibody framework. The amino acid residues involved in antigen binding derived from non-human antibody or antigen-binding fragment thereof can be identified by checking the sequence and structure of animal monoclonal antibody variable region. The different amino acid residues between the donor CDR framework and the germlines may be considered to be related. If it is not possible to determine the most closely related germline, the sequence may be aligned against the consensus sequence shared among subtypes or against the murine sequence having high similarity percentage. Rare residues in framework are thought to be the result of a mutation in somatic cells, and play an important role in binding.

In some specific embodiments of the present disclosure, the antibody light chain of the CD38 humanized antibody further comprises a light chain constant region of a human kappa, lambda chain or a conventional variant thereof. The antibody heavy chain of the CD38 humanized antibody further comprises a heavy chain constant region of human IgG1, IgG2, IgG3, IgG4 or a conventional variant thereof, preferably comprises a heavy chain constant region of human IgG1, and more preferably comprises an IgG1 heavy chain constant region with amino acid mutations (such as E333A mutation) which enhance the CDC function.

The "conventional variants" of the human antibody heavy chain constant region and the antibody light chain constant region described in the present disclosure refer to the human heavy or light chain constant region variants disclosed in the prior art which do not change the structure and function of the antibody variable regions. Exemplary variants include IgG1, IgG2, IgG3 or IgG4 heavy chain constant region variants by site-directed modification and amino acid substitutions on the heavy chain constant region. The specific substitutions are, for example, YTE mutation, L234A and/or L235A mutation, S228P mutation, E333A mutation, and/or mutations resulting in a knob-into-hole structure (making the antibody heavy chain have a combination of knob-Fc and hole-Fc), etc. These mutations have been proven to confer the antibody with new properties, without affecting the function of the antibody variable region.

The term "back-mutation" refers to reversion of the amino acid residues in FR regions derived from human antibody back to the amino acid residues corresponding to those from the original antibody. In order to avoid the decrease in activity caused by the humanized antibody, usually the variable regions of the humanized antibodies can be subjected to minimal reverse mutations to maintain the activity of the antibody.

"Human antibody (HuMAb)", "antibody derived from human", "full human antibody" and "complete human antibody" may be used interchangeably, and may be antibodies derived from human or antibodies obtained from a genetically modified organism which has been "engineered" by any method known in the art to produce specific human antibodies in response to antigen stimulation. In some technologies, elements of human heavy and light chain loci are introduced into organism cell strains derived from embryonic stem cell lines, in which the endogenous heavy and light chain loci have been knocked out specifically. Transgenic organisms can synthesize human antibodies specific for human antigens, and the organisms can be used to produce hybridomas that secrete human antibodies. A human antibody can also be such antibody in which the heavy and light chains are encoded by nucleotide sequences derived from one or more DNA of human sources. Full human antibodies can also be constructed by gene or chromosome transfection methods and phage display technology, or constructed from B cells activated *in vitro,* all of methods are known in the art.

The terms "full-length antibody", "full antibody", "whole antibody" and "complete antibody" are used interchangeably herein and refer to an antibody in a substantially complete form, which is distinguished from the antigen-binding fragment defined below. The term specifically refers to antibodies that contain constant regions in the light and heavy chains.

In some embodiments, the full-length antibodies of the present disclosure include full-length antibodies formed by linking the light chain variable region to the light chain constant region, and the heavy chain variable region to the heavy chain constant region, as shown in Table 1 below. Those skilled in the art can select the light chain constant region and heavy chain constant region from various antibody sources according to need, such as human antibody-derived light chain constant region (or conventional variant thereof) and heavy chain constant region (or conventional variant thereof). At the same time, the combination of the light and heavy chain variable regions described in Table 1 can form a single chain antibody (scFv), Fab, or other forms of antigen-binding fragments comprising scFv or Fab.

Table 1: Combinations of light chain variable region and heavy chain variable region of anti-CD38 humanized antibody

| Combination of variable regions | Heavy chain variable region VH | Light chain variable region VL |
|---|---|---|
| h009-01V | h009 VH1 | h009 VL1 |
| h009-02V | h009 VH2 | h009 VL1 |
| h009-03V | h009 VH3 | h009 VL1 |
| h009-04V | h009 VH4 | h009 VL1 |
| h009-05V | h009 VH5 | h009 VL1 |
| h009-06V | h009 VH1 | h009 VL2 |
| h009-07V | h009 VH2 | h009 VL2 |
| h009-08V | h009 VH3 | h009 VL2 |
| h009-09V | h009 VH4 | h009 VL2 |
| h009-10V | h009 VH5 | h009 VL2 |
| h009-11 V | h009 VH1 | h009 VL3 |
| h009-12V | h009 VH2 | h009 VL3 |
| h009-13V | h009 VH3 | h009 VL3 |
| h009-14V | h009 VH4 | h009 VL3 |
| h009-15V | h009 VH5 | h009 VL3 |
| h011-01V | h011 VH1 | h011VL1 |
| h011-02V | h011 VH2 | h011VL2 |
| h011-03V | h011 VH1 | h011VL3 |
| h011-04V | h011 VH2 | h011VL1 |
| h011-05V | h011 VH1 | h011VL2 |
| h011-06V | h011 VH2 | h011VL3 |
| h160-01V | h160 VH1 | h160VL1 |
| h160-02V | h160 VH1 | h160 VL2 |
| h160-03V | h160 VH1 | h160 VL3 |
| h160-04V | h160 VH1 | h160 VL4 |
| h160-05V | h160 VH2 | h160 VL1 |
| h160-06V | h160 VH2 | h160 VL2 |
| h160-07V | h160 VH2 | h160 VL3 |
| h160-08V | h160 VH2 | h160 VL4 |

Note: For example, "h009-01V" represents the light/heavy chain variable region pair of h009-01V, in which the heavy chain variable region is h009VH1 (SEQ ID No: 24), and the light chain variable region is h009VL1 (SEQ ID No: 25), and so on.

The term "monoclonal antibody" refers to an antibody obtained from a population of substantially homogeneous antibodies, that is, the individual antibodies constituting the population are identical and/or bind to the same epitope, except for possible variant antibodies (for example, variants containing naturally occurring mutations or mutations produced during the manufacture of monoclonal antibody preparations, which are usually present in minimal amounts). Unlike polyclonal antibody preparations that usually contain different antibodies directed against different determinants (epitopes), each monoclonal antibody of a monoclonal antibody preparation (formulation) is directed against a single determinant on the antigen. Therefore, the prefix "monoclonal" indicates the characteristics of the antibody obtained from a substantially homogeneous antibody population, and should not be interpreted as antibody manufactured by particular method. For example, monoclonal antibodies used in accordance with the present disclosure can be prepared by various techniques, including but not limited to hybridoma methods, recombinant DNA methods, phage display methods, and methods by using transgenic animals containing all or part of human immunoglobulin loci. Such methods, and other exemplary methods for preparing monoclonal antibodies are described herein.

The term "antigen-binding fragment" or "functional fragment" of an antibody refers to one or more fragments of the antibody that retain the ability to specifically bind to an antigen (e.g., CD38). It has been shown that fragments of a full-length antibody can be used to achieve function of binding to a specific antigen. Examples of the binding fragments contained in the term "antigen-binding fragment" of an antibody include (i) Fab fragment, a monovalent fragment composed of VL, VH, CL and CH1 domains; (ii) F(ab')$_2$ fragment, a bivalent fragment formed by two Fab fragments connected by a disulfide bridge at the hinge region, (iii) Fv fragment composed of the VH and VL domains of one arm of the antibody; (iv) dsFv, a stable antigen-binding fragment formed by VH and VL via interchain disulfide bond(s); and (v) diabody, bispecific antibody and multispecific antibody containing fragments such as scFv, dsFv, and Fab. In addition, the VL domain and VH domain in a Fv fragment are encoded by two separate genes; however they can be linked by a synthetic linker by using recombinant methods, to generate a single protein chain in which a monovalent molecular is formed by pairing the VL and VH domain (referred to as single chain Fv (scFv); see, e.g., Bird et al. (1988) Science 242: 423-426; and Huston et al (1988) Proc. Natl. Acad. Sci USA85:5879-5883). Such single chain antibodies are also intended to be included in the term "antigen-binding fragment". Such antibodie fragments are obtained using conventional techniques known in the field, and screened for functional fragments by using the same method as that for an intact antibody. Antigen binding portions can be produced by recombinant DNA technology or by enzymatic or chemical digestion of an intact immunoglobulin. Antibodies can be in the form of different isotypes, e.g., IgG (e.g., IgG1, IgG2, IgG3 or IgG4 subtype), IgA1, IgA2, IgD, IgE or IgM antibody. In some embodiments, the antigen-binding fragments thereof of the present disclosure include Fab, F(ab')2, Fab', single-chain antibody (scFv), dimerized V region (diabody), V region stabilized by disulfide linkage (dsFv), and so on.

Fab is an antibody fragment obtained by treating an IgG antibody molecule with a papain (which cleaves the amino acid residue at position 224 of the H chain). The obtained fragment has a molecular weight of about 50,000 and has antigen binding activity, in which about a half of H chain at the N-terminal side and the entire L chain are bound together through disulfide bond(s).

Fab of the present disclosure can be produced by treating the monoclonal antibody of the present disclosure with papain. Further, the Fab can be produced by inserting DNA encoding Fab of the antibody into a prokaryotic expression vector or eukaryotic expression vector, and introducing the vector into a prokaryote or eukaryote to express the Fab.

"F(ab')2" refers to an antibody fragment with a molecular weight of about 100,000 and antigen-binding activity, which is obtained by digesting IgG by pepsin at the part downstreaming the two disulfide bonds in the hinge region. F(ab')2 contains two Fabs connected at the hinge region.

F(ab')2 of the present disclosure can be produced by treating the monoclonal antibody of the present disclosure with pepsin. Also, F(ab')2 can be produced by binding the Fab' described below via thioether bond or disulfide bond.

"Fab'" is an antibody fragment having a molecular weight of about 50,000 and having antigen binding activity, which is obtained by cleaving a disulfide bond at the hinge region of the F(ab')2 described above. The Fab' of the present disclosure can be produced by treating F(ab')2 of the present disclosure with a reducing agent such as dithiothreitol. Further, the Fab' can be produced by inserting DNA encoding Fab' of the antibody into a prokaryotic expression vector or eukaryotic expression vector and introducing the vector into a prokaryote or eukaryote to express the Fab'.

The term "single chain antibody", "single chain Fv" or "scFv" refers to a molecule comprising antibody heavy chain variable domain (or region; VH) connected to antibody light chain variable domain (or region; VL) by a linker. Such scFv molecules have general structure of $NH_2$-VL-linker-VH-COOH or $NH_2$-VH-linker-VL-COOH. A suitable linker in the prior art consists of repeated GGGGS amino acid sequence or variant thereof, for example, variant with 1-4 repeats (Holliger et al. (1993), Proc. Natl. Acad. Sci. USA 90:6444-6448). Other linkers that can be used in the present disclosure are described by Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56 and Roovers et al. (2001), Cancer Immunol.

The scFv of the present disclosure can be produced by the following steps: obtaining cDNAs encoding the VH and VL of the monoclonal antibody of the present disclosure, constructing a DNA encoding the scFv, inserting the DNA into a prokaryotic or eukaryotic expression vector, and then introducing the expression vector into a prokaryote or eukaryote to express the scFv.

"Diabody" is an antibody fragment wherein the scFv is dimerized, and it is an antibody fragment having divalent antigen binding activity. In the divalent antigen binding activity, the two antigens may be the same or different.

Bispecific and multispecific antibody refer to an antibody that can simultaneously bind to two or more antigens or antigenic determinants, including scFv or Fab fragments that can bind to CD38.

The diabody of the present disclosure can be produced by the following steps: obtaining cDNAs encoding VH and VL of the monoclonal antibody of the present disclosure, constructing a DNA encoding scFv to make the length of a linker peptide of 8 or less amino acid residues, inserting the DNA into a prokaryotic or eukaryotic expression vector, and then introducing the expression vector into a prokaryote or eukaryote to express the diabody.

"dsFv" is obtained by substituting one amino acid residue in each of VH and VL with a cysteine residue, and then connecting the substituted polypeptides via a disulfide bond between the two cysteine residues. The amino acid residues to be substituted with a cysteine residue can be selected based on three-dimensional structure prediction of the antibody in accordance with known methods (Protein Engineering, 7, 697 (1994)).

The dsFv of the present disclosure can be produced by the following steps: obtaining cDNAs encoding the VH and VL of the monoclonal antibody of the present disclosure, constructing a DNA encoding the dsFv, inserting the DNA into a prokaryotic or eukaryotic expression vector, and then introducing the expression vector into a prokaryote or eukaryote to express the dsFv.

As used herein, the term "framework (FR)" refers to a part of the variable domain (either VL or VH), which serves as a scaffold for the antigen-binding loops (CDRs) in the variable domain. Essentially, it is a variable domain without CDRs.

The term "amino acid difference" or "amino acid mutation" refers to the difference or mutation between a polypeptide and its variant, and refers to the amino acid change or mutation present in the protein or polypeptide variant when compared to the original protein or polypeptide, including 1, 2, 3 or more amino acid substitution(s), insertion(s) or deletion(s) on the basis of the original protein or polypeptide.

The term "complementarity determining region", "CDR" or "hypervariable region" refers to one of the six hypervariable regions present in the antibody variable domain that mainly contribute to antigen binding. Generally, there are three CDRs (HCDR1, HCDR2, HCDR3) in each heavy chain variable region, and three CDRs (LCDR1, LCDR2, LCDR3) in each light chain variable region. The amino acid sequence boundaries of CDRs can be determined by any well-known criteria, including the "Kabat" numbering criteria (see Kabat et al. (1991), "Sequences of Proteins of Immunological Interest", 5th edition, Public Health Service, National Institutes of Health, Bethesda, MD), "Chothia" numbering criteria (see Al-Lazikani et al., (1997) JMB 273:927-948) and ImMunoGenTics (IMGT) numbering criteria (Lefranc MP, Immunologist, 7, 132- 136 (1999); Lefranc, MP, etc., Dev. Comp. Immunol., 27, 55-77 (2003), and the like. For example, for the classical format, the Kabat criteria can be followed, the CDR amino acid residues in the heavy chain variable domain (VH) are numbered as 31-35 (HCDR1), 50-65 (HCDR2) and 95-102 (HCDR3); the CDR amino acid residues in the light chain variable domain (VL) are numbered as 24-34 (LCDR1), 50-56 (LCDR2), and 89-97 (LCDR3). Following the Chothia criteria, the CDR amino acid residues in VH are numbered as 26-32 (HCDR1), 52-56 (HCDR2) and 95-102 (HCDR3); and the amino acid residues in VL are numbered as 26-32 (LCDR1), 50-52 (LCDR2) and 91-96 (LCDR3). By combining both Kabat and Chothia to define CDR, CDRs are composed of amino acid residues 26-35 (HCDR1), 50-65 (HCDR2) and 95-102 (HCDR3) in the human VH and amino acid residues 24-34 (LCDR1), 50-56 (LCDR2) and 89-97 (LCDR3) in the human VL. Following IMGT criteria, the CDR amino acid residues in VH are roughly numbered as 26-35 (CDR1), 51-57 (CDR2) and 93-102 (CDR3), and the CDR amino acid residues in VL are roughly numbered as 27-32 (CDR1), 50-52 (CDR2) and 89-97 (CDR3). Following IMGT criteria, the CDR regions of an antibody can be determined using IMGT/DomainGap Align Program.

The term "epitope" or "antigenic determinant" refers to a site on an antigen to which an immunoglobulin or antibody specifically binds (e.g., a specific site on CD38 molecule). Epitopes typically include at least 3, 4, 5, 6, 7, 8, 9, 10, 11, 12, 13, 14 or 15 contiguous or non-contiguous amino acids in a unique spatial conformation. See, for example, Epitope Mapping Protocols in Methods in Molecular Biology, Vol. 66, G. E. Morris Ed. (1996).

The term "specifically bind to" or "selectively bind to" refers to the binding of an antibody to a predetermined epitope on an antigen. Typically, the antibody binds with an affinity (KD) of less than about $10^{-8}$M, for example, less than about $10^{-9}$ M, $10^{-10}$ M or $10^{-11}$ M or even less.

The term "KD" refers to the dissociation equilibrium constant for particular antibody-antigen interaction. Typically, the antibody of the present disclosure binds to CD38 with a dissociation equilibrium constant (KD) of less than about $10^{-7}$M, for example, less than about $10^{-8}$M, $10^{-9}$M or $10^{-10}$M or even less, for example, as determined by Surface Plasma Resonance (SPR) technology in BIACORE instrument.

When the term "competition" is used in the context of antigen-binding proteins (e.g., neutralizing antigen-binding proteins or neutralizing antibodies) that compete for the same epitope, it means that competition occurs between the antigen-binding proteins, which is determined by an assay wherein an antigen-binding protein to be tested (e.g., an antibody or antigen-binding fragment thereof) prevents or inhibits (e.g., reduces) the specific binding of a reference antigen-binding protein (e.g., a ligand or reference antibody) to a common antigen (e.g., a CD38 antigen or fragment thereof). Numerous types of competitive binding assays are available to determine whether an antigen-binding protein competes with another. These assays are, for example, solid phase direct or indirect radioimmunoassay (RIA), solid phase direct or indirect enzyme immunoassay (EIA), Sandwich competition assay (see, e.g., Stahli et al, 1983, Methods in Enzymology 9: 242-253); solid phase direct biotin-avidin EIA (see, e.g., Kirkland et al, 1986, J. Immunol. 137: 3614-3619; Cheung et al., 1990, Virology 176: 546-552), solid phase direct labeling assay, solid phase direct labeling sandwich assay (see, e.g., Harlow and Lane, 1988, Antibodies, A Laboratory Manual, Cold Spring Harbor Press); solid phase direct labeling RIA with 1-125 label (see, e.g., Morel et al, 1988, Molec. Immunol. 25: 7-15); and direct labeling RIA (Moldenhauer et al, 1990, Scand. J. Immunol. 32: 77-82). Typically, the assay involves the use of a purified antigen capable of binding to both an unlabeled test antigen-binding protein and a labeled reference antigen-binding protein (the antigen is on a solid surface or cell surface). Competitive inhibition is determined by measuring the amount of label bound to the solid surface or to the cell surface in the presence of the test antigen-binding protein. Usually, the test antigen-binding protein is present in excess. Antigen binding proteins identified by competitive assay (competitive antigen-binding protein) includes: antigen-binding proteins that bind to the same epitope as the reference antigen-binding protein; and antigen-binding proteins that bind to an epitope that is sufficiently close to the epitope to which the reference antigen-binding protein binds, where the two epitopes spatially interfere with each other, thereby interfering the binding. Additional details regarding methods for determining competitive binding are provided in the Examples herein. Typically, when a competitive antigen-binding protein is present in excess, it will inhibit (e.g., reduce) at least 40-45%, 45-50%, 50-55%, 55-60%, 60-65%, 65-70%, 70-75% or even more of the specific binding of the reference antigen-binding protein to the common antigen. In some cases, the binding is inhibited by at least 80-85%, 85-90%, 90-95%, 95-97% or even more.

As used herein, the term "nucleic acid molecule" refers to DNA molecules and RNA molecules. A nucleic acid molecule may be single-stranded or double-stranded, but preferably is double-stranded DNA. A nucleic acid is "operably linked" when it is placed into a functional relationship with another nucleic acid sequence. For instance, a promoter or enhancer is operably linked to a coding sequence, when it affects the transcription of the sequence.

The term "vector" or "expression vector" refers to a nucleic acid molecule capable of transporting another nucleic acid to which it has been linked. In one embodiment, the vector is a "plasmid," which refers to a circular double stranded DNA loop into which additional DNA segments may be ligated. In another embodiment, the vector is a viral vector, wherein additional DNA segments may be ligated into the viral genome. The vectors disclosed herein are capable of self-replicating in the host cell into which they are introduced (e.g., bacterial vectors having a bacterial replication origin and episomal mammalian vectors), or may be integrated into the genome of a host cell upon introduction into the host cell, and thereby are replicated along with the host genome (e.g., non-episomal mammalian vectors).

Methods for producing and purifying antibodies and antigen-binding fragments thereof are well known in the art, for example, A Laboratory Manual for Antibodies, Cold Spring Harbor, New York, chapters 5-8 and 15. For example, mice can be immunized with human CD38 or fragments thereof, and the resulting antibodies can then be renatured, purified, and sequenced for amino acid sequences by using conventional methods well known in the art. Antigen-binding fragments can also be prepared by conventional methods. The antibodies or the antigen-binding fragments thereof of the present disclosure are engineered to graft one or more human FR regions onto CDRs derived from non-human antibody. Human FR germline sequences can be obtained from ImMunoGeneTics (IMGT) via their website http://imgt.cines.fr, or from The Immunoglobulin Facts Book, 2001, ISBN 012441351, by aligning against IMGT human antibody variable germline gene database using MOE software.

The term "host cell" refers to a cell into which an expression vector has been introduced. Host cells may include microorganisms (such as bacteria), plants or animal cells. Bacteria susceptible to be transformed include members of the family *Enterobacteriaceae,* such as strains of *Escherichia coli* or *Salmonella;* the family *Bacillaceae* such as *Bacillus subtilis; Pneumococcus; Streptococcus* and *Haemophilus influenzae.* Suitable microorganisms include *Saccharomyces cerevisiae* and *Pichia pastoris.* Suitable animal host cell lines include CHO (Chinese Hamster Ovary Cell Line) and NS0 cells.

The engineered antibodies or the antigen-binding fragments thereof of the present disclosure may be prepared and purified using known methods. For example, cDNA sequences encoding a heavy chain and a light chain may be cloned and engineered into a GS expression vector. The vectors expressing recombinant immunoglobulin may then be stably transfected into CHO cells. As a more recommended method well known in the art, mammalian expression systems will result in glycosylation, typically at highly conserved N-terminal sites in the Fc region. Stable clones expressing an antibody specifically binding to human CD38 were obtained. Positive clones may be expanded in serum-free culture medium in bioreactors for antibody production. Culture medium, into which an antibody has been secreted, may be purified by conventional techniques. For example, purification may be performed on Protein A or G Sepharose FF column that has been modified with buffer. The nonspecific binding components are removed by washing. The bound antibody is eluted by pH gradient and antibody fragments are detected by SDS-PAGE, and then pooled. The antibodies may be filtered and concentrated using common techniques. Soluble mixtures and aggregates may be effectively removed by common techniques, such as size exclusion or ion exchange. The resulting product is sometimes needed to be frozen immediately, such as at -70°C, or lyophilized.

"Administration", "administering" or "treatment," as it applies to an animal, human, subject, cell, tissue, organ, or biological fluid, refers to contacting an exogenous pharmaceutical, therapeutic, diagnostic agent, or composition with the animal, human, subject, cell, tissue, organ, or biological fluid. "Administration", "administering" or "treatment" can refer, e.g., to therapeutic, pharmacokinetic, diagnostic, research, and experimental methods. Treatment of a cell involves contacting a reagent with the cell, as well as contacting a reagent with a fluid, where the fluid is in contact with the cell.

"Administration", "administering" or "treatment" also means *in vitro or ex vivo* treatments, e.g., of a cell, with a reagent, diagnostic, binding compound, or with another cell. "Treatment", as it applies to a human, veterinary, or research subject, refers to therapeutic treatment, prophylactic or preventative measures, to research and diagnostic applications.

"Treatment" means administering a therapeutic agent, such as a composition containing any of antibodies or the antigen-binding fragments thereof of the present disclosure, or a nucleic acid molecule encoding the antibody or the antigen-binding fragment thereof, internally or externally to a patient having one or more disease symptoms for which the therapeutic agent is known to have therapeutic effect. Typically, the agent is administered in an amount effectively to alleviate one or more disease symptoms in the patient or population to be treated, whether by inducing the regression of or inhibiting the progression of such symptom(s) by any clinically measurable degree. The amount of a therapeutic agent that is effective to alleviate any particular disease symptom (also referred to as the "therapeutically effective amount") may vary according to various factors such as the disease state, age, and body weight of the patient, and the ability of the drug to elicit a desired response in the patient. Whether a disease symptom has been alleviated can be assessed by any clinical measurement typically used by physicians or other skilled healthcare providers to assess the severity or progression status of that symptom. While one embodiment of the present disclosure (e.g., a treatment method or article of manufacture) may not be effective in alleviating each target disease symptom, it should alleviate the target disease symptom(s) in a statistically significant number of subjects as determined by any statistical test known in the art such as Student's t-test, chi-square test, U-test according to Mann and Whitney, Kruskal-Wallis test (H-test), Jonckheere-Terpstra-test and Wilcoxon-test.

"Conservative modification" or "conservative substitution or replacement" refers to substitution of amino acid(s) in a protein with other amino acid(s) having similar characteristics (e.g. charge, side-chain size, hydrophobicity/hydrophilicity, backbone conformation and rigidity, etc.), such that the changes can frequently be made without affecting the biological activity of the protein. Those skilled in the art recognize that, in general, single amino acid substitution in non-essential regions of a polypeptide does not substantially alter biological activity (see, e.g., Watson et al. (1987) Molecular Biology of the Gene, The Benjamin/Cummings Pub. Co., p. 224 (4th Ed.)). In addition, substitutions of structurally or functionally similar amino acids are less likely to disrupt biological activity. Exemplary conservative substitutions are set forth in Table 2 "Exemplary Amino Acid Conservative Substitutions" below.

Table 2. Exemplary amino acid conservative substitutions

| Original residue | Conservative substitution |
|---|---|
| Ala (A) | Gly; Ser |
| Arg(R) | Lys; His |
| Asn (N) | Gln; His; Asp |
| Asp (D) | Glu; Asn |
| Cys (C) | Ser; Ala; Val |
| Gln(Q) | Asn; Glu |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro(P) | Ala |
| Ser(S) | Thr |
| Thr(T) | Ser |
| Trp (W) | Tyr; Phe |
| Tyr (Y) | Trp; Phe |

(continued)

| Original residue | Conservative substitution |
|---|---|
| Val (V) | Ile; Leu |

"Effective amount" or "effective dosage" refers to the amount of the agent, compound, or pharmaceutical composition necessary to obtain any one or more beneficial or desired results. For prophylactic applications, beneficial or desired results include elimination or reduction of risk, reduction of severity, or delay of the onset of the disease, including the biochemical, histological, and behavioral symptoms of the condition, its complications, and intermediate pathological phenotypes during the development of the condition. For therapeutic applications, beneficial or desired results include clinical results, such as reduction of the incidence of various conditions associated with target antigen of the present disclosure or improvement of one or more symptoms of the condition, reduction of the dosage of other agents required to treat the condition, enhancement of the efficacy of another agent, and/or delay of the progression of the condition associated with the target antigen of the present disclosure in patients.

"Exogenous" refers to substances produced outside organisms, cells, or humans according to circumstances.

"Endogenous" refers to substances produced inside organisms, cells, or human bodies according to circumstances.

The "mutated sequence" mentioned in the present disclosure refers to the nucleotide sequence and amino acid sequence of varying percentage sequence identity to those of the present disclosure, which are obtained after modifying the nucleotide sequence and amino acid sequence of the present disclosure by appropriate substitution, insertion or deletion. The sequence identity described in the present disclosure may be at least 85%, 90% or 95%, non-limiting examples include 85%, 86%, 87%, 88%, 89%, 90%, 91%, 92%, 93%, 94%, 95%, 96%, 97%, 98%, 99%, 100%.

As used herein, "homology" or "identity" refers to sequence similarity between two polynucleotide sequences or between two polypeptide sequences. When a position in both of the two sequences to be compared is occupied by the same base or amino acid monomer subunit, e.g., when a position in each of two DNA molecules is occupied by the same base, then the molecules are known as homologous at that position. The percent of homology between two sequences is a function of the number of matching or homologous positions shared by the two sequences divided by the number of total positions to be compared and then multiplied by 100. For example, when two sequences are optimally aligned, if 6 out of 10 positions in the two sequences are matched or homologous, then the two sequences are 60% homologous; if 95 out of 100 positions in the two sequences re matched or homologous, then the two sequences are 95% homologous. Generally, the two sequences to be compared are subjected to alignment to give a maximum homology percentage. For example, the comparison can be performed by the BLAST algorithm, in which the parameters of the algorithm are selected to give the maximum match between each sequence over the entire length of each reference sequence. The following references refer to the BLAST algorithm frequently used for sequence analysis: BLAST algorithm (BLAST ALGORITHMS): Altschul, SF et al., (1990) J. Mol. Biol. 215:403-410; Gish, W. et al., (1993) Nature Genet. 3:266-272; Madden, TL et al., (1996) Meth. Enzymol. 266:131-141; Altschul, SF et al., (1997) Nucleic Acids Res. 25:3389-3402; Zhang, J. et al. (1997) Genome Res. 7:649-656. Other conventional BLAST algorithms such as those available from NCBI BLAST are also well known to those skilled in the art.

As used herein, the expressions "cell," "cell line," and "cell culture" are used interchangeably and all such designations include progeny. Thus, "transformant" and "transformed cell" include the primary subject cells and cultures derived therefrom regardless of the number of passages. It should be also understood that all progeny may not be precisely identical in DNA content, due to intended or non-intended mutations. Mutant progeny that have the same function or biological activity as screened in the originally transformed cells are included. Where different designations are intended to, it will be clearly understood from the context.

As used herein, "polymerase chain reaction" or "PCR" refers to a procedure or technique in which minute amounts of a specific portion of nucleic acid, RNA and/or DNA, are amplified as described in, e.g., U.S. Pat. No. 4,683,195. Generally, sequence information at the ends of or beyond the region of interest needs to be available, such that oligonucleotide primers can be designed; these primers will be identical to or similar to the sequence of complementay strand of the template to be amplified. The 5' terminal nucleotides of the two primers can be identical to the ends of the amplified material. PCR can be used to amplify specific RNA sequences, specific DNA sequences from total genome and cDNA transcribed from total cellular RNA, bacteriophage or plasmid sequences, etc. See generally Mullis et al. (1987) Cold Spring Harbor Symp. Ouant. Biol. 51:263; Erlich editor, (1989) PCR TECHNOLOGY (Stockton Press, NY). The PCR used in the present disclosure is considered to be one (but not the only) example of polymerase reaction methods for amplifying a nucleic acid sample to be tested. The method comprises the use of nucleic acid sequences known as primers together with nucleic acid polymerase to amplify or generate a specific portion of nucleic acid.

"Isolated" refers to a purified state, in which the designated molecule is substantially free of other biological molecules, such as nucleic acids, proteins, lipids, carbohydrates, or other materials, such as cell debris and growth medium. In general, the term "isolated" is not intended to mean the complete absence of these materials or the absence of water, buffers or salts, unless they are present in an amount that significantly interferes with the experimental or therapeutic use of the compound as described herein.

"Optional" or "optionally" means that the event or circumstance that follows may but does not necessarily occur, and the description will indicate the instances where the event or circumstance does or does not occur. For example, "optionally contains 1-3 antibody heavy chain variable regions" means the antibody heavy chain variable region with specific sequence can be, but need not be, present.

"Pharmaceutical composition" refers to a mixture containing one or more antibodies or the antigen-binding fragments thereof according to the present disclosure and other chemical components, such as physiologically/pharmaceutically acceptable carriers or excipients. The pharmaceutical composition aims at promoting the administration to an organism, facilitating the absorption of the active ingredient and thereby exerting a biological effect.

The term "pharmaceutically acceptable carrier" refers to any inactive substance suitable for use in a formulation for the delivery of antibodies or antigen-binding fragments. The carrier can be an anti-adhesive agent, binder, coating agent, disintegrating agent, filler or diluent, preservative (such as antioxidant, antibacterial or antifungal agent), sweetener, absorption delaying agent, wetting agent, emulsifier, buffer, and the like. Examples of suitable pharmaceutically acceptable carriers include water, ethanol, polyols (such as glycerol, propylene glycol, polyethylene glycol, and the like) dextrose, vegetable oil (such as olive oil), saline, buffer, buffered saline, and isotonic agent, such as sugars, polyols, sorbitol and sodium chloride.

"CD38-positive disease or disorder" is a disease or disorder in which CD38-expressing cells are present. Without limitation, regarding the immune diseases involving CD38-expressing B cells, plasma cells, monocytes and T cells, one characteristics of the disease is, for example, a tumor disease with CD38-expressing tumor cells, such as CD38-expressing leukemia, B cell lymphoma, plasma cell malignant tumor, T/NK cell lymphoma and myeloma. In some embodiments of the present disclosure, the leukemia is selected from the group consisting of acute lymphocytic leukemia, acute lymphoblastic leukemia, acute promyelocytic leukemia, chronic lymphocytic leukemia, acute and chronic myeloid leukemia. In some embodiments, the myeloma is selected from the group consisting of multiple myeloma, anterior medullary tumor, and light chain amyloidosis. In some embodiments, the lymphoma is non-Hodgkin's lymphoma or Hodgkin's lymphoma. In some embodiments, the tumor may be selected from B cell lymphoma/leukemia, including but not limited to: precursor B cell lymphoblastic leukemia/lymphoma, B cell non-Hodgkin's lymphoma or B cell Hodgkin Lymphoma, mature B cell tumor. In some embodiments, the tumor is selected from the group consisting of: B cell chronic lymphocytic leukemia (CLL), small lymphocytic leukemia (SLL), B cell acute lymphocytic leukemia, B cell prelymphocytic leukemia, lymphoplasmacytoid lymphoma, mantle cell lymphoma (MCL), follicular lymphoma (including low-grade, intermediate or high-grade FL), cutaneous follicular central lymphoma, marginal zone B cell lymphoma (including MALT type, lymph node MZBL type, spleen MZBL type), hairy cell leukemia, diffuse large B cell lymphoma, Burkitt lymphoma, plasma cell tumor, plasma cell myeloma, plasma cell leukemia, post-transplant lymphoproliferative disease, Waldenstrom macroglobulinemia, plasma cell leukemia, anaplastic large cell lymphoma (ALCL) and hairy cell lymphoma. In some embodiments, the tumor is multiple myeloma. In some embodiments, the immune disease may be selected from the group consisting of rheumatoid arthritis, psoriasis, ankylosing spondylitis, joint psoriasis, dermatitis, systemic scleroderma and sclerosis, inflammatory bowel disease (IBD), Crohn's disease, ulcerative colitis, respiratory distress syndrome, meningitis, encephalitis, gastritis, uveitis, glomerulonephritis, eczema, asthma, arteriosclerosis, leukocyte adhesion deficiency, Raynaud syndrome, Sjogren syndrome, juvenile diabetes, Reiter disease, Behcet disease, immune complex nephritis, IgA nephropathy, IgM polyneuropathy, immune-mediated thrombocytopenia symptom (e.g. acute idiopathic thrombocytopenic purpura, chronic idiopathic thrombocytopenic purpura), hemolytic anemia, myasthenia gravis, lupus nephritis, systemic lupus erythematosus, rheumatoid arthritis (RA), atopic dermatitis, pemphigus, Graves disease, Hashimoto's thyroiditis, Wegener's granulomatosis, Omenn syndrome, chronic renal failure, acute infectious mononucleosis, multiple sclerosis, HIV and herpes virus-related diseases, severe acute respiratory syndrome, chorioretinitis, graft versus host disease, and immune disease caused by virus infection (such as disease caused or mediated by B cells infected with Ebola virus (EBV)). In some embodiments, the immune disease is selected from the group consisting of rheumatoid arthritis, systemic lupus erythematosus, asthma, inflammatory bowel disease, multiple sclerosis, Crohn's disease, gastritis, Hashimoto's thyroiditis, ankylosing spondylitis and graft versus host disease. In some embodiments, the immune disease is rheumatoid arthritis.

In addition, the present disclosure provides agents for the treatment or prevention of diseases related to target antigen (e.g. CD38) positive cells. The agent contains the anti-CD38 antibody or the antigen-binding fragment thereof of the present disclosure as an active ingredient and a therapeutically or prophylactically effective amount of the agent can be administered to a subject in need for the treatment or prevention of CD38-positive diseases. The anti-CD38 antibodies or the antigen-binding fragments thereof can inhibit disease-related activities induced by CD38 or eliminate or reduce the number of CD38 expressing cells. The therapeutically or prophylactically effective amount of the composition emprises 0.1-3000 mg (preferably 0.1-2000 mg, more preferably 1-1000 mg) of the anti-CD38 antibody or the antigen-binding fragment thereof described above, in a unit dosage.

In addition, the present disclosure relates to methods for immunodetection or determination of target antigens (for example, CD38), reagents for immunodetection or determination of target antigens (for example, CD38), methods for immunodetection or determination of cells expressing target antigens (for example, CD38), and the diagnostic agents for diagnosing diseases associated with target antigen (for example, CD38)-positive cells, comprising the antibody or the antibody fragment of the present disclosure that specifically recognizes and binds to the target antigen (for example, human CD38), as an active ingredient.

In the present disclosure, the method for detecting or measuring the amount of the target antigen (e.g. CD38) may be any known method. For example, it includes immunoassay or immunodetection method.

The immunoassay or immunodetection method is a method of detecting or measuring the amount of an antibody or antigen with a labeled antigen or antibody. Examples of immunoassay or immunodetection methods include immunomethod using antibody labeled with radioactive substance (RIA), enzyme immunoassay (EIA or ELISA), fluorescence immunoassay (FIA), luminescence immunoassay, western blotting, physicochemical method, and the like.

The diseases related to CD38-positive cells described above can be diagnosed by detecting or measuring CD38-expressing cells using the antibodies or the antibody fragments thereof of the present disclosure.

Cells expressing the polypeptide can be detected by the known immunodetection methods, preferably by immuno-precipitation, fluorescent cell staining, immunohistochemistry staining, and the like. In addition, the method, such as a staining method of fluorescent antibody using FMAT8100HTS system (Applied Biosystem), can be used.

In the present disclosure, samples to be detected or measured for the target antigen (e.g. CD38) are not particularly limited, as long as they are possible to contain cells expressing the target antigen (e.g. CD38), such as tissue cells, blood, plasma, serum, pancreatic secretion, urine, feces, tissue fluid or culture medium.

Dependent on the required diagnostic method, the diagnostic agent containing the monoclonal antibody or antibody fragment thereof of the present disclosure may also contain reagents for performing an antigen-antibody reaction or reagents for detecting the reaction. The reagents for performing an antigen-antibody reaction include buffers, salts and the like. The reagents for detection include agents commonly used in immunoassay or immunodetection methods, for example, a labeled secondary antibody that recognizes the monoclonal antibody, antibody fragment or conjugate thereof, and a substrate corresponding to the label.

The details of one or more embodiments of the present disclosure are set forth in the description above. The preferred methods and materials are described below, although any method and material similar or identical to those described herein can be used in the practice or testing of the present disclosure. Through the specification and claims, other features, purposes and advantages of the present disclosure will become apparent.

In the specification and claims, the singular form also refers to its plural counterparts, unless the context clearly dictates otherwise. Unless otherwise defined explicitly herein, all technical and scientific terms used herein have the meaning commonly understood by those skilled in the art to which this disclosure pertains. All patents and publications cited in the specification are incorporated by reference. The following examples are provided to more fully illustrate the preferred embodiments of the present disclosure. These examples should not be construed as limiting the scope of the present disclosure in any way, and the scope of the present disclosure is defined by the claims.

**Examples and Test Examples**

The following examples and test examples are provided to further describe the present disclosure, but are not intended to limit the scope of the disclosure. Experimental methods for which the specific conditions are not indicated in the examples and test examples of the present disclosure are generally carried out according to conventional conditions, such as Sambrook et al., Antibodies Laboratory Manual, Molecular Cloning, by Cold Spring Harbor Laboratory; or according to the conditions recommended by the manufacturer. Reagents for which the sources are not specifically indicated are commercially available reagents.

**Example 1. Preparation of CD38 antigen**

The amino acid sequences of the antigens and proteins for detection used in the present disclosure were designed using UniProt ADP-Ribocycliase/Cyclic ADP-Ribohydrolase1 (human CD38 protein, Uniprot No.: P28907) as CD38 template, optionally, various tags such as His tag or Fc were fused onto CD38 protein. The antigens and proteins for detection used in the present disclosure were obtained by a process comprising: cloning into pTT5 vector (Biovector, Cat#: 102762) or pTargeT vector (Promega, A1410) respectively, transiently expressing in 293 cells or stably expressing in CHO-S cells, and purification.

Fusion protein of CD38 extracellular domain and mouse IgG2a-Fc: CD38-ECD-mFc, used as an immunogen;

VPRWRQQWSGPGTTKRFPETVLARCVKYTEIHPEMRHVDCQSVWDAFKGAFIS
KHPCNITEEDYQPLMKLGTQTVPCNKILLWSRIKDLAHQFTQVQRDMFTLEDTL
LGYLADDLTWCGEFNTSKINYQSCPDWRKDCSNNPVSVFWKTVSRRFAEAAC
DVVHVMLNGSRSKIFDKNSTFGSVEVHNLQPEKVQTLEAWVIHGGREDSRDLC
QDPTIKELESIISKRNIQFSCKNIYRPDKFLQCVKNPEDSSCTSEIEPRGPTIKPCPP
CKCPAPNLLGGPSVFIFPPKIKDVLMISLSPIVTCVVVDVSEDDPDVQISWFVNN
VEVHTAQTQTHREDYNSTLRVVSALPIQHQDWMSGKEFKCKVNNKDLPAPIER
TISKPKGSVRAPQVYVLPPPEEEMTKKQVTLTCMVTDFMPEDIYVEWTNNGKT
ELNYKNTEPVLDSDGSYFMYSKLRVEKKNWVERNSYSCSVVHEGLHNHHTTK
SFSRTPGK (SEQ ID No: 1)

Note: The underlined part represents mouse IgG2a-Fc part;
Fusion protein of CD38 extracellular domain and human IgG1Fc: CD38-ECD-Fc, used as an immunogen;

VPRWRQQWSGPGTTKRFPETVLARCVKYTEIHPEMRHVDCQSVWDAFKGAFIS
KHPCNITEEDYQPLMKLGTQTVPCNKILLWSRIKDLAHQFTQVQRDMFTLEDTL
LGYLADDLTWCGEFNTSKINYQSCPDWRKDCSNNPVSVFWKTVSRRFAEAAC
DVVHVMLNGSRSKIFDKNSTFGSVEVHNLQPEKVQTLEAWVIHGGREDSRDLC
QDPTIKELESIISKRNIQFSCKNIYRPDKFLQCVKNPEDSSCTSEIEPKSSDKTHTC
PPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK (SEQ ID No: 2)

Note: The underlined part represents human IgG1-Fc part;
Fusion protein of CD38 extracellular domain with His tag: CD38-ECD-His, for use as an immunogen or detection reagent:

VPRWRQQWSGPGTTKRFPETVLARCVKYTEIHPEMRHVDCQSVWDAFKGAFIS
KHPCNITEEDYQPLMKLGTQTVPCNKILLWSRIKDLAHQFTQVQRDMFTLEDTL
LGYLADDLTWCGEFNTSKINYQSCPDWRKDCSNNPVSVFWKTVSRRFAEAAC
DVVHVMLNGSRSKIFDKNSTFGSVEVHNLQPEKVQTLEAWVIHGGREDSRDLC
QDPTIKELESIISKRNIQFSCKNIYRPDKFLQCVKNPEDSSCTSEIHHHHHH（SEQ
ID No: 57）

Note: The underlined part represents 6×His tag.

**Example 2. Purification of CD38-related recombinant protein**

1. Steps for purifying the recombinant protein with His tag:

The sample of cell expression supernatant was centrifuged at high speed to remove impurities, the buffer was substituted for PBS, and imidazole was added to a final concentration of 5mM. The nickel column was equilibrated with PBS solution containing 5mM imidazole, and washed with 2-5 times column volume. The substituted cell supernatant sample was applied onto Ni Sepharose excel column (GE, 17-3712-02). The column was washed with PBS solution containing 5 mM imidazole until the A280 reading dropped to the baseline. The chromatography column was rinsed with PBS+10mM imidazole to remove non-specifically bound protein impurities, and the effluent was collected. The target protein was eluted with PBS solution containing 300 mM imidazole, and the elution peak was collected. The collected eluate was concentrated and further purified by gel chromatography Superdex200 (GE, 28-9893-35) with PBS as the mobile phase. The aggregate peak was removed and the main peak was collected. The resulting protein was identified by electrophoresis, peptide map and LC-MS, and the confirmed proteins were aliquoted for use. His-tagged CD38-ECD-His (SEQ ID No: 57) was obtained, for use as an immunogen for preparing the antibodies of the present disclosure or as detection reagent. CD38-ECD-His was coupled to KLH by *in vitro* chemistry and was used as immunogen to stimulate mouse immunity.

2. Steps for purifying CD38-ECD-Fc fusion protein:

The sample of cell expression supernatant was centrifuged at high speed to remove impurities, and the supernatant was subjected to MabSelect Sure (GE, 17-5438-01) affinity chromatography. The MabSelect Sure column was regenerated first with 0.1M NaOH, washed with pure water and then equilibrated with PBS, after the supernatant was bound, PBS was used to wash until the A280 value dropped to the baseline. The target protein was eluted with 0.1M acetic acid buffer, pH3.5, and neutralized with 1M Tris-HCl. The eluted sample was properly concentrated, and then was further purified by PBS-equilibrated gel chromatography Superdex200 (GE, 28-9893-35), several tubes of the target proteins were pooled and concentrated to an appropriate concentration. This method was used to purify CD38-ECD-Fc (SEQ ID No: 2) fusion protein. It can also be used to purify the humanized antibody proteins in the present disclosure.

**Example 3. Obtaining and preparation of anti-human CD38 hybridoma monoclonal antibody**

1. Immunization

Anti-human CD38 monoclonal antibodies were produced by immunizing mice. SJL white mice, female, 4-6 weeks old were used for experiment (Beijing Charles River Experimental Animal Technology Co., Ltd., animal production license number: SCXK (Beijing) 2012-0001).

Feeding environment: SPF grade. After the mice were purchased, they were adapted to the laboratory environment for 1 week, 12/12 hours light/dark cycle, at temperature of 20 to 25°C; humidity of 40 to 60%. The mice that have adapted to the environment were immunized according to various protocols, 3-5 mice in each group.

The immune antigen can be CD38-ECD-His, CD38-ECD-Fc, CD38-FL-CHOS (CHOS cells transfected with human full-length of CD38), and the like. The immunization was performed with either a single reagent in combination with different immune adjuvants, or with different types of immunogens for purpose of cross-immunization The immunized site was either intraperitoneal or subcutaneous on the back, alternatively, immunization was performed alternatively on both sites. Exemplary immunization method was, for example, immunization with Titermax (Sigma Lot Num: T2684) or alum (Thremo Lot Num: 77161). The ratio of antigen to adjuvant (titermax) was 1:1, and the ratio of antigen to adjuvant (alum) was 4:1, 25-50$\mu$g or $1\times10^7$ cells/mouse (primary immunization), 25-50$\mu$g or $1\times10^7$ cells/mouse (booster immunization). On day 0, the antigen was injected intraperitoneally (IP) or subcutaneously (SC), and the immunization was repeated every two weeks after the primary immunization. Blood samples were collected every three weeks, and the antibody titer in mouse serum was determined by ELISA method. After 8 to 12 immunizations, mice with a high serum antibody titer reaching to the plateau were selected for splenocyte fusion. Three days before the splenocyte fusion, antigen solution prepared with saline was intraperitoneally injected (IP), with 25-50 $\mu$g/mouse or $1\times10^7$ cells/mouse, for booster immunization.

2. Cell Fusion

Mice with a high serum antibody titer (see Test Example 1, ELISA method for CD38-binding) reaching to the plateau were selected for splenocyte fusion, and the selected mice were subjected to booster immunization 3 days before fusion. Hybridoma cells were obtained by fusing splenic lymphocytes with myeloma Sp2/0 cells (ATCC® CRL-8287™) using an optimized PEG-mediated fusion procedure. The fused hybridoma cells were suspended in HAT complete medium (RPMI-1640 medium containing 20% FBS, 1×HAT and 1×OPI), and aliquoted into 96-well cell culture plate ($1\times10^5$ cells/ 150$\mu$l/well), and incubated at 37°C, 5% $CO_2$. On day 5 after fusion, HAT complete medium was added, 50$\mu$l/well, and incubated at 37°C, 5% $CO_2$. From day 7 to day 8 after fusion, the medium was completely changed with HT complete medium (RPMI-1640 medium containing 20% FBS, 1×HT and 1×OPI), 200$\mu$l/well, according to the cell growth density, and incubated at 37°C, 5% $CO_2$.

3. Screening of Hybridoma cells

10-11 days after fusion, CD38 binding ELISA assay was performed according to the growth density of the cells (see Test Example 1). The cell supernatant of positive wells detected by ELISA was tested by FACS method for the binding of CD38-FL-CHO-S (see Test Example 2). The medium in the positive wells were changed, and the cells were expanded in 24-well plates according to the density of the cells. The cell lines transferred into a 24-well plate were tested again for confirmation, and then sub-cloned for the first time. After screening the first sub-cloned cells (see Test examples 1 and 2), positive cells were preserved and subjected to the second sub-cloning. The positive cells screened in the second sub-cloning (see Test examples 1 and 2) were preserved, and used for protein expression. Hybridoma cells with high affinity to CD38 were obtained after several fusions.

The hybridoma clones m009, m011 and m160 were obtained by screening via blocking assay and binding assay. The antibodies were further prepared by serum-free cell culture method. The antibodies were purified according to the example of purification, and used for the Test Examples.

The sequence of the murine antibody variable region of hybridoma clone 009 is shown as follows:

> m009 VH: m009 heavy chain variable region sequence

*EFQLQQSGPELVKPGASVKISCKASGYSFT*<u>DYNLN</u>*WVKQSNGKSLEWIG*<u>VINPKYD</u>

<u>AINYNQKFKD</u>*KATLTVDQSSSTAYMQLSSLTSEDSAVYYCAR*<u>EGWGKALDY</u>*WGPGT*

*SVIVSS*

SEQ ID No: 3;

>m009 mVL: m009 light chain variable region sequence

*DFVLTQSPATLSVTPGDSVSLSC*<u>RASQSIYTNLH</u>*WYQQKSHESPRLLIK*<u>YASQSIS</u>*GIP*

*SRFSGSGSGTDFTLSINSVETEDSGMYFC*<u>QQSNSWPLT</u>*FGAGTKLELK*

SEQ ID No: 4;

Note: The CDR sequences determined according to Kabat Numbering Criteria are underlined, the FR sequences are presented in italic, and the sequences are arranged in the order of FR1-CDR1-FR2-CDR2-FR3-CDR3-FR4.

The murine antibody variable region sequence of hybridoma clone m011 is as follows:

>m011 VH: m011 heavy chain variable region sequence

*EVQLVESGGGLVKPGGSLKLSCAASGFTFS*<u>DYGMH</u>*WVRQAPEKGLEWVA*<u>FISSGSS</u>

<u>SIYYADTVKG</u>*RFTISRDNAKNTLFLQMTSLRSEDTAMYSCAR*<u>NYVSSYGYFDY</u>*WG*

*QGTTLTVSS*

SEQ ID No: 5;

>m011 VL: m011 light chain variable region sequence

*DIVMTQSPASLAVSLGQRATISC*<u>RASENVDNYGISFMH</u>*WYQQKPGQPPKLLIY*<u>RASN</u>

<u>LES</u>*GIPARFSGSGSRTDFTLTINPVETDDVATYYC*<u>QQSNKDPLT</u>*FGSGTKLEIK*

SEQ ID No: 6;

Note: The CDR sequences determined according to Kabat Numbering Criteria are underlined, the FR sequences are presented in italic, and the sequences are arranged in the order of FR1-CDR1-FR2-CDR2-FR3-CDR3-FR4.

The murine antibody variable region sequence of hybridoma clone m160 is as follows:

>m160 VH: m160 heavy chain variable region sequence

*EVQLVESGGGLVKPGGSLKLSCVASGFTFS*DYGMH*WVRQAPEKGLEWIA*FISTGSS
NIYYVDKVKG*RFTISRDNAKNTLFLQMTSLRSEDTAMYYCAR*NYVSSYGYFDY*WG
QGTTLTVSS*

SEQ ID No: 7;

>m160 VL: m160 light chain variable region sequence

*DIVLTQSPASLAVSLGQRATVSC*RASESVDNYGISFMH*WYQQKPGQPPKLLIY*RASN
LES*GIPARFSGSGSRTDFTLTINPVETDDVATYYC*QQTNKDPLT*FGGGTKLELK*

SEQ ID No: 8;

Note: The CDR sequences determined according to Kabat Numbering Criteria are underlined, the FR sequences are presented in italic, and the sequences are arranged in the order of FR1-CDR1-FR2-CDR2-FR3-CDR3-FR4.
The sequence of each heavy chain and light chain CDR region is shown in Table 3:

Table 3. Sequences of heavy and light chain CDR regions

| Antibody | Heavy chain | | Light chain | |
|---|---|---|---|---|
| m009 | HCDR 1 | DYNLN SEQ ID No: 9 | LCDR 1 | RASQSIYTNLH SEQ ID No: 12 |
| | HCDR 2 | VINPKYDAINYNQKFKD SEQ ID No: 10 | LCDR 2 | YASQSIS SEQ ID No: 13 |
| | HCDR 3 | EGWGKALDY SEQ ID No: 11 | LCDR 3 | QQSNSWPLT SEQ ID No: 14 |
| m011 | HCDR 1 | DYGMH SEQ ID No: 15 | LCDR 1 | RASENVDNYGISFMH SEQ ID No: 18 |
| | HCDR 2 | FISSGSSSIYYADTVKG SEQ ID No: 16 | LCDR 2 | RASNLES SEQ ID No: 19 |
| | HCDR 3 | NYVSSYGYFDY SEQ ID No: 17 | LCDR 3 | QQSNKDPLT SEQ ID No: 20 |
| m160 | HCDR 1 | DYGMH SEQ ID No: 15 | LCDR 1 | RASESVDNYGISFMH SEQ ID No: 22 |
| | HCDR 2 | FISTGSSNIYYVDKVKG SEQ ID No: 21 | LCDR 2 | RASNLES SEQ ID No: 19 |
| | HCDR 3 | NYVSSYGYFDY SEQ ID No: 17 | LCDR 3 | QQTNKDPLT SEQ ID No: 23 |

4. Preparation of human IgG1 chimeric antibody

The candidate molecules obtained from hybridoma screening were amplified and sequenced to obtain the gene sequences encoding the variable regions. Forward and reverse primers were designed on the basis of the sequences obtained by sequencing, the genes sequenced were used as templates to construct the VH/VK gene fragment of each antibody via PCR, and then inserted into the expression vector pHr (with a signal peptide and hIgG1/hkappa constant region gene (CH1-Fc/CL) fragment) via homologous recombination to construct an expression plasmid for the expression of a full-length of recombinant chimeric antibody VH-CH1-Fc-pHr/VL-CL-pHr, resulting in chimeric antibodies ch-009, ch-011 and ch-160 of hybridoma clones m009, m011 and m160.

**Example 4. Humanization of anti-CD38 hybridoma monoclonal antibodies**

The heavy/light chain variable region germline genes with high homology to m009, m011 and m160 respectively were selected as templates by aligning the IMGT human antibody heavy and light chain variable region germline gene database using MOE software analysis. The CDRs of the three murine antibodies were grafted into the corresponding human template to form a humanized antibody variable region sequence in the order of FR1-CDR1-FR2-CDR2-FR3-CDR3-FR4.

Selection of human FR regions and amino acid back-mutations on FR regions: Based on the resulting typical structure of murine antibody VH/VL CDR, homologous sequences of the light chain variable region (VL) and heavy chain variable region (VH) were retrieved from human germline database, arranged by FR homology from high to low, and the germline with the highest FR homology was selected as the main template; The CDR regions of the murine antibody were grafted onto the human template; Further, back-mutations designs were performed on the embedded residues, the residues that directly interact with CDR regions, and the residues that have an important influence on the conformation of VL and VH using software on the basis of the three-dimensional structure of the murine antibody; The chemically unstable amino acid residues were optimized, and the final humanized molecules were obtained.

1. Selection of frameworks for humanized hybridoma clone m009

For the murine antibody m009, the humanized light chain templates were IGKV3-11*01 and hJK4.1, and the humanized heavy chain templates were IGHV1-3*01 and hJH4.1. The CDRs of m009 were grafted onto the human templates and the resulting humanized variable region sequences are as follows:

> h009VH- CDR graft

*EVQLVQSGAEVKKPGASVKVSCKASGYTFT*<u>DYNLN</u>*WVRQAPGQRLEWMG*<u>VINPKY DAINYNQKFKD</u>*RVTITRDTSASTAYMELSSLRSEDTAVYYCAR*<u>EGWGKALDY</u>*WGQG TLVTVSS*

SEQ ID No: 24;

> H009VL -CDR graft

*EIVLTQSPATLSLSPGERATLSC*<u>RASQSIYTNLH</u>*WYQQKPGQAPRLLIY*<u>YASQSIS</u>*GIP ARFSGSGSGTDFTLTISSLEPEDFAVYYC*<u>QQSNSWPLT</u>*FGGGTKVEIK*

SEQ ID No: 25;

Note: The CDR sequences determined according to Kabat Numbering Criteria are underlined, the FR sequences are presented in italic, and the sequences are arranged in the order of FR1-CDR1-FR2-CDR2-FR3-CDR3-FR4.

2. The back-mutations designed for the humanization of hybridoma clone m009 are shown in Table 4:

Table 4. Back-mutations for the humanization of hybridoma clone m009

| VL | | VH | |
|---|---|---|---|
| h009 VL1 | graft | h009 VH1 | graft |
| h009 VL2 | A43S, Y49K | h009 VH2 | V2F, R44S, R71V |
| h009 VL3 | I2F, A43S, Y49K, Y87F | h009 VH3 | V2F, R44S, M48I, V67A, R71V |

(continued)

| VL | | VH | |
|---|---|---|---|
| | | h009 VH4 | V2F, R38K, R44S, R66K, I69L, R71V, T73Q |
| | | h009 VH5 | V2F, R38K, R44S, M48I, V67A, R66K, I69L, R71V, T73Q |
| Note: Graft means that CDRs of the murine antibodies were grafted onto the human germline FR regions; "A43S" means the "A" at position 43 (numbered according to the Kabat Numbering criteria) was back-mutated to "S", and so on. | | | |

The humanized antibody variable region sequences of the hybridoma clone m009 are as follows:

> h009 VH1 (SEQ ID No: 24)

EVQLVQSGAEVKKPGASVKVSCKASGYTFTDYNLNWVRQAPGQRLEWMG
VINPKYDAINYNQKFKDRVTITRDTSASTAYMELSSLRSEDTAVYYCAREGW
GKALDYWGQGTLVTVSS

> h009 VH2 (SEQ ID No: 26)

E**F**QLVQSGAEVKKPGASVKVSCKASGYTFTDYNLNWVRQAPGQ**S**LEWMGV
INPKYDAINYNQKFKDRVTIT**V**DTSASTAYMELSSLRSEDTAVYYCAREGWG
KALDYWGQGTLVTVSS

> h009 VH3 (SEQ ID No: 27)

E**F**QLVQSGAEVKKPGASVKVSCKASGYTFTDYNLNWVRQAPGQ**S**LEW**I**GVI
NPKYDAINYNQKFKDR**A**TIT**V**DTSASTAYMELSSLRSEDTAVYYCAREGWG
KALDYWGQGTLVTVSS

> h009 VH4 (SEQ ID No: 28)

EFQLVQSGAEVKKPGASVKVSCKASGYTFTDYNLNWVKQAPGQSLEWMGV
INPKYDAINYNQKFKDKVTLTVDQSASTAYMELSSLRSEDTAVYYCAREGW
GKALDYWGQGTLVTVSS

> h009 VH5 (SEQ ID No: 29)

EFQLVQSGAEVKKPGASVKVSCKASGYTFTDYNLNWVKQAPGQSLEWIGVI
NPKYDAINYNQKFKDKATLTVDQSASTAYMELSSLRSEDTAVYYCAREGWG
KALDYWGQGTLVTVSS

> h009 VL1 (SEQ ID No: 25)

EIVLTQSPATLSLSPGERATLSCRASQSIYTNLHWYQQKPGQAPRLLIYYASQS
ISGIPARFSGSGSGTDFTLTISSLEPEDFAVYYCQQSNSWPLTFGGGTKVEIK

> h009 VL2 (SEQ ID No: 30)

EIVLTQSPATLSLSPGERATLSCRASQSIYTNLHWYQQKPGQSPRLLIKYASQS
ISGIPARFSGSGSGTDFTLTISSLEPEDFAVYYCQQSNSWPLTFGGGTKVEIK

> h009 VL3 (SEQ ID No: 31)

EFVLTQSPATLSLSPGERATLSCRASQSIYTNLHWYQQKPGQSPRLLIKYASQS
ISGIPARFSGSGSGTDFTLTISSLEPEDFAVYFCQQSNSWPLTFGGGTKVEIK.

The humanized light chain variable region and heavy chain variable region described above were respectively combined with human germline light chain constant region (such as human κ, λ chain light chain constant regions) and heavy chain constant region (such as the heavy chain constant region of human IgG1, IgG2, IgG3, or IgG4 or variant thereof), to form a heavy chain and light chain of the humanized antibody, thereby resulting in a complete humanized antibody of m009 (h009). As an example, full-length humanized antibodies (h009-01 to h009-15) were obtained by combining the above-mentioned h009 antibody heavy chain variable region and light chain variable region with the human IgG1 heavy chain constant region as shown in SEQ ID No: 43 and the human kappa light chain constant region as shown in SEQ ID No: 45 respectively. The variable region sequences are shown in Table 5:

Table 5. Heavy chain variable region and light chain variable sequences of humanized antibody h009

| Variable region | h009 VL1 | h009 VL2 | h009 VL3 |
|---|---|---|---|
| h009 VH1 | h009-01 | h009-06 | h009-11 |
| h009 VH2 | h009-02 | h009-07 | h009-12 |
| h009 VH3 | h009-03 | h009-08 | h009-13 |
| h009 VH4 | h009-04 | h009-09 | h009-14 |
| h009 VH5 | h009-05 | h009-10 | h009-15 |
| Note: For example, for "h009-07" in the table, it suggests that the heavy and light chain variable region of the humanized antibody h009-07 are h009 VH2 and h009VL2 respectively, and so on. | | | |

3. Selection of frameworks for humanized hybridoma clone m011

For the murine antibody m011, the humanized light chain templates were IGKV4-1*01 and hJK4.1, and the humanized heavy chain templates were IGHV3-7*01 and hJH6.1. In order to eliminate potential hot spots, N 82A T (according to the Kabat Numbering criteria, asparagine (abbr. N or Asn) on position 82A was replaced with threonine (abbr. T or Thr)) and N76S (according to the Kabat Numbering criteria, asparagine (abbr. N or Asn) on position 76 was replaced with serine (abbr. S or Ser)) were introduced into the FR regions of human germline IGHV3-7*01 and hJH6.1; the CDRs of m011 were grafted onto human templates and the resulting humanized variable region sequences are as follows:

> h011VH- CDR graft

*EVQLVESGGGLVQPGGSLRLSCAASGFTFS*DYGMH*WVRQAPGKGLEWVA*FISSGSS
SIYYADTVKG*RFTISRDNAKSSLYLQMTSLRAEDTAVYYCAR*NYVSSYGYFDY*WGQ*
*GTTVTVSS*

SEQ ID No: 32;

> h011 VL-CDR graft

*DIVMTQSPDSLAVSLGERATINC*<u>RASENVDNYGISFMH</u>*WYQQKPGQPPKLLIY*<u>RAS</u>
<u>NLES</u>*GVPDRFSGSGSGTDFTLTISSLQAEDVAVYYC*<u>QQSNKDPLT</u>*FGGGTKVEIK*

SEQ ID No: 33;

Note: The CDR sequences determined according to Kabat Numbering Criteria are underlined, the FR sequences are presented in italic, and the sequences are arranged in the order of FR1-CDR1-FR2-CDR2-FR3-CDR3-FR4.

4. The back-mutations designed for the hybridoma clone m011 are shown in Table 6:

Table 6. Back-mutations for the humanization of hybridoma clone m011

| VL | | VH | |
|---|---|---|---|
| h011 VL1 | graft | h011 VH1 | graft, N <u>82A</u> T, N76S |
| h011 VL2 | G68R | h011 VH2 | Y79F, N <u>82A</u> T, Y91S, N76S |
| h011 VL3 | V58I, G68R, V85T | | |
| Note: graft means that the murine antibody CDRs were grafted onto the human germline FR region sequences. G68R means that "G" at position 68 (numbered according to the Kabat Numbering criteria) was back-mutated to R after grafting, and so on. | | | |

The specific sequences of the variable regions of the h011 humanized antibody are as follows:

> h011 VH1 (SEQ ID No: 32)

      EVQLVESGGGLVQPGGSLRLSCAASGFTFSDYGMHWVRQAPGKGLEW
VAFISSGSSSIYYADTVKGRFTISRDNAKSSLYLQMTSLRAEDTAVYYCARNY
VSSYGYFDYWGQGTTVTVSS

> h011 VH2 (SEQ ID No: 34)

      EVQLVESGGGLVQPGGSLRLSCAASGFTFSDYGMHWVRQAPGKGLEW
VAFISSGSSSIYYADTVKGRFTISRDNAKSSL<u>F</u>LQMTSLRAEDTAVY<u>S</u>CARNY
VSSYGYFDYWGQGTTVTVSS

> h011 VL1 (SEQ ID No: 33)

      DIVMTQSPDSLAVSLGERATINCRASENVDNYGISFMHWYQQKPGQPPK
LLIYRASNLESGVPDRFSGSGSGTDFTLTISSLQAEDVAVYYCQQSNKDPLTF

GGGTKVEIK

> h011 VL2 (SEQ ID No: 35)

DIVMTQSPDSLAVSLGERATINCRASENVDNYGISFMHWYQQKPGQPPK LLIYRASNLESGVPDRFSGSGSRTDFTLTISSLQAEDVAVYYCQQSNKDPLTF GGGTKVEIK

> h011VL3 (SEQ ID No: 36)

DIVMTQSPDSLAVSLGERATINCRASENVDNYGISFMHWYQQKPGQP PKLLIYRASNLESGIPDRFSGSGSRTDFTLTISSLQAEDVATYYCQQSNKDPLTFG GGTKVEIK.

The humanized light chain variable region and heavy chain variable region described above were respectively combined with human germline light chain constant region (such as human κ, λ chain light chain constant regions) and heavy chain constant regions (such as the heavy chain constant region of human IgG1, IgG2, IgG3, or IgG4 or variant thereof), to form a heavy chain and light chain of the humanized antibody, thereby resulting in a complete humanized antibody of m011 (h011). As an example, full-length humanized antibodies (h011-01 to h011-06) were obtained by combining the above-mentioned h011 antibody heavy chain variable region and light chain variable region with the human IgG1 heavy chain constant region as shown in SEQ ID No: 43 and the human kappa light chain constant region as shown in SEQ ID No: 45 respectively. The variable region sequences are shown in Table 7:

Table 7: Combinations of heavy and light chain variable regions of humanized antibody h0011

|  | h011VL1 | h011VL2 | h011 VL3 |
|---|---|---|---|
| h011 VH1 | h011-01 | h011-03 | h011-05 |
| h011 VH2 | h011-02 | h011-04 | h011-06 |
| Note: For example, for "h011-04" in the table, it suggests that the heavy and light chain variable region of the humanized antibody h011-04 are h011 VH2 and h011VL2 respectively, and so on. | | | |

5. Selection of frameworks for humanized hybridoma clone m160

For the murine antibody m160, the humanized light chain templates were IGKV4-1*01 and hJK4.1 and the humanized heavy chain templates were IGHV3-7*01 and hJH6.1. In order to eliminate potential hot spots present in the human germline FR regions, mutations S77T (according to the Kabat Numbering criteria, serine (abbr. S or Ser) on position 77 was replaced with threonine (Abbr. T or Thr)) and N 82A T (according to the Kabat Numbering criteria, asparagine (abbr. N or Asn) on position 82A was replaced with threonine (abbr. T or Thr)) were introduced into the FR regions of human germline IGHV3-7*01 and hJH6.1. The m160 CDRs were grafted onto the human template, and the resulting humanized variable region sequences are as follows:

> h160VH-CDR graft

*EVQLVESGGGLVQPGGSLRLSCAASGFTFS*DYGMH*WVRQAPGKGLEWVA*FISTGSS NIYYVDKVKG*RFTISRDNAKNTLYLQMTSLRAEDTAVYYCAR*NYVSSYGYFDY*WG QGTTVTVSS*

SEQ ID No: 37

> h160VL-CDR graft

*DIVMTQSPASLAVSLGERATINC*<u>RASESVDNYGISFMH</u>*WYQQKPGQPPKLLIY*<u>RASN
LES</u>*GVPDRFSGSGSGTDFTLTISSLQAEDVAVYYC*<u>QQTNKDPLT</u>*FGGGTKVEIK*

SEQ ID No: 38

Note: The CDR sequences determined according to Kabat Numbering Criteria are underlined, the FR sequences are presented in italic, and the sequences are arranged in the order of FR1-CDR1-FR2-CDR2-FR3-CDR3-FR4.

6. The back-mutations designed for the humanization of hybridoma clone m160 are shown in Table 8:

Table 8. Back-mutations for the humanization of hybridoma clone m160

| VL | | VH | |
|---|---|---|---|
| h160 VL1 | Graft, D9A | h160 VH1 | Graft, S77T, N <u>82A</u> T |
| h160 VL2 | M4L, D9A | h160 VH2 | V48I, S77T, N <u>82A</u> T |
| h160 VL3 | M4L, D9A, D60A, G68R | | |
| h160 VL4 | M4L, D9A, N22S, V58I, D60A, G68R | | |
| Note: Graft means that CDRs of the murine antibodies were grafted onto the human germline FR regions; "M4L" means the "M" on position 4 (numbered according to the Kabat Numbering criteria) was back-mutated to "L" after grafting, and so on. | | | |

The specific sequences of the variable regions of the humanized antibody h160 are as follows:

> h160 VH1 (SEQ ID No: 37)

EVQLVESGGGLVQPGGSLRLSCAASGFTFSDYGMHWVRQAPGKGLEWVAFI
STGSSNIYYVDKVKGRFTISRDNAKNTLYLQMTSLRAEDTAVYYCARNYVSS
YGYFDYWGQGTTVTVSS;

> h160 VH2 (SEQ ID No: 39)

EVQLVESGGGLVQPGGSLRLSCAASGFTFSDYGMHWVRQAPGKGLEW<u>I</u>AFIS
TGSSNIYYVDKVKGRFTISRDNAKNTLYLQMTSLRAEDTAVYYCARNYVSS
YGYFDYWGQGTTVTVSS;

> h160 VL1 (SEQ ID No: 38)

DIVMTQSPASLAVSLGERATINCRASESVDNYGISFMHWYQQKPGQPPKLLIY

RASNLESGVPDRFSGSGSGTDFTLTISSLQAEDVAVYYCQQTNKDPLTFGGGT
KVEIK;

> h160 VL2 (SEQ ID No: 40)

DIVLTQSPASLAVSLGERATINCRASESVDNYGISFMHWYQQKPGQPPKLLIY
RASNLESGVPDRFSGSGSGTDFTLTISSLQAEDVAVYYCQQTNKDPLTFGGGT
KVEIK;

> h160 VL3 (SEQ ID No: 41)

DIVLTQSPASLAVSLGERATINCRASESVDNYGISFMHWYQQKPGQPPKLLIY
RASNLESGVPARFSGSGSRTDFTLTISSLQAEDVAVYYCQQTNKDPLTFGGGT
KVEIK;

> h160 VL4 (SEQ ID No: 42)

DIVLTQSPASLAVSLGERATISCRASESVDNYGISFMHWYQQKPGQPPKLLIYRA
SNLESGIPARFSGSGSRTDFTLTISSLQAEDVAVYYCQQTNKDPLTFGGGTKVEI
K.

The humanized light chain variable region and heavy chain variable region described above were respectively combined with human germline light chain constant region (such as human κ, λ chain light chain constant regions) and heavy chain constant regions (such as the heavy chain constant region of human IgG1, IgG2, IgG3, or IgG4 or variant thereof), to form a heavy chain and light chain of the humanized antibody, thereby resulting in a complete humanized antibody of m160 (h160). As an example, full-length humanized antibodies (h160-01 to h160-08) were obtained by combining the above-mentioned h160 antibody heavy chain variable region and light chain variable region with the human IgG1 heavy chain constant region as shown in SEQ ID No: 43 and the human kappa light chain constant region as shown in SEQ ID No: 45 respectively. The variable region sequences are shown in Table 9:

Table 9. Heavy chain variable region and light chain variable sequences of humanized antibody h016

|  | h160VL1 | h160 VL2 | h160 VL3 | h160 VL4 |
|---|---|---|---|---|
| h160 VH1 | h160-01 | h160-02 | h160-03 | h160-04 |
| h160 VH2 | h160-05 | h160-06 | h160-07 | h160-08 |
| Note: For example, for "h160-07" in the table, it suggests that the heavy and light chain variable region of the humanized antibody h160-07 are h160 VH2 and h160 VL3 respectively, and so on. | | | | |

**Example 5. Construction and expression of anti-human CD38 humanized antibody IgG1 or IgG1-E333A format**

Various primers were designed, VH/VK gene fragment of each humanized antibody was amplified by PCR and then inserted into the expression vector pHr (with a signal peptide and constant region gene (CH1-FC/CL) fragment, constructed in laboratory) via homologous recombination to construct an expression vector for a full-length antibody VH-CH1-FC-pHr/VK-CL-pHr. For the humanized antibody, the light chain constant region may be selected from human κ or λ chain light chain constant region, and the heavy chain constant region may be selected from the heavy chain constant region of human IgG1, IgG2, IgG3, or IgG4 or variant thereof. Non-limiting examples include optimizing the constant region of human IgG1, IgG2 or IgG4 to improve antibody's function. For example, the IgG1-E333A constant region can be obtained by introducing E333A point-mutation into IgG1, which can enhance the binding ability of IgG1-Fc to C1q and consequently enhance the CDC function of the antibody (see US6528624). The following specific light/heavy chain constant regions are not intended to limit the antibody constant regions of the present disclosure, and other antibody light/heavy chain constant regions and variants thereof known in the art can also be used.

Exemplary heavy and light chain constant regions are as follows:

IgG1 heavy chain constant region:

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK

SEQ ID No: 43;

IgG1-E333A heavy chain constant region:

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPI
**A**KTISKAKGQPREPQVYTLPPSRDELTKNQVSLTCLVKGFYPSDIAVEWESNGQP
ENNYKTTPPVLDSDGSFFLYSKLTVDKSRWQQGNVFSCSVMHEALHNHYTQKS
LSLSPGK

SEQ ID No: 44;

kappa light chain constant region:

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

SEQ ID No: 45.

As an example, the humanized light chain variable region and heavy chain variable region of the above-mentioned hybridoma clones m009, m011, and m160 were respectively combined with the human IgG1 heavy chain constant region as shown in SEQ ID No: 43 and the human kappa light chain constant region as shown in SEQ ID No: 45, and the resulting full-length humanized antibodies are shown in Table 5, Table 7 and Table 9; As another example, the humanized light chain variable region and heavy chain variable region of the above-mentioned hybridoma clones m009, m011, and m160 were respectively combined with the human IgG1-E333A heavy chain constant region as shown in SEQ ID No: 44 and the human kappa light chain constant region as shown in SEQ ID No: 45, and the resulting full-length humanized antibodies are shown in Table 10:

Table 10. Variable region sequences of the humanized antibodies (heavy chain constant region is human IgG1-E333A, and light chain constant region is kappa)

| Antibody | Heavy chain variable region VH | Light chain variable region VL |
|---|---|---|
| h009-01E | h009 VH1 | h009 VL1 |
| h009-02E | h009 VH2 | h009 VL1 |
| h009-03E | h009 VH3 | h009 VL1 |
| h009-04E | h009 VH4 | h009 VL1 |
| h009-05E | h009 VH5 | h009 VL1 |

(continued)

| Antibody | Heavy chain variable region VH | Light chain variable region VL |
|---|---|---|
| h009-06E | h009 VH1 | h009 VL2 |
| h009-07E (Also referred to as hu9E) | h009 VH2 | h009 VL2 |
| h009-08E | h009 VH3 | h009 VL2 |
| h009-09E | h009 VH4 | h009 VL2 |
| h009-10E | h009 VH5 | h009 VL2 |
| h009-11E | h009 VH1 | h009 VL3 |
| h009-12E | h009 VH2 | h009 VL3 |
| h009-13E | h009 VH3 | h009 VL3 |
| h009-14E | h009 VH4 | h009 VL3 |
| h009-15E | h009 VH5 | h009 VL3 |
| h011-01E (Also referred to as hul IE) | h011 VH1 | h011VL1 |
| h011-02E | h011 VH2 | h011VL2 |
| h011-03E | h011 VH1 | h011VL3 |
| h011-04E | h011 VH2 | h011VL1 |
| h011-05E | h011 VH1 | h011VL2 |
| h011-06E | h011 VH2 | h011VL3 |
| h160-01E | h160 VH1 | h160VL1 |
| (Also referred to as hu160E) | | |
| h160-02E | h160 VH1 | h160 VL2 |
| h160-03E | h160 VH1 | h160 VL3 |
| h160-04E | h160 VH1 | h160 VL4 |
| h160-05E | h160 VH2 | h160VL1 |
| h160-06E | h160 VH2 | h160 VL2 |
| h160-07E | h160 VH2 | h160 VL3 |
| h160-08E | h160 VH2 | h160 VL4 |

As as example, the full-length amino acid sequences of humanized antibodies h009-07, hu9E, h011-01, hu11E, h160-01 and hu160E are as follows:

Heavy chain sequence of the antibody h009-07:

EFQLVQSGAEVKKPGASVKVSCKASGYTFTDYNLNWVRQAPGQSLEWMGVIN PKYDAINYNQKFKDRVTITVDTSASTAYMELSSLRSEDTAVYYCAREGWGKAL DYWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEY KCKVSNKALPAPIEKTISKAKGQPREPQVYTLPPSRDELTKNQVSLTCLVKGFYP SDIAVEWESNGQPENNYKTTPPVLDSDGSFFLYSKLTVDKSRWQQGNVFSCSVM HEALHNHYTQKSLSLSPGK

SEQ ID No: 46;

Light chain sequence of the antibody h009-07:

EIVLTQSPATLSLSPGERATLSCRASQSIYTNLHWYQQKPGQSPRLLIKYASQSISG IPARFSGSGSGTDFTLTISSLEPEDFAVYYCQQSNSWPLTFGGGTKVEIKRTVAAPS VFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC

SEQ ID No: 47;

Heavy chain sequence of the antibody hu9E:

EFQLVQSGAEVKKPGASVKVSCKASGYTFTDYNLNWVRQAPGQSLEWMGVIN PKYDAINYNQKFKDRVTITVDTSASTAYMELSSLRSEDTAVYYCAREGWGKAL DYWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEY KCKVSNKALPAPIAKTISKAKGQPREPQVYTLPPSRDELTKNQVSLTCLVKGFYP SDIAVEWESNGQPENNYKTTPPVLDSDGSFFLYSKLTVDKSRWQQGNVFSCSVM HEALHNHYTQKSLSLSPGK                    SEQ ID No: 48;

Light chain sequence of the antibody hu9E:

EIVLTQSPATLSLSPGERATLSCRASQSIYTNLHWYQQKPGQSPRLLIKYASQSISG IPARFSGSGSGTDFTLTISSLEPEDFAVYYCQQSNSWPLTFGGGTKVEIKRTVAAPS VFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC

SEQ ID No: 47;

Heavy chain sequence of the antibody h011-01:

EVQLVESGGGLVQPGGSLRLSCAASGFTFSDYGMHWVRQAPGKGLEWVAFISS GSSSIYYADTVKGRFTISRDNAKSSLYLQMTSLRAEDTAVYYCARNYVSSYGYF DYWGQGTTVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEY KCKVSNKALPAPIEKTISKAKGQPREPQVYTLPPSRDELTKNQVSLTCLVKGFYP SDIAVEWESNGQPENNYKTTPPVLDSDGSFFLYSKLTVDKSRWQQGNVFSCSVM HEALHNHYTQKSLSLSPGK

SEQ ID No: 49;

Light chain sequence of the antibody h011-01:

DIVMTQSPDSLAVSLGERATINCRASENVDNYGISFMHWYQQKPGQPPKLLIYR ASNLESGVPDRFSGSGSGTDFTLTISSLQAEDVAVYYCQQSNKDPLTFGGGTKVE IKRTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRGE C

SEQ ID No: 50;

Heavy chain sequence of the antibody hu11E:

EVQLVESGGGLVQPGGSLRLSCAASGFTFSDYGMHWVRQAPGKGLEWVAFISS GSSSIYYADTVKGRFTISRDNAKSSLYLQMTSLRAEDTAVYYCARNYVSSYGYF DYWGQGTTVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEY KCKVSNKALPAPIAKTISKAKGQPREPQVYTLPPSRDELTKNQVSLTCLVKGFYP SDIAVEWESNGQPENNYKTTPPVLDSDGSFFLYSKLTVDKSRWQQGNVFSCSVM HEALHNHYTQKSLSLSPGK

SEQ ID No: 51;

Light chain sequence of the antibody hu11E:

DIVMTQSPDSLAVSLGERATINCRASENVDNYGISFMHWYQQKPGQPPKLLIYR ASNLESGVPDRFSGSGSGTDFTLTISSLQAEDVAVYYCQQSNKDPLTFGGGTKVE IKRTVAAPSVFIFPPSDEQLKSGTASVVCLLNNFYPREAKVQWKVDNALQSGNS

QESVTEQDSKDSTYSLSSTLTLSKADYEKHKVYACEVTHQGLSSPVTKSFNRGE
C

SEQ ID No: 50;

Heavy chain sequence of the antibody h160-01:

EVQLVESGGGLVQPGGSLRLSCAASGFTFSDYGMHWVRQAPGKGLEWVAFIST
GSSNIYYVDKVKGRFTISRDNAKNTLYLQMTSLRAEDTAVYYCARNYVSSYGY
FDYWGQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK

SEQ ID No: 52;

Light chain sequence of the antibody h160-01:

DIVMTQSPASLAVSLGERATINCRASESVDNYGISFMHWYQQKPGQPPKLLIYR
ASNLESGVPDRFSGSGSGTDFTLTISSLQAEDVAVYYCQQTNKDPLTFGGGTKVE
IKRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGE
C

SEQ ID No: 53;

Heavy chain sequence of the antibody hu160E:

EVQLVESGGGLVQPGGSLRLSCAASGFTFSDYGMHWVRQAPGKGLEWVAFIST
GSSNIYYVDKVKGRFTISRDNAKNTLYLQMTSLRAEDTAVYYCARNYVSSYGY
FDYWGQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIAKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK

SEQ ID No: 54;

Light chain sequence of the antibody hu160E :

DIVMTQSPASLAVSLGERATINCRASESVDNYGISFMHWYQQKPGQPPKLLIYR
ASNLESGVPDRFSGSGSGTDFTLTISSLQAEDVAVYYCQQTNKDPLTFGGGTKVE
IKRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGE
C

SEQ ID No: 53;

The anti-CD38 antibody, Daratumumab (abbr. Dara, refer to WHO Drug Information, Vol. 24, No.1, 2010 for sequences) was used as a control antibody in the present disclosure, and its heavy chain and light chain sequences are as follows:

Heavy chain sequence of Dara:

EVQLLESGGGLVQPGGSLRLSCAVSGFTFNSFAMSWVRQAPGKGLEWVSA
ISGSGGGTYYADSVKGRFTISRDNSKNTLYLQMNSLRAEDTAVYFCAKDKILWF
GEPVFDYWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSN
TKVDKRVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQQGN
VFSCSVMHEALHNHYTQKSLSLSPGK

SEQ ID No: 55;

Light chain sequence of Dara:

EIVLTQSPATLSLSPGERATLSCRASQSVSSYLAWYQQKPGQAPRLLIYDAS
NRATGIPARFSGSGSGTDFTLTISSLEPEDFAVYYCQQRSNWPPTFGQGTKVEIKR
TVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

SEQ ID No: 56.

The performance and effect of the present antibdoies were verified by the following tests:

**Test Example 1. CD38 protein-binding ELISA test for CD38 antibodies**

The affinity of the anti-CD38 antibodies were determined by the amount of the antibodies binding to CD38 immobilized on the ELISA plate. 2 μg/ml streptavidin (Abcam, CAT #ab123480) was coated on a 96-well ELISA plate (Costar, CAT#3590), the plate was washed, blocked, and then 2 μg/ml biotin-labeled CD38-ECD-His was added. After incubation, the diluted anti-CD38 antibody samples with various concentrations were added , washed, and then added with horseradish peroxidase-goat anti-human F(ab') 2 antibody (Jackson, CAT# 109-036-097). The plate was washed again and tetramethyl benzidine solution was added for color reaction. Finally, stop solution was added. OD450 was measured on a microplate reader and EC50 value was calculated. The results are shown in Table 11.

Table 11. Affinity of CD38 humanized antibody

| Antibody | EC50 ($\mu$g/ml) |
|----------|------------------|
| hu9E | 0.02594 |
| hu11E | 0.02762 |
| hu160E | 0.02801 |

The results show that the humanized antibodies of the present disclosure can specifically bind to CD38 protein with strong binding ability.

**Test Example 2. Binding Assay of CD38 antibodies to CD38-FL-CHO-S cells**

CHO-S cells (FreeStyle™ CHO-S cells, Invitrogen, R80007) stably transfected with full-length human CD38 (Uniprot number: P28907) (CD38-FL-CHOS) were cultivated in CD CHO culture medium (Gibco, REF#10743-029). $1\times10^6$ cells/ml of CD38-FL-CHO-S cells were blocked with 1% BSA, and the diluted anti-CD38 antibody samples with various concentrations were added, washed twice, and then Alexa Fluor 488-goat anti-human (H+L) antibody (Invitrogen, CAT#A11013) was added, washed twice, and the fluorescence signal values were read with flow cytometer. The results are shown in Table 12.

Table 12. Affinity of CD38 humanized antibody

| Antibody | EC50 ($\mu$g/ml) |
|----------|------------------|
| hu9E | 0.4020 |
| hu11E | 0.4813 |
| hu160E | 0.4740 |

FACS test results show that the humanized antibodies of the present disclosure have strong binding ability to natural CD38 present on the cell surface.

**Test Example 3. Inhibitory assay of CD38 antibodies on CD38 enzyme activity**

CD38-ECD-His was prepared to a solution with a concentration of 4 $\mu$g/ml with 20 mMTris-HCl (pH 6.5) buffer. Similarly, various concentrations of anti-CD38 antibody samples were prepared with buffer. 25$\mu$l of each of CD38-ECD-His and anti-CD38 antibody sample were added into a 96-well plate with a transparent bottom and black wall (Corning, CAT#3603). After incubated at room temperature for 15 minutes, 50$\mu$l of 200$\mu$M substrate NGD (Sigma, CAT#N5131-25MG) was added. After incubated at room temperature for 2 hours, the production of cyclic GDP ribose (cGDPR) was measured by FlexStation 3 (Molecular Devices), at emission wavelength of 410 nm (excitation light of 300 nm). The results are shown in Table 13.

Table 13. Inhibitory results of CD38 humanized antibody on CD38 enzyme activity

| Antibody | IC50 ($\mu$g/ml) | Imax (Maximum inhibition rate %) |
|----------|------------------|----------------------------------|
| Dara | 3.147 | 44.54 |
| hu9E | 1.559 | 89.63 |
| hu11E | 1.827 | 44.31 |
| hu160E | 1.199 | 47.67 |

The test results show that hu9E exhibits a maximum inhibition rate of 89.63% for enzyme activity, significantly superior to that of the control antibody Dara; whereas hu11E and hu160E have a maximum inhibition rates of 44.31% and 47.67% respectively for enzyme activity, comparable to that of the control antibody.

**Test Example 4. *In vitro* ADCC assay of CD38 antibodies on Molp-8 and Daudi cells**

Human multiple myeloma cell line Molp-8 (Cobioer, Nanjing, CBP60562) or human Burkitt's lymphoma cell line Daudi cells (ATCC, CCL-123) were collected, centrifuged at 1000 rpm for 5 minutes, and suspended with phenol red-free RPMI 1640 medium (Gibco, CAT# 11835-030) containing 10% ultra-low IgG Fetal Bovine Serum (Gibco, CAT#1921005PJ). The cells were counted with Cytometer (Countstar, IC1000) and diluted to $1 \times 10^5$ cells/ml.

Peripheral blood mononuclear cells (PBMCs) were isolated from fresh human blood by Ficoll (GE, CAT# 17-5442-02), re-suspended in phenol red-free RPMI 1640 medium, counted with Cytometer, and diluted to $3 \times 10^6$ cells/ml.

50 µl of each of Molp-8 (or Daudi) and different concentrations of CD38 antibodies or negative control IgG (C25- hIgG1 (WT), prepared in the laboratory) were added into a 96-well plate at a ratio of 1:1, incubated for 30 minutes (37°C, 5% $CO_2$), and 50 µl of effector cell PBMCs were added at the ratio of 30:1 (effector cell:target cell). After incubated for 4 hours (37°C, 5% $CO_2$), the release of LDH (lactate dehydrogenase) was detected with CytoTox 96 Non-Radiocytotoxicity Test Kit (Promega, CAT#G1780). 50 µl of cell supernatant and 50µl of CytoTox 96® Reagent were added. After incubated at room temperature for 30 minutes, stop solution was added. The absorbance values (490 nm) were detected with FlexStation 3 (Molecular Devices). The results are shown in Table 14.

Table 14. ADCC results of CD38 humanized antibodies on target cells

| Antibody | Molp-8 | | Daudi | |
|---|---|---|---|---|
| | IC50 (µg/ml) | Emax (Maximum efficiency, %) | IC50 (µg/ml) | Emax (Maximum efficiency %) |
| hu9E | 4.5 | 83.6 | 4.9 | 102.5 |
| hu11E | 3.8 | 72.0 | 2.6 | 92.5 |
| hu160E | 2.7 | 71.5 | 3.1 | 96.7 |

Experimental results show that the humanized antibodies of the present disclosure have strong ADCC effect on Molp-8 and Daudi cells *in vitro,* and can significantly achieve the lysis the target cells.

**Test Example 5. CDC of CD38 antibodies on Molp-8 and Daudi cells by *in vitro* assay**

Molp-8 or Daudi cells were collected, centrifuged at 1000 rpm for 5 minutes and re-suspended. The cells were counted with Cytometer (Countstar, IC1000), and re-suspended in phenol red-free RPMI 1640 medium (Gibco, CAT# 11835-030) containing 10% ultra-low IgG Fetal Bovine Serum (Gibco, CAT# 1921005PJ) at $1 \times 10^6$ cells/ml. Subsequently, the cells were plated in a 96-well plate (Corning, CAT#3903) at $5 \times 10^4$ cells/well (50 µl/well). Then, 50 µl of various concentrations of anti-CD38 antibodies and negative control were added. After incubated for 30 minutes (37°C, 5% $CO_2$), 50 µl of human serum (laboratory-made) was added into each well. After incubated for 2 hours (37°C, 5% $CO_2$), 16.6 µl of Alamar Blue Reagent (Thermo, CAT#DAL1025) was added to each well, and incubated for 20 hours (37°C, 5% $CO_2$). Finally, the detection was performed with FlexStation 3 (Molecular Devices) at emission wavelength of 585 nm (excitation wavelength of 570 nm), and the results are shown in Table 15.

Table 15. CDC results of humanized CD38 antibodies on target cells

| Antibody | Molp-8 | | Daudi | |
|---|---|---|---|---|
| | IC50 (µg/ml) | Emax (Maximum efficiency, %) | IC50 (µg/ml) | Emax (Maximum efficiency, %) |
| hu9E | 281.4 | 79.7 | 51.1 | 92.8 |
| hu11E | 289.9 | 67.4 | 70.1 | 91.4 |
| hu160E | 393.2 | 61.5 | 65.7 | 88.1 |

Experimental results show that the humanized antibodies of the present disclosure have strong CDC effect on Molp-8 and Daudi cells *in vitro,* and can significantly achieve the lysis the target cells.

**Test Example 6. *In vitro* ADCP reporting system test of CD38 antibodies on Molp-8 and Daudi cells**

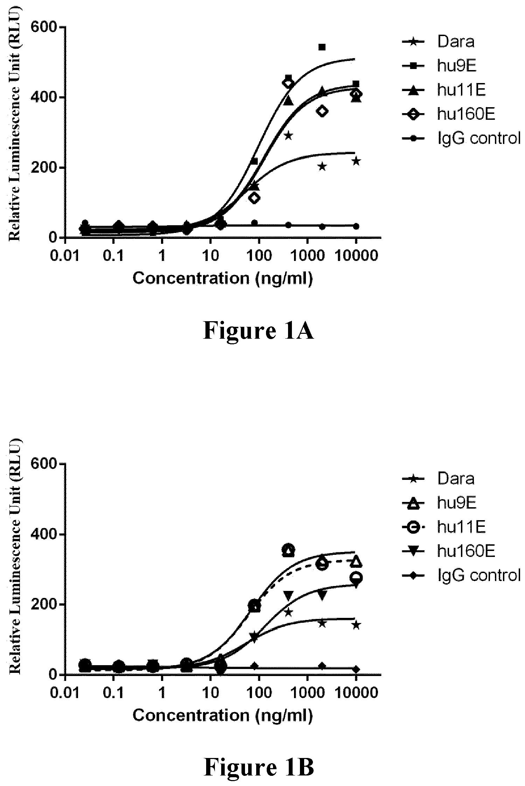

Molp-8 or Daudi cells were collected, centrifuged at 1000 rpm for 5 minutes and re-suspended. The cells were counted with Cytometer (Countstar, IC1000), and re-suspended in phenol red-free RPMI 1640 medium (Gibco, CAT#11835-030) containing 10% ultra-low IgG Fetal Bovine Serum (Gibco, CAT#1921005PJ) at $1\times10^6$ cells/ml. Subsequently, the cells were plated in a 96-well plate (Corning, CAT#3903) at $2.5\times10^4$ cells/well (25 $\mu$l/well), 25 $\mu$l of various concentrations of anti-CD38 antibodies and negative control were added, and incubated for 30 minutes (37°C, 5% $CO_2$). Jurkat (Jurkat-Lucia™ NFAT Cells, Invivogene) cells stably transformed with full-length human Fc$\gamma$IIa (Uniprot number: P12318) were collected as effector cells, and $7.5\times10^4$ cells/well (50 $\mu$l/well) of effector cells were added into a 96-well plate incubated with the target cells and antibodies. After incubated for 6 hours (37°C, 5% $CO_2$), 10 $\mu$l of supernatant was transferred into a new 96-well plate (Corning, CAT#3903), 90 $\mu$l/well of QUANTI-Luc (Invivogene, rep-q1c1) was added, and the chemiluminescence was detected with VITOR (VITOR3, PerkinElmer). The results are shown in Table 16 and Figure 1.

Table 16. *In vitro* ADCP reporter system test results of CD38 humanized antibodies on target cells

| Antibody | Molp-8 | Daudi |
|---|---|---|
| | Signal ratio relative to Dara (fold) | |
| Dara | 1.00 | 1.00 |
| hu9E | 2.00 | 2.30 |
| hu11E | 1.83 | 2.00 |
| hu160E | 1.87 | 1.80 |

The experimental results show that all the humanized antibodies of the present disclosure have *in vitro* ADCP reporter system test effects on Molp-8 and Daudi cells significantly better than that of the control antibody Dara.

**Test Example 7. Affinity of CD38 antibodies detected by BIAcore**

The affinities of the chimeric antibodies (ch-009, -011 and -160 prepared according to Example 3), humanized antibodies of the present disclosure and Dara to the human CD38-ECD-His antigen were detected by Biacore instrument.

Human Fc Capture Molecule was covalently coupled to CM5 biosensing chip (CAT#BR-1005-30, GE) according to the method described in the manual of the Human Fc Capture Kit (CAT#BR-1008-39, GE), for affinity capture of the antibodies to be tested. Then the human CD38-ECD-His antigen passed through the surface of the chip, and a real-time detection for the reaction signal was performed with Biacore instrument. The resulting binding and dissociation curves were fitted to calculate the affinity values. After the dissociation of each cycle was completed in the experiment, the biochip was washed and regenerated with the regeneration solution supplied in the Human Fc Capture Kit (GE). The results are shown in Table 17 and Table 18.

Table 17. Results of the affinity of anti-CD38 antibody to human CD38 by BIAcore

| Antibody | ka (1/Ms) | kd (1/s) | KD (M) |
|---|---|---|---|
| **ch-009** | 2.04E+06 | 4.77E-04 | 2.34E-10 |
| h009-07 | 2.11E+06 | 2.10E-03 | 9.95E-10 |
| h009-08 | 1.89E+06 | 2.23E-03 | 1.18E-09 |
| h009-09 | 2.29E+06 | 4.89E-03 | 2.14E-09 |
| h009-10 | 2.04E+06 | 4.33E-03 | 2.12E-09 |
| h009-11 | 2.33E+06 | 1.21E-02 | 5.17E-09 |
| h009-12 | 2.24E+06 | 9.17E-04 | 4.09E-10 |
| h009-13 | 2.28E+06 | 1.06E-03 | 4.65E-10 |
| h009-14 | 2.27E+06 | 1.85E-03 | 8.15E-10 |
| h009-15 | 2.28E+06 | 1.70E-03 | 7.45E-10 |
| **ch-011** | 7.46E+06 | 1.48E-03 | 1.99E-10 |
| h011-01 | 6.68E+06 | 2.08E-03 | 3.11E-10 |

(continued)

| Antibody | ka (1/Ms) | kd (1/s) | KD (M) |
|---|---|---|---|
| h011-02 | 6.59E+06 | 1.95E-03 | 2.97E-10 |
| h011-03 | 6.97E+06 | 2.13E-03 | 3.06E-10 |
| h011-04 | 6.76E+06 | 2.02E-03 | 2.99E-10 |
| h011-05 | 6.89E+06 | 2.13E-03 | 3.09E-10 |
| h011-06 | 6.71E+06 | 2.01E-03 | 3.00E-10 |
| **ch-160** | 2.74E+06 | 1.70E-04 | 6.20E-11 |
| h160-01 | 1.99E+06 | 1.36E-04 | 6.87E-11 |
| h160-02 | 1.93E+06 | 1.63E-04 | 8.46E-11 |
| h160-03 | 2.40E+06 | 1.87E-04 | 7.79E-11 |
| h160-04 | 2.12E+06 | 1.84E-04 | 8.67E-11 |
| h160-05 | 2.05E+06 | 1.41E-04 | 6.91E-11 |
| h160-06 | 2.09E+06 | 1.64E-04 | 7.82E-11 |
| h160-07 | 2.43E+06 | 1.82E-04 | 7.48E-11 |
| h160-08 | 2.29E+06 | 1.79E-04 | 7.82E-11 |

The results show that all the humanized antibodies obtained in the present disclosure have high affinity to human CD38.

Table 18. Results of the affinity of anti-CD38 antibody to human CD38 by BIAcore

| Ligand | Analyte | ka (1/Ms) | kd (1/s) | KD (M) |
|---|---|---|---|---|
| Dara | Human | 6.45E+05 | 1.51E-03 | 2.35E-09 |
| hu9E | CD38-His | 1.05E+06 | 1.38E-03 | 1.31E-09 |
| huIIE | | 2.07E+06 | 1.17E-03 | 5.68E-10 |
| hu160E | | 1.96E+06 | 1.15E-04 | 5.85E-11 |

The results show that antibodies hu9E, hu11E and hu160E obtained in the present disclosure all have high affinity to human CD38, and the KD values are lower than that of the control antibody Dara, better than that of the control antibody.

## *In vivo* evaluation of biological activity

### Test Example 8. *In vivo* pharmacokinetic test of CD38 antibodies

18 SD rats, male, were divided into 6 groups equally. The animals were provided by Sipur-Bikai Laboratory Animal Co., Ltd.; The animals were respectively administered by intravenous injection or subcutaneous injection, at a dosage of 3 mg/kg. For the group administered by intravenous injection, 0.2 ml of whole blood was collected without anticoagulation before dosing, 5 min, 8h, 1d, 2d, 4d, 7d, 10d, 14d, 21d and 28 d after administration; After collection, the blood was placed at 4°C for 30 min, centrifuged at 1000g for 15 min, and the supernatant (serum) was transferred into EP tubes and stored at -80°C; For the group administered by subcutaneous injection, whole blood was collected before dosing, 1h, 8h, 1d, 2d, 4d, 7d, 10d, 14d, 21d and 28d after administration. The whole blood was collected on days 7, 10, 14, 21, and 28. The serum was isolated, transferred in EP tubes and store at -80°C.

Standard curves for the different samples were generated according to the method described in Test Example 1 (CD38 protein-binding ELISA of the anti-CD38 antibodies). The serum samples, in replacement of the anti-CD38 antibodies at 1:1000 dilution, were added into the the reaction system. The serum concentrations of the anti-CD38 antibodies at different time points were calculated based on OD450, and pharmacokinetic parameters were analyzed and calculated on the basis of the collected data by Phoenix WinNonlin software. The *in vivo* pharmacokinetic results of antibodies hu9E, hu11E and hu160E are shown in Table 19.

Table 19. Evaluation of pharmacokinetics in rat for antibodies

|  | hu9E | | hu11E | | hu160E | |
|---|---|---|---|---|---|---|
| dosage | IV 3mg/kg | SC 3mg/kg | IV 3mg/kg | SC 3mg/kg | IV 3mg/kg | SC 3mg/kg |
| Bioavailability | - | 81.4% | - | 113.8% | - | 113.9% |
| T1/2 (day) | 11.4 | 11.1 | 13.3 | 12.1 | 13.6 | 10.9 |
| Note: In the table, T1/2 means half-life, IV means intravenous injection, SC means subcutaneous injection. | | | | | | |

PK (Pharmacokinetics) of hu9E, hu11E, and hu160E was measured in rats after subcutaneous and intravenous injection at a dosage of 3mpk. The results show that the antibodies have favorable PK performance in rats: high bioavailability is observed in all antibodies when injected subcutaneously, and with the average T1/2 of intravenous injection is 11.4 days, 13.3 days and 13.6 days, respectively; the average T1/2 of subcutaneous injection is 11.1 days, 12.1 days and 10.9 days, respectively, suggesting favorable stability of antibodies in rats and the possibility to develop subcutaneous formulations.

**Test Example 9. *In vivo* pharmacodynamics test of CD38 antibodies on tumor in mice**

Balb/c nude mice, SPF, 14-16g, female, were purchased from Shanghai SLAC Laboratory Animal Co.,Ltd. Balb/c nude mice were allowed to adapt for laboratory environment for 6 days, and were subcutaneously inoculated with AMO-1 cells (Cobioer, Nanjing, CBP60242, $5\times10^6$ +50% matrigel/mouse, basement membrane Matrigel, BD, Cat. No. #356237) into the right ribs. 9 days later, the mice were divided into a total of 7 groups, 8 mice/group, with average tumor volume of about $197.21\pm9.25mm^3$ (d0). The groups were as follows:

Blank control group, IgG (3mg/kg) (C25-hIgG1 (WT), laboratory-made);
Dara (1 mg/kg) group;
Dara (3 mg/kg) group;
hu11E (1 mg/kg) group;
hu11E (3 mg/kg) group;
hu160E (1 mg/kg) group;
hu160E (3 mg/kg) group;
Intraperitoneal injection was performed, twice a week, for 3 weeks. The tumor volume and body weight were measured twice a week, and the data were recorded. The mice were sacrificed and the tumors were removed after all the administrations were completed.

Excel 2003 statistical software was used to calculate the average (avg), SD value (STDEV) and SEM value (STDEV/SQRT); the P value for the difference between groups was calculated by TTEST.

The tumor volume (V) was calculated according to the following formula:

$$V = 1/2 \times L_{long} \times L_{short}^2$$

$$\text{Relative volume (RTV)} = V_T / V_0$$

$$\text{Tumor-inhibition rate (\%)} = (C_{RTV} - T_{RTV})/C_{RTV} (\%)$$

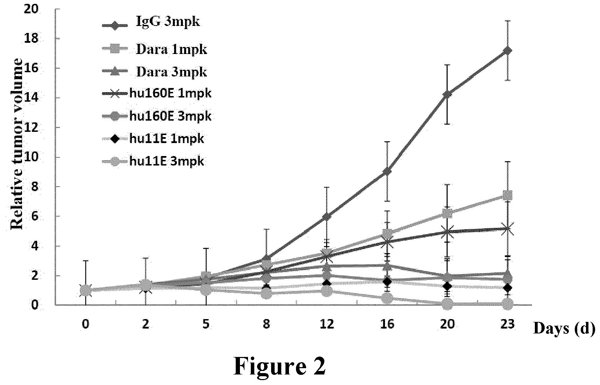

wherein $V_0$ and $V_T$ refer to the tumor volumes at the beginning and at the end of the experiment, respectively. $C_{RTV}$ and $T_{RTV}$ refer to the relative tumor volumes of the control group and the test group at the end of the experiment, respectively. The results are shown in Table 20 and Figure 2.

Table 20. *In vivo* anti-tumor results of anti-CD38 antibodies

| Antibody | dosage | Tumor-inhibition rate (%) |
|---|---|---|
| Dara | 1 mpk | 56.83 |
| | 3 mpk | 87.44 |
| hu11E | 1 mpk | 93.14 |
| | 3 mpk | 99.52 |
| hu160E | 1 mpk | 70.02 |
| | 3 mpk | 89.89 |
| Note: mpk means mg/kg. | | |

The results of *in vivo* anti-tumor efficiency in mice show that both the humanized antibodies hu11E and hu160E of the present disclosure can significantly inhibit the growth of tumor. Compared to the blank control IgG (3 mg/kg) group, hu11E (1 mg/kg) group and hu160E (1 mg/kg) group exhibit tumor-inhibition rate of 93.14% and 70.02%, respectively; hu11E (3 mg/kg) group and hu160E (3 mg/kg) group exhibit tumor-inhibition rate of 99.52% and 89.89%, respectively. During the administration process, the animals in each group displayed normal body weight, indicating that the antibodies of the present disclosure do not have obvious toxic and side effects.

**Claims**

1. An anti-CD3 8 antibody or an antigen-binding fragment thereof, wherein the anti-CD38 antibody or the antigen-binding fragment specifically binds to human CD38, the antibody or the antigen-binding fragment thereof comprising:

(i) a heavy chain HCDR1, the amino acid sequence thereof is as shown in SEQ ID No: 9 or has 3, 2 or 1 amino acid(s) difference(s) when compared with SEQ ID No: 9,

a heavy chain HCDR2, the amino acid sequence thereof is as shown in SEQ ID No: 10 or has 3, 2 or 1 amino acid(s) difference(s) when compared with SEQ ID No: 10,

a heavy chain HCDR3, the amino acid sequence thereof is as shown in SEQ ID No: 11 or has 3, 2 or 1 amino acid(s) difference(s) when compared with SEQ ID No: 11,

a light chain LCDR1, the amino acid sequence thereof is as shown in SEQ ID No: 12 or has 3, 2 or 1 amino acid(s) difference(s) when compared with SEQ ID No: 12,

a light chain LCDR2, the amino acid sequence thereof is as shown in SEQ ID No: 13 or has 3, 2 or 1 amino acid(s) difference(s) when compared with SEQ ID No: 13, and

a light chain LCDR3, the amino acid sequence thereof is as shown in SEQ ID No: 14 or has 3, 2 or 1 amino acid(s) difference(s) when compared with SEQ ID No: 14; or

(ii) a heavy chain HCDR1, the amino acid sequence thereof is as shown in SEQ ID No: 15 or has 3, 2 or 1 amino acid(s) difference(s) when compared with SEQ ID No: 15,

a heavy chain HCDR2, the amino acid sequence thereof is as shown in SEQ ID No: 16 or has 3, 2 or 1 amino acid(s) difference(s) when compared with SEQ ID No: 16,

a heavy chain HCDR3, the amino acid sequence thereof is as shown in SEQ ID No: 17 or has 3, 2 or 1 amino acid(s) difference(s) when compared with SEQ ID No: 17,

a light chain LCDR1, the amino acid sequence thereof is as shown in SEQ ID No: 18 or has 3, 2 or 1 amino acid(s) difference(s) when compared with SEQ ID No: 18,

a light chain LCDR2, the amino acid sequence thereof is as shown in SEQ ID No: 19 or has 3, 2 or 1 amino acid(s) difference(s) when compared with SEQ ID No: 19, and

a light chain LCDR3, the amino acid sequence thereof is as shown in SEQ ID No: 20 or has 3, 2 or 1 amino acid(s) difference(s) when compared with SEQ ID No: 20; or

(iii) a heavy chain HCDR1, the amino acid sequence thereof is as shown in SEQ ID No: 15 or has 3, 2 or 1 amino acid(s) difference(s) when compared with SEQ ID No: 15,

a heavy chain HCDR2, the amino acid sequence thereof is as shown in SEQ ID No: 21 or has 3, 2 or 1 amino acid(s) difference(s) when compared with SEQ ID No: 21,

a heavy chain HCDR3, the amino acid sequence thereof is as shown in SEQ ID No: 17 or has 3, 2 or 1 amino acid(s) difference(s) when compared with SEQ ID No: 17,

a light chain LCDR1, the amino acid sequence thereof is as shown in SEQ ID No: 22 or has 3, 2 or 1 amino acid(s) difference(s) when compared with SEQ ID No: 22,

a light chain LCDR2, the amino acid sequence thereof is as shown in SEQ ID No: 19 or has 3, 2 or 1 amino acid(s) difference(s) when compared with SEQ ID No: 19, and

a light chain LCDR3, the amino acid sequence thereof is as shown in SEQ ID No: 23 or has 3, 2 or 1 amino acid(s) difference(s) when compared with SEQ ID No: 23.

2. The anti-CD38 antibody or the antigen-binding fragment thereof according to claim 1, wherein the antibody is murine antibody, chimeric antibody or humanized antibody.

3. The anti-CD38 antibody or the antigen-binding fragment thereof according to claim 2, wherein the murine antibody or chimeric antibody comprises a heavy chain variable region and a light chain variable region, wherein:

(a) the amino acid sequence of the heavy chain variable region is as shown in SEQ ID No: 3 or has at least 95% sequence identity to SEQ ID No: 3, and the amino acid sequence of the light chain variable region is as shown in SEQ ID No: 4 or has at least 95% sequence identity to SEQ ID No: 4;
(b) the amino acid sequence of the heavy chain variable region is as shown in SEQ ID No: 5 or has at least 95% sequence identity to SEQ ID No: 5, and the amino acid sequence of the light chain variable region is as shown in SEQ ID No: 6 or has at least 95% sequence identity to SEQ ID No: 6; or
(c) the amino acid sequence of the heavy chain variable region is as shown in SEQ ID No: 7 or has at least 95% sequence identity to SEQ ID No: 7, and the amino acid sequence of the light chain variable region is as shown in SEQ ID No: 8 or has at least 95% sequence identity to SEQ ID No: 8.

4. The anti-CD38 antibody or the antigen-binding fragment thereof according to claim 2, wherein the antibody is humanized antibody comprising framework regions or framework region variants derived from human antibody, and the framework region variants have up to 10 amino acid back-mutation(s) on light chain framework regions and/or heavy chain framework regions of the human antibody, respectively;
preferably, the humanized antibody comprises any one selected from the following (d) to (f):

(d) a heavy chain variable region, wherein the heavy chain variable region comprising: heavy chain HCDR1, HCDR2, HCDR3 and heavy chain framework region(s), wherein the amino acid sequence of the HCDR1 is as shown in SEQ ID No: 9 or has 3, 2 or 1 amino acid(s) difference(s) when compared with SEQ ID No: 9, the amino acid sequence of the HCDR2 is as shown in SEQ ID No: 10 or has 3, 2 or 1 amino acid(s) difference(s) when compared with SEQ ID No: 10, the amino acid of the HCDR3 is as shown in SEQ ID No: 11 or has 3, 2 or 1 amino acid(s) difference(s) when compared with SEQ ID No: 11, and the heavy chain framework region(s) comprise(s) one or more back-mutation(s) selected from the group consisting of: 2F, 38K, 44S, 48I, 67A, 66K, 69L, 71V and 73Q; and/or

a light chain variable region, wherein the light chain variable region comprising: light chain LCDR1, LCDR2, LCDR3 and light chain framework region(s), wherein the amino acid sequence of the LCDR1 is as shown in SEQ ID No: 12 or has 3, 2 or 1 amino acid(s) difference(s) when compared with SEQ ID No: 12, the amino acid sequence of the LCDR2 is as shown in SEQ ID No: 13 or has 3, 2 or 1 amino acid(s) difference(s) when compared with SEQ ID No: 13, the amino acid of the LCDR3 is as shown in SEQ ID No: 14 or has 3, 2 or 1 amino acid(s) difference(s) when compared with SEQ ID No: 14, and the light chain framework region(s) comprise(s) one or more back-mutation(s) selected from the group consisting of: 2F, 43S, 49K and 87F;

(e) a heavy chain variable region, wherein the heavy chain variable region comprising: heavy chain HCDR1, HCDR2, HCDR3 and heavy chain framework region(s), wherein the amino acid sequence of the HCDR1 is as shown in SEQ ID No: 15 or has 3, 2 or 1 amino acid(s) difference(s) when compared with SEQ ID No: 15, the amino acid sequence of the HCDR2 is as shown in SEQ ID No: 16 or has 3, 2 or 1 amino acid(s) difference(s) when compared with SEQ ID No: 16, the amino acid of the HCDR3 is as shown in SEQ ID No: 17 or has 3, 2 or 1 amino acid(s) difference(s) when compared with SEQ ID No: 17, and the heavy chain framework region(s) comprise(s) one or more back-mutation(s) selected from the group consisting of: 79F, 82AT, 91S and 76S; and/or

a light chain variable region, wherein the light chain variable region comprising: light chain LCDR1, LCDR2, LCDR3 and light chain framework region(s), wherein the amino acid sequence of the LCDR1 is as shown in SEQ ID No: 18 or has 3, 2 or 1 amino acid(s) difference(s) when compared with SEQ ID No: 18, the amino acid sequence of the LCDR2 is as shown in SEQ ID No: 19 or has 3, 2 or 1 amino acid(s) difference(s) when compared with SEQ ID No: 19, the amino acid of the LCDR3 is as shown in SEQ ID No: 20 or has 3, 2 or 1 amino acid(s) difference(s) when compared with SEQ ID No: 20, and the light chain framework region(s) comprise(s) one or more back-mutation(s) selected from the group consisting of: 58I, 68R and 85T; and

(f) a heavy chain variable region, wherein the heavy chain variable region comprising: heavy chain HCDR1, HCDR2, HCDR3 and heavy chain framework region(s), wherein the amino acid sequence of the HCDR1 is as shown in SEQ ID No: 15 or has 3, 2 or 1 amino acid(s) difference(s) when compared with SEQ ID No: 15, the amino acid sequence of the HCDR2 is as shown in SEQ ID No: 21 or has 3, 2 or 1 amino acid(s) difference(s) when compared with SEQ ID No: 21, the amino acid of the HCDR3 is as shown in SEQ ID No: 17 or has 3, 2 or 1 amino acid(s) difference(s) when compared with SEQ ID No: 17, and the heavy chain framework region(s) comprise(s) one or more back-mutation(s) selected from the group consisting of: 48I, 77T and 82A T; and/or

a light chain variable region, wherein the light chain variable region comprising: light chain LCDR1, LCDR2, LCDR3 and light chain framework region(s), wherein the amino acid sequence of the LCDR1 is as shown in SEQ ID No: 22 or has 3, 2 or 1 amino acid(s) difference(s) when compared with SEQ ID No: 22, the amino acid sequence of the LCDR2 is as shown in SEQ ID No: 19 or has 3, 2 or 1 amino acid(s) difference(s) when compared with SEQ ID No: 19, the amino acid of the LCDR3 is as shown in SEQ ID No: 23 or has 3, 2 or 1 amino acid(s) difference(s) when compared with SEQ ID No: 23, and the light chain framework region(s) comprise(s) one or more back-mutation(s) selected from the group consisting of: 4L, 9A, 22S, 58I, 60A and 68R; wherein, the back-mutation sites are numbered according to Kabat numbering criteria.

5. The anti-CD38 antibody or the antigen-binding fragment thereof according to claim 4, wherein the humanized antibody comprises: a heavy chain variable region as shown in SEQ ID Nos: 24, 32, or 37, or a variant thereof, wherein the variant has 1-10 amino acid mutation(s) on the framework regions of the heavy chain variable region as shown in SEQ ID Nos: 24, 32 or 37.

6. The anti-CD38 antibody or the antigen-binding fragment thereof according to claim 5, wherein the variant is selected from any one of the following (g) to (i): (g) one or more back-mutations selected from the the group consisting of 2F, 38K, 44S, 48I, 67A, 66K, 69L, 71V and 73Q on the framework regions of the heavy chain variable region as shown in SEQ ID No: 24;

(h) one or more back-mutations selected from the the group consisting of 79F and 91S on the framework regions of the heavy chain variable region as shown in SEQ ID No: 32; and
(i) back-mutation of 48I on the framework regions of the heavy chain variable region as shown in SEQ ID No: 37;

preferably, wherein the humanized antibody comprises:

a heavy chain variable region as shown in SEQ ID Nos: 26, 27, 28, 29, 34 or 39, or
a heavy chain variable region having at least 95% sequence identity to SEQ ID Nos: 26, 27, 28, 29, 34, or 39.

7. The anti-CD38 antibody or the antigen-binding fragment thereof according to claim 4, wherein the humanized antibody comprises a light chain variable region as shown in SEQ ID Nos: 25, 33 or 38 or a variant thereof, wherein the variant has 1-10 amino acid mutation(s) on the framework regions of the light chain variable region as shown in SEQ ID Nos: 25, 33 or 38.

8. The anti-CD38 antibody or the antigen-binding fragment thereof according to claim 7, wherein the variant is selected from any one of the following (j) to (1):

(j) one or more back-mutations selected from the the group consisting of 2F, 43S, 49K and 87F on the framework regions of the light chain variable region as shown in SEQ ID No: 25;
(k) one or more back-mutations selected from the the group consisting of 581, 68R and 85T on the framework regions of the light chain variable region as shown in SEQ ID No: 33;
(1) one or more back-mutations selected from the the group consisting of 4L, 9A, 22S, 581, 60A and 68R on the framework regions of the light chain variable region as shown in SEQ ID No: 38;

preferably, wherein the humanized antibody comprises:

a light chain variable region as shown in SEQ ID Nos: 30, 31, 35, 36, 40, 41 or 42, or
a light chain variable region having at least 95% sequence identity to SEQ ID Nos: 30, 31, 35, 36, 40, 41 or 42.

9. The anti-CD38 antibody or the antigen-binding fragment thereof according to claim 1, comprising:

(m) a heavy chain variable region as shown in SEQ ID Nos: 24, 26, 27, 28 or 29, and a light chain variable region as shown in SEQ ID Nos: 25, 30 or 31;
(n) a heavy chain variable region as shown in SEQ ID Nos: 32 or 34, and a light chain variable region as shown in SEQ ID No:33, 35 or 36; or
(o) a heavy chain variable region as shown in SEQ ID Nos: 37 or 39, and a light chain variable region as shown in SEQ ID No:38, 40, 41 or 42; preferably, the humanized antibody comprises:
(p) a heavy chain variable region as shown in SEQ ID No: 26 and a light chain variable region as shown in sequence SEQ ID No: 30; or
(q) a heavy chain variable region as shown in SEQ ID No: 32 and a light chain variable region as shown in SEQ ID No: 33; or
(r) a heavy chain variable region as shown in SEQ ID No: 37 and a light chain variable region as shown in SEQ ID No: 38.

10. The anti-CD38 antibody or the antigen-binding fragment thereof according to any one of claims 1 to 9, wherein the antibody comprises constant regions;
preferably, the antibody is chimeric antibody or humanized antibody, and the heavy chain constant region thereof is derived from human antibody IgG1, IgG2, IgG3 or IgG4, or a conventional variant of IgG1, IgG2, IgG3 or IgG4, and the light chain constant region thereof is derived from human antibody $\kappa$, $\lambda$ chain, or a conventional variant of $\kappa$, $\lambda$ chain; more preferably, the amino acid sequence of the heavy chain constant region is as shown in SEQ ID Nos: 43 or 44 or has at least 95% sequence identity to SEQ ID Nos: 43 or 44, more preferably, the amino acid sequence of the light chain constant region is as shown in SEQ ID No:45 or has at least 95% sequence identity to SEQ ID No:45.

11. The anti-CD38 antibody or the antigen-binding fragment thereof according to claim 10, comprising:

a heavy chain as shown in the amino acid sequence of SEQ ID Nos: 46, 48, 49, 51, 52 or 54 or having at least 85% sequence identity to the amino acid sequence of SEQ ID Nos: 46, 48, 49, 51, 52 or 54; and/or
a light chain as shown in the amino acid sequence of SEQ ID Nos: 47, 50 or 53, or having at least 85% sequence identity to the amino acid sequence of SEQ ID Nos: 47, 50 or 53.

12. The anti-CD38 antibody or the antigen-binding fragment thereof according to claim 11, wherein the antibody comprises:

> a heavy chain as shown in SEQ ID No: 46 and a light chain as shown in SEQ ID No: 47;
> a heavy chain as shown in SEQ ID No: 48 and a light chain as shown in SEQ ID No: 47;
> a heavy chain as shown in SEQ ID No: 49 and a light chain as shown in SEQ ID No: 50;
> a heavy chain as shown in SEQ ID No: 51 and a light chain as shown in SEQ ID No: 50;
> a heavy chain as shown in SEQ ID No: 52 and a light chain as shown in SEQ ID No: 53; or
> a heavy chain as shown in SEQ ID No: 54 and a light chain as shown in SEQ ID No: 53.

13. An isolated monoclonal antibody or an antigen-binding fragment thereof, which competes for binding to human CD38with the anti-CD38 antibody or the antigen-binding fragment thereof according to any one of claims 1 to 12 for binding to human CD38.

14. A pharmaceutical composition comprising:

> a therapeutically effective amount of the anti-CD38 antibody or the antigen-binding fragment thereof according to any one of claims 1 to 13, and
> one or more pharmaceutically acceptable carriers, diluents, buffers or excipients.

15. An isolated nucleic acid molecule encoding the anti-CD38 antibody or the antigen-binding fragment thereof according to any one of claims 1 to 13.

16. A vector comprising the nucleic acid molecule of claim 15.

17. A host cell comprising the vector of claim 16; preferably, the host cell is selected from the group consisting of a prokaryotic cell and a eukaryotic cell, preferably a eukaryotic cell, more preferably a mammalian cell.

18. A method for preparing the anti-CD38 antibody or the antigen-binding fragment thereof according to any one of claims 1 to 13, comprising the steps of:

> cultivating the host cells of claim 17,
> recovering the anti-CD38 antibody or the antigen-binding fragment thereof;
> optionally, purifying the anti-CD38 antibody or the antigen-binding fragment thereof.

19. A method for detecting or measuring human CD38, comprising:

> contacting the anti-CD38 antibody or the antigen-binding fragment thereof according to any one of claims 1 to 13 with a sample to be tested;
> determining the presence or level of human CD38 in the sample to be tested.

20. A reagent for detecting or measuring human CD38, comprising:

> the anti-CD38 antibody or the antigen-binding fragment thereof according to any one of claims 1 to 13; or
> the pharmaceutical composition of claim 16.

21. Use of a substance selected from any one of the following (m) to (o) in the preparation of a medicament for the treatment or prevention of disease or disorder:

> (m) the anti-CD38 antibody or the antigen-binding fragment thereof according to any one of claims 1 to 13,
> (n) the pharmaceutical composition of claim 14, and
> (o) the nucleic acid molecule of claim 15;

> preferably, the disease or disorder is tumor or immune disease.

22. The use according to claim 21, wherein the disease or disorder is CD38 positive disease or disorder.

23. The use according to claim 21 or 22, wherein:

the disease or disorder is tumor or immune disease,

the immune disease is selected from the group consisting of: rheumatoid arthritis, psoriasis, ankylosing spondylitis, joint psoriasis, dermatitis, systemic scleroderma and sclerosis, inflammatory bowel disease, Crohn's disease, ulcerative colitis, respiratory distress syndrome, meningitis, encephalitis, gastritis, uveitis, glomerulonephritis, eczema, asthma, arteriosclerosis, leukocyte adhesion deficiency, Raynaud syndrome, Sjogren syndrome, juvenile diabetes, Reiter disease, Behcet disease, immune complex nephritis, IgA nephropathy, IgM polyneuropathy, immune-mediated thrombocytopenia symptom, hemolytic anemia, myasthenia gravis, lupus nephritis, systemic lupus erythematosus, rheumatoid arthritis, atopic dermatitis, pemphigus, Graves disease, Hashimoto's thyroiditis, Wegener's granulomatosis, Omenn syndrome, chronic renal failure, acute infectious mononucleosis, multiple sclerosis, HIV and herpes virus-related diseases, severe acute respiratory syndrome and chorioretinitis, graft versus host disease, and immune disease caused by virus infection;

preferably, the autoimmune disease is selected from the group consisting of: rheumatoid arthritis, systemic lupus erythematosus, asthma, inflammatory bowel disease, multiple sclerosis, Crohn's disease, gastritis, Hashimoto's thyroiditis, ankylosing spondylitis, graft versus host disease, immune-mediated thrombocytopenia symptom;

preferably, the immune-mediated thrombocytopenia symptom is acute idiopathic thrombocytopenic purpura or chronic idiopathic thrombocytopenic purpura;

more preferably, the immune disease is rheumatoid arthritis;

the tumor is selected from the group consisting of: leukemia, B cell lymphoma, T cell lymphoma, NK cell lymphoma, plasma cell malignant tumor and myeloma;

preferably, the B cell lymphoma is selected from the group consisting of: mature B cell tumor, precursor B cell lymphoblastic leukemia/lymphoma, B cell non-Hodgkin's lymphoma and B cell Hodgkin's lymphoma;

more preferably, the tumor is selected from the group consisting of: acute lymphocytic leukemia, acute lymphoblastic leukemia, acute promyelocytic leukemia, chronic lymphocytic leukemia, acute or chronic myeloid leukemia, multiple myeloma, anterior medullary tumor, light chain amyloidosis, B cell chronic lymphocytic leukemia, small lymphocytic leukemia, B cell acute lymphocytic leukemia, B cell prelymphocytic leukemia, lymphoplasmacytoid lymphoma, mantle cell lymphoma, follicular lymphoma, cutaneous follicular central lymphoma, marginal zone B cell lymphoma, hairy cell leukemia, diffuse large B cell lymphoma, Burkitt lymphoma, plasma cell tumor, plasma cell myeloma, plasma cell leukemia, post-transplantation lymphoproliferative diseases, Waldenstrom macroglobulinemia, plasma cell leukemia and anaplastic large cell lymphoma and hairy cell lymphoma;

more preferably, the tumor is multiple myeloma.

**Figure 1A**

**Figure 1B**

Figure 2

**EP 3 851 456 A1**

## INTERNATIONAL SEARCH REPORT

International application No.

**PCT/CN2019/105119**

### A. CLASSIFICATION OF SUBJECT MATTER

C07K 16/28(2006.01)i; C12N 15/13(2006.01)i; C12N 15/63(2006.01)i; C12N 5/10(2006.01)i; G01N 33/53(2006.01)i; A61K 39/395(2006.01)i; A61P 35/00(2006.01)i; A61P 37/00(2006.01)i

According to International Patent Classification (IPC) or to both national classification and IPC

### B. FIELDS SEARCHED

Minimum documentation searched (classification system followed by classification symbols)

C07K; C12N; G01N; A61K; A61P

Documentation searched other than minimum documentation to the extent that such documents are included in the fields searched

Electronic data base consulted during the international search (name of data base and, where practicable, search terms used)

CNABS, CNTXT, CNKI and key words: CD38, 抗体, 等; DWPI, SIPOABS, WOTXT, EPTXT, USTXT, ISI_Web of Science and key words: CD38, antibody, et al.; Genbank; EMBL; 中国专利生物序列检索系统和检索的序列: SEQ ID NO: 9-23, NATIONAL BIO-SEQUENCE DATABASE OF CHINESE PATENT and Search sequence: SEQ ID NO: 9-23

### C. DOCUMENTS CONSIDERED TO BE RELEVANT

| Category* | Citation of document, with indication, where appropriate, of the relevant passages | Relevant to claim No. |
|---|---|---|
| A | CN 107921122 A (SORRENTO THERAPEUTICS INC.) 17 April 2018 (2018-04-17) claims 1-15 | 1-12, 14-23 |
| A | CN 107365385 A (TAKEDA PHARMACEUTICAL COMPANY LIMITED) 21 November 2017 (2017-11-21) claims 1-10 | 1-12, 14-23 |
| A | US 2009076249 A1 (MICHEL, D.W. et al.) 19 March 2009 (2009-03-19) claim 1, and description, paragraphs 1-141 | 1-12, 14-23 |

☐ Further documents are listed in the continuation of Box C.  ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **28 November 2019** | **10 December 2019** |

| Name and mailing address of the ISA/CN | Authorized officer |
|---|---|
| **China National Intellectual Property Administration**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

80

**INTERNATIONAL SEARCH REPORT**

| International application No. |
|---|
| **PCT/CN2019/105119** |

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑  forming part of the international application as filed:

        ☑  in the form of an Annex C/ST.25 text file.

        ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

## INTERNATIONAL SEARCH REPORT

| International application No. |
| --- |
| **PCT/CN2019/105119** |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **1-8、 10、 11、 13、 14-23**
because they relate to parts of the international application that do not comply with the prescribed requirements to such an extent that no meaningful international search can be carried out, specifically:

[1]   (1) An anti-CD38 antibody or an antigen-binding fragment thereof defined in claims 1-8, 10, 11 and 14-23 contains an HCDR variant or LCDR variant which has three, two or one amino acid difference with HCDR1-3 or LCDR1-3 defined a specific sequence, or definitions of a heavy chain and light chain of the anti-CD38 antibody or an antigen-binding fragment thereof comprise a case where they are defined based on a sequence identity percentage, or only one of the heavy chain or light chain of the antibody is specifically defined but the other chain does not defined, which all results in that the claims are not supported by the description, and the degree of non-compliance thereof results in that no meaningful search can be performed.

[2]   The international search will be carried out with regard to the subject matter that may be expected to be claimed, that is, the subject matter defined due to a fact that the protection scope of claim 1 excludes all CDR variants "with three, two or one amino acid difference", and the other subject matter thus defined due to referring to claim 1.

[3]   (2) Claim 13 only defines an antibody or an antigen-binding fragment thereof based on the binding activity of the antibody, but does not provide a technical feature of a chemical structure which is necessary to realize the result. A person skilled in the art needs undue experimentation to screen out an antibody or an antigen-binding fragment with the expected activity so that the claim is not supported by the description, and the degree of non-compliance thereof results in that no meaningful search is performed.

[4]   No international search will be performed on claim 13.

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

| International application No. |
| --- |
| **PCT/CN2019/105119** |

| Patent document cited in search report | | | Publication date (day/month/year) | Patent family member(s) | | | Publication date (day/month/year) |
| --- | --- | --- | --- | --- | --- | --- | --- |
| CN | 107921122 | A | 17 April 2018 | EP | 3280443 | A4 | 26 December 2018 |
| | | | | JP | 2018513149 | A | 24 May 2018 |
| | | | | AR | 104213 | A1 | 05 July 2017 |
| | | | | US | 2019048092 | A1 | 14 February 2019 |
| | | | | TW | 201702264 | A | 16 January 2017 |
| | | | | US | 2017291959 | A1 | 12 October 2017 |
| | | | | US | 10059774 | B2 | 28 August 2018 |
| | | | | US | 2016297888 | A1 | 13 October 2016 |
| | | | | EP | 3280443 | A2 | 14 February 2018 |
| | | | | WO | 2016164669 | A2 | 13 October 2016 |
| | | | | US | 9951144 | B2 | 24 April 2018 |
| | | | | WO | 2016164656 | A1 | 13 October 2016 |
| | | | | WO | 2016164669 | A3 | 12 January 2017 |
| CN | 107365385 | A | 21 November 2017 | EA | 201390993 | A1 | 30 December 2013 |
| | | | | JP | 6563446 | B2 | 21 August 2019 |
| | | | | EP | 2658871 | A1 | 06 November 2013 |
| | | | | US | 9790285 | B2 | 17 October 2017 |
| | | | | JP | 5843884 | B2 | 13 January 2016 |
| | | | | BR | 112013017009 | A2 | 25 July 2017 |
| | | | | HU | E038535 | T2 | 29 October 2018 |
| | | | | EP | 3284754 | A1 | 21 February 2018 |
| | | | | JP | 2014509837 | A | 24 April 2014 |
| | | | | JP | 2018029581 | A | 01 March 2018 |
| | | | | SG | 191211 | A1 | 31 July 2013 |
| | | | | MX | 350903 | B | 25 September 2017 |
| | | | | PL | 2658870 | T3 | 30 November 2018 |
| | | | | US | 10336833 | B2 | 02 July 2019 |
| | | | | US | 2015203587 | A1 | 23 July 2015 |
| | | | | US | 8362211 | B2 | 29 January 2013 |
| | | | | AR | 084747 | A1 | 05 June 2013 |
| | | | | EP | 2658870 | A1 | 06 November 2013 |
| | | | | EP | 3284755 | A1 | 21 February 2018 |
| | | | | KR | 20190015764 | A | 14 February 2019 |
| | | | | WO | 2012092612 | A1 | 05 July 2012 |
| | | | | NZ | 705848 | A | 29 July 2016 |
| | | | | US | 2015291702 | A1 | 15 October 2015 |
| | | | | TW | 201247705 | A | 01 December 2012 |
| | | | | CO | 6761368 | A2 | 30 September 2013 |
| | | | | KR | 101945002 | B1 | 07 February 2019 |
| | | | | KR | 20140032963 | A | 17 March 2014 |
| | | | | PL | 2658871 | T3 | 30 November 2018 |
| | | | | LT | 2658871 | T | 10 September 2018 |
| | | | | CA | 2822061 | A1 | 05 July 2012 |
| | | | | AU | 2011351921 | B2 | 13 April 2017 |
| | | | | MY | 160499 | A | 15 March 2017 |
| | | | | JP | 6148984 | B2 | 14 June 2017 |
| | | | | EP | 2658871 | B1 | 02 May 2018 |
| | | | | US | 2014155584 | A1 | 05 June 2014 |
| | | | | JP | 2018019689 | A | 08 February 2018 |
| | | | | ES | 2674175 | T3 | 27 June 2018 |

Form PCT/ISA/210 (patent family annex) (January 2015)

**INTERNATIONAL SEARCH REPORT**
Information on patent family members

| International application No. |
| --- |
| **PCT/CN2019/105119** |

| Patent document cited in search report | Publication date (day/month/year) | Patent family member(s) | | | Publication date (day/month/year) |
| --- | --- | --- | --- | --- | --- |
| | | AU | 2017204571 | B2 | 19 September 2019 |
| | | JP | 6425635 | B2 | 21 November 2018 |
| | | WO | 2012092616 | A1 | 05 July 2012 |
| | | ES | 2690095 | T3 | 19 November 2018 |
| | | EP | 2658870 | B1 | 25 April 2018 |
| | | CN | 103282383 | A | 04 September 2013 |
| | | PT | 2658870 | T | 03 July 2018 |
| | | MA | 34763 | B1 | 03 December 2013 |
| | | EA | 029303 | B1 | 30 March 2018 |
| | | ZA | 201304696 | B | 25 September 2014 |
| | | NZ | 612272 | A | 24 April 2015 |
| | | RS | 57526 | B1 | 31 October 2018 |
| US 2009076249 A1 | 19 March 2009 | None | | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006099875 A
- WO 2007042309 A
- WO 2008047242 A
- WO 2012092612 A
- WO 2016164656 A
- WO 2017149122 A
- WO 2008140603 A
- US 20080260731 A
- US 4683195 A
- US 6528624 B

### Non-patent literature cited in the description

- *J. biol. chem,* 1968, vol. 243, 3558
- **KABAT, EA et al.** Sequences of Proteins of Immunological Interest. 1991
- **BIRD et al.** *Science,* 1988, vol. 242, 423-426
- **HUSTON et al.** *Proc. Natl. Acad. Sci USA,* 1988, vol. 85, 5879-5883
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS et al.** *Cancer Immunol.,* 2001
- *Protein Engineering,* 1994, vol. 7, 697
- **KABAT et al.** Sequences of Proteins of Immunological Interest. Public Health Service, National Institutes of Health, 1991
- **AL-LAZIKANI et al.** *JMB,* 1997, vol. 273, 927-948
- **LEFRANC MP.** *Immunologist,* 1999, vol. 7, 132-136
- **LEFRANC, MP.** *Dev. Comp. Immunol.,* 2003, vol. 27, 55-77
- Epitope Mapping Protocols in Methods in Molecular Biology. 1996, vol. 66
- **STAHLI et al.** *Methods in Enzymology,* 1983, 242-253
- **KIRKLAND et al.** *J. Immunol.,* 1986, vol. 137, 3614-3619
- **CHEUNG et al.** *Virology,* 1990, vol. 176, 546-552
- **HARLOW ; LANE.** Antibodies, A Laboratory Manual. Cold Spring Harbor Press, 1988
- **MOREL et al.** *Molec. Immunol.,* 1988, vol. 25, 7-15
- **MOLDENHAUER et al.** *Scand. J. Immunol.,* 1990, vol. 32, 77-82
- A Laboratory Manual for Antibodies. Cold Spring Harbor
- The Immunoglobulin Facts Book. 2001
- **WATSON et al.** Molecular Biology of the Gene. The Benjamin/Cummings Pub. Co, 1987, 224
- BLAST ALGORITHMS. **ALTSCHUL, SF et al.** J. Mol. Biol. 1990, vol. 215, 403-410
- **GISH, W. et al.** *Nature Genet.,* 1993, vol. 3, 266-272
- **MADDEN, TL et al.** *Meth. Enzymol.,* 1996, vol. 266, 131-141
- **ALTSCHUL, SF et al.** *Nucleic Acids Res.,* 1997, vol. 25, 3389-3402
- **ZHANG, J. et al.** *Genome Res.,* 1997, vol. 7, 649-656
- **MULLIS et al.** Cold Spring Harbor Symp. Ouant. Biol. 1987, vol. 51, 263
- PCR TECHNOLOGY. Stockton Press, 1989
- **SAMBROOK et al.** Antibodies Laboratory Manual, Molecular Cloning. Cold Spring Harbor Laboratory
- *WHO Drug Information,* 2010, vol. 24 (1

**Sequence listing**

<110>    JIANGSU HENGRUI MEDICINE CO., LTD.;
SHANGHAI HENGRUI PHARMACEUTICAL CO., LTD.

<120>    ANTI-CD38 ANTIBODY, ANTIGEN BINDING FRAGMENT THEREOF,
 AND PHARMACEUTICAL USE

<140> PCT/CN2019/105119
<141> 2019-09-10

<150>    201811060341.2
<151>    2018-09-11

<160>    57

<170>    SIPOSequenceListing 1.0

<210>    1
<211>    491
<212>    PRT
<213>    Artificial Sequence

<220>
<221>    PEPTIDE
<223>    Fusion protein of CD38 extracellular domain
         and mouse IgG2a-Fc:CD38-ECD-mFc

<400>    1
Val Pro Arg Trp Arg Gln Gln Trp Ser Gly Pro Gly Thr Thr Lys Arg
1               5                   10                  15
Phe Pro Glu Thr Val Leu Ala Arg Cys Val Lys Tyr Thr Glu Ile His
            20                  25                  30
Pro Glu Met Arg His Val Asp Cys Gln Ser Val Trp Asp Ala Phe Lys
        35                  40                  45
Gly Ala Phe Ile Ser Lys His Pro Cys Asn Ile Thr Glu Glu Asp Tyr
    50                  55                  60
Gln Pro Leu Met Lys Leu Gly Thr Gln Thr Val Pro Cys Asn Lys Ile
65                  70                  75                  80
Leu Leu Trp Ser Arg Ile Lys Asp Leu Ala His Gln Phe Thr Gln Val
                85                  90                  95
Gln Arg Asp Met Phe Thr Leu Glu Asp Thr Leu Leu Gly Tyr Leu Ala
            100                 105                 110
Asp Asp Leu Thr Trp Cys Gly Glu Phe Asn Thr Ser Lys Ile Asn Tyr
        115                 120                 125
Gln Ser Cys Pro Asp Trp Arg Lys Asp Cys Ser Asn Asn Pro Val Ser
    130                 135                 140
Val Phe Trp Lys Thr Val Ser Arg Arg Phe Ala Glu Ala Ala Cys Asp
145                 150                 155                 160
Val Val His Val Met Leu Asn Gly Ser Arg Ser Lys Ile Phe Asp Lys
                165                 170                 175
Asn Ser Thr Phe Gly Ser Val Glu Val His Asn Leu Gln Pro Glu Lys
            180                 185                 190
Val Gln Thr Leu Glu Ala Trp Val Ile His Gly Gly Arg Glu Asp Ser
            195                 200                 205
Arg Asp Leu Cys Gln Asp Pro Thr Ile Lys Glu Leu Glu Ser Ile Ile
    210                 215                 220
Ser Lys Arg Asn Ile Gln Phe Ser Cys Lys Asn Ile Tyr Arg Pro Asp
225                 230                 235                 240
Lys Phe Leu Gln Cys Val Lys Asn Pro Glu Asp Ser Ser Cys Thr Ser
                245                 250                 255
Glu Ile Glu Pro Arg Gly Pro Thr Ile Lys Pro Cys Pro Pro Cys Lys
                260                 265                 270

```
Cys Pro Ala Pro Asn Leu Leu Gly Gly Pro Ser Val Phe Ile Phe Pro
        275                 280             285
Pro Lys Ile Lys Asp Val Leu Met Ile Ser Leu Ser Pro Ile Val Thr
    290                 295             300
Cys Val Val Val Asp Val Ser Glu Asp Asp Pro Asp Val Gln Ile Ser
305                 310                 315                 320
Trp Phe Val Asn Asn Val Glu Val His Thr Ala Gln Thr Gln Thr His
            325                 330                 335
Arg Glu Asp Tyr Asn Ser Thr Leu Arg Val Val Ser Ala Leu Pro Ile
            340                 345                 350
Gln His Gln Asp Trp Met Ser Gly Lys Glu Phe Lys Cys Lys Val Asn
        355                 360                 365
Asn Lys Asp Leu Pro Ala Pro Ile Glu Arg Thr Ile Ser Lys Pro Lys
        370                 375                 380
Gly Ser Val Arg Ala Pro Gln Val Tyr Val Leu Pro Pro Pro Glu Glu
385                 390                 395                 400
Glu Met Thr Lys Lys Gln Val Thr Leu Thr Cys Met Val Thr Asp Phe
            405                 410                 415
Met Pro Glu Asp Ile Tyr Val Glu Trp Thr Asn Asn Gly Lys Thr Glu
            420                 425                 430
Leu Asn Tyr Lys Asn Thr Glu Pro Val Leu Asp Ser Asp Gly Ser Tyr
        435                 440                 445
Phe Met Tyr Ser Lys Leu Arg Val Glu Lys Lys Asn Trp Val Glu Arg
    450                 455                 460
Asn Ser Tyr Ser Cys Ser Val Val His Glu Gly Leu His Asn His His
465                 470                 475                 480
Thr Thr Lys Ser Phe Ser Arg Thr Pro Gly Lys
                485                 490
```

<210> 2
<211> 490
<212> PRT
<213> Artificial Sequence

<220>
<221> PEPTIDE
<223> Fusion protein of CD38 extracellular domain and human
IgG1Fc

<400> 2
```
Val Pro Arg Trp Arg Gln Gln Trp Ser Gly Pro Gly Thr Thr Lys Arg
1               5                   10                  15
Phe Pro Glu Thr Val Leu Ala Arg Cys Val Lys Tyr Thr Glu Ile His
            20                  25                  30
Pro Glu Met Arg His Val Asp Cys Gln Ser Val Trp Asp Ala Phe Lys
        35                  40                  45
Gly Ala Phe Ile Ser Lys His Pro Cys Asn Ile Thr Glu Glu Asp Tyr
    50                  55                  60
Gln Pro Leu Met Lys Leu Gly Thr Gln Thr Val Pro Cys Asn Lys Ile
65                  70                  75                  80
Leu Leu Trp Ser Arg Ile Lys Asp Leu Ala His Gln Phe Thr Gln Val
                85                  90                  95
Gln Arg Asp Met Phe Thr Leu Glu Asp Thr Leu Leu Gly Tyr Leu Ala
        100                 105                 110
Asp Asp Leu Thr Trp Cys Gly Glu Phe Asn Thr Ser Lys Ile Asn Tyr
    115                 120                 125
Gln Ser Cys Pro Asp Trp Arg Lys Asp Cys Ser Asn Asn Pro Val Ser
    130                 135                 140
Val Phe Trp Lys Thr Val Ser Arg Arg Phe Ala Glu Ala Ala Cys Asp
145                 150                 155                 160
Val Val His Val Met Leu Asn Gly Ser Arg Ser Lys Ile Phe Asp Lys
                165                 170                 175
```

44

```
Asn Ser Thr Phe Gly Ser Val Glu Val His Asn Leu Gln Pro Glu Lys
            180                 185                 190
Val Gln Thr Leu Glu Ala Trp Val Ile His Gly Gly Arg Glu Asp Ser
            195                 200                 205
Arg Asp Leu Cys Gln Asp Pro Thr Ile Lys Glu Leu Glu Ser Ile Ile
    210                 215                 220
Ser Lys Arg Asn Ile Gln Phe Ser Cys Lys Asn Ile Tyr Arg Pro Asp
225                 230                 235                 240
Lys Phe Leu Gln Cys Val Lys Asn Pro Glu Asp Ser Ser Cys Thr Ser
                245                 250                 255
Glu Ile Glu Pro Lys Ser Ser Asp Lys Thr His Thr Cys Pro Pro Cys
                260                 265                 270
Pro Ala Pro Glu Leu Leu Gly Gly Pro Ser Val Phe Leu Phe Pro Pro
        275                 280                 285
Lys Pro Lys Asp Thr Leu Met Ile Ser Arg Thr Pro Glu Val Thr Cys
        290                 295                 300
Val Val Val Asp Val Ser His Glu Asp Pro Glu Val Lys Phe Asn Trp
305                 310                 315                 320
Tyr Val Asp Gly Val Glu Val His Asn Ala Lys Thr Lys Pro Arg Glu
                325                 330                 335
Glu Gln Tyr Asn Ser Thr Tyr Arg Val Val Ser Val Leu Thr Val Leu
                340                 345                 350
His Gln Asp Trp Leu Asn Gly Lys Glu Tyr Lys Cys Lys Val Ser Asn
        355                 360                 365
Lys Ala Leu Pro Ala Pro Ile Glu Lys Thr Ile Ser Lys Ala Lys Gly
    370                 375                 380
Gln Pro Arg Glu Pro Gln Val Tyr Thr Leu Pro Pro Ser Arg Asp Glu
385                 390                 395                 400
Leu Thr Lys Asn Gln Val Ser Leu Thr Cys Leu Val Lys Gly Phe Tyr
                405                 410                 415
Pro Ser Asp Ile Ala Val Glu Trp Glu Ser Asn Gly Gln Pro Glu Asn
            420                 425                 430
Asn Tyr Lys Thr Thr Pro Pro Val Leu Asp Ser Asp Gly Ser Phe Phe
        435                 440                 445
Leu Tyr Ser Lys Leu Thr Val Asp Lys Ser Arg Trp Gln Gln Gly Asn
    450                 455                 460
Val Phe Ser Cys Ser Val Met His Glu Ala Leu His Asn His Tyr Thr
465                 470                 475                 480
Gln Lys Ser Leu Ser Leu Ser Pro Gly Lys
                485                 490
```

```
<210>   3
<211>   118
<212>   PRT
<213>   Mus musculus

<220>
<221>   DOMAIN
<223>   m009 heavy chain variable region sequence


<400>   3
Glu Phe Gln Leu Gln Gln Ser Gly Pro Glu Leu Val Lys Pro Gly Ala
1               5                   10                  15
Ser Val Lys Ile Ser Cys Lys Ala Ser Gly Tyr Ser Phe Thr Asp Tyr
            20                  25                  30
Asn Leu Asn Trp Val Lys Gln Ser Asn Gly Lys Ser Leu Glu Trp Ile
        35                  40                  45
Gly Val Ile Asn Pro Lys Tyr Asp Ala Ile Asn Tyr Asn Gln Lys Phe
    50                  55                  60
Lys Asp Lys Ala Thr Leu Thr Val Asp Gln Ser Ser Ser Thr Ala Tyr
65                  70                  75                  80
Met Gln Leu Ser Ser Leu Thr Ser Glu Asp Ser Ala Val Tyr Tyr Cys
```

```
                        85                      90                      95
        Ala Arg Glu Gly Trp Gly Lys Ala Leu Asp Tyr Trp Gly Pro Gly Thr
                    100                     105                     110
        Ser Val Ile Val Ser Ser
                    115


        <210>   4
        <211>   107
        <212>   PRT
        <213>   Mus musculus

        <220>
        <221>   DOMAIN
        <223>   m009 light chain variable region sequence


        <400>   4
        Asp Phe Val Leu Thr Gln Ser Pro Ala Thr Leu Ser Val Thr Pro Gly
        1               5                   10                      15
        Asp Ser Val Ser Leu Ser Cys Arg Ala Ser Gln Ser Ile Tyr Thr Asn
                    20                      25                      30
        Leu His Trp Tyr Gln Gln Lys Ser His Glu Ser Pro Arg Leu Leu Ile
                    35                      40                      45
        Lys Tyr Ala Ser Gln Ser Ile Ser Gly Ile Pro Ser Arg Phe Ser Gly
                50                      55                      60
        Ser Gly Ser Gly Thr Asp Phe Thr Leu Ser Ile Asn Ser Val Glu Thr
        65                      70                      75                      80
        Glu Asp Ser Gly Met Tyr Phe Cys Gln Gln Ser Asn Ser Trp Pro Leu
                        85                      90                      95
        Thr Phe Gly Ala Gly Thr Lys Leu Glu Leu Lys
                    100                     105


        <210>   5
        <211>   120
        <212>   PRT
        <213>   Mus musculus

        <220>
        <221>   DOMAIN
        <223>   m011 heavy chain variable region sequence


        <400>   5
        Glu Val Gln Leu Val Glu Ser Gly Gly Gly Leu Val Lys Pro Gly Gly
        1               5                   10                      15
        Ser Leu Lys Leu Ser Cys Ala Ala Ser Gly Phe Thr Phe Ser Asp Tyr
                    20                      25                      30
        Gly Met His Trp Val Arg Gln Ala Pro Glu Lys Gly Leu Glu Trp Val
                    35                      40                      45
        Ala Phe Ile Ser Ser Gly Ser Ser Ser Ile Tyr Tyr Ala Asp Thr Val
                50                      55                      60
        Lys Gly Arg Phe Thr Ile Ser Arg Asp Asn Ala Lys Asn Thr Leu Phe
        65                      70                      75                      80
        Leu Gln Met Thr Ser Leu Arg Ser Glu Asp Thr Ala Met Tyr Ser Cys
                        85                      90                      95
        Ala Arg Asn Tyr Val Ser Ser Tyr Gly Tyr Phe Asp Tyr Trp Gly Gln
                    100                     105                     110
        Gly Thr Thr Leu Thr Val Ser Ser
                    115                     120


        <210>   6
        <211>   111
        <212>   PRT
```

<213> Mus musculus

<220>
<221> DOMAIN
<223> m011 light chain variable region sequence

<400> 6
Asp Ile Val Met Thr Gln Ser Pro Ala Ser Leu Ala Val Ser Leu Gly
1               5                   10                  15
Gln Arg Ala Thr Ile Ser Cys Arg Ala Ser Glu Asn Val Asp Asn Tyr
            20                  25                  30
Gly Ile Ser Phe Met His Trp Tyr Gln Gln Lys Pro Gly Gln Pro Pro
        35                  40                  45
Lys Leu Leu Ile Tyr Arg Ala Ser Asn Leu Glu Ser Gly Ile Pro Ala
    50                  55                  60
Arg Phe Ser Gly Ser Gly Ser Arg Thr Asp Phe Thr Leu Thr Ile Asn
65                  70                  75                  80
Pro Val Glu Thr Asp Asp Val Ala Thr Tyr Tyr Cys Gln Gln Ser Asn
                85                  90                  95
Lys Asp Pro Leu Thr Phe Gly Ser Gly Thr Lys Leu Glu Ile Lys
            100                 105                 110

<210> 7
<211> 120
<212> PRT
<213> Mus musculus

<220>
<221> DOMAIN
<223> m160 heavy chain variable region sequence

<400> 7
Glu Val Gln Leu Val Glu Ser Gly Gly Gly Leu Val Lys Pro Gly Gly
1               5                   10                  15
Ser Leu Lys Leu Ser Cys Val Ala Ser Gly Phe Thr Phe Ser Asp Tyr
            20                  25                  30
Gly Met His Trp Val Arg Gln Ala Pro Glu Lys Gly Leu Glu Trp Ile
        35                  40                  45
Ala Phe Ile Ser Thr Gly Ser Ser Asn Ile Tyr Tyr Val Asp Lys Val
    50                  55                  60
Lys Gly Arg Phe Thr Ile Ser Arg Asp Asn Ala Lys Asn Thr Leu Phe
65                  70                  75                  80
Leu Gln Met Thr Ser Leu Arg Ser Glu Asp Thr Ala Met Tyr Tyr Cys
                85                  90                  95
Ala Arg Asn Tyr Val Ser Ser Tyr Gly Tyr Phe Asp Tyr Trp Gly Gln
            100                 105                 110
Gly Thr Thr Leu Thr Val Ser Ser
        115                 120

<210> 8
<211> 111
<212> PRT
<213> Mus musculus

<220>
<221> DOMAIN
<223> m160 light chain variable region sequence

<400> 8
Asp Ile Val Leu Thr Gln Ser Pro Ala Ser Leu Ala Val Ser Leu Gly

```
1                       5                       10                      15
Gln Arg Ala Thr Val Ser Cys Arg Ala Ser Glu Ser Val Asp Asn Tyr
                20                      25                      30
Gly Ile Ser Phe Met His Trp Tyr Gln Gln Lys Pro Gly Gln Pro Pro
                35                      40                      45
Lys Leu Leu Ile Tyr Arg Ala Ser Asn Leu Glu Ser Gly Ile Pro Ala
            50                      55                      60
Arg Phe Ser Gly Ser Gly Ser Arg Thr Asp Phe Thr Leu Thr Ile Asn
65                      70                      75                      80
Pro Val Glu Thr Asp Asp Val Ala Thr Tyr Tyr Cys Gln Gln Thr Asn
                85                      90                      95
Lys Asp Pro Leu Thr Phe Gly Gly Gly Thr Lys Leu Glu Leu Lys
            100                     105                     110
```

<210> 9
<211> 5
<212> PRT
<213> Mus musculus

<220>
<221> DOMAIN
<223> m009 HCDR1


<400> 9
```
Asp Tyr Asn Leu Asn
1               5
```

<210> 10
<211> 17
<212> PRT
<213> Mus musculus

<220>
<221> DOMAIN
<223> m009 HCDR2


<400> 10
```
Val Ile Asn Pro Lys Tyr Asp Ala Ile Asn Tyr Asn Gln Lys Phe Lys
1               5                       10                      15
Asp
```

<210> 11
<211> 9
<212> PRT
<213> Mus musculus

<220>
<221> DOMAIN
<223> m009 HCDR3


<400> 11
```
Glu Gly Trp Gly Lys Ala Leu Asp Tyr
1               5
```

<210> 12
<211> 11
<212> PRT
<213> Mus musculus

<220>
<221> DOMAIN
<223> m009 LCDR1

<400> 12
Arg Ala Ser Gln Ser Ile Tyr Thr Asn Leu His
1               5                   10

<210> 13
<211> 7
<212> PRT
<213> Mus musculus

<220>
<221> DOMAIN
<223> m009 LCDR2

<400> 13
Tyr Ala Ser Gln Ser Ile Ser
1               5

<210> 14
<211> 9
<212> PRT
<213> Mus musculus

<220>
<221> DOMAIN
<223> m009 LCDR3

<400> 14
Gln Gln Ser Asn Ser Trp Pro Leu Thr
1               5

<210> 15
<211> 5
<212> PRT
<213> Mus musculus

<220>
<221> DOMAIN
<223> m011 HCDR1

<400> 15
Asp Tyr Gly Met His
1               5

<210> 16
<211> 17
<212> PRT
<213> Mus musculus

<220>
<221> DOMAIN
<223> m011 HCDR2

<400> 16
Phe Ile Ser Ser Gly Ser Ser Ser Ile Tyr Tyr Ala Asp Thr Val Lys

```
                1                    5                    10                   15
                Gly


                <210>  17
                <211>  11
                <212>  PRT
                <213>  Mus musculus


                <220>
                <221>  DOMAIN
                <223>  m011 HCDR3


                <400>  17
                Asn Tyr Val Ser Ser Tyr Gly Tyr Phe Asp Tyr
                1                    5                    10


                <210>  18
                <211>  15
                <212>  PRT
                <213>  Mus musculus


                <220>
                <221>  DOMAIN
                <223>  m011 LCDR1


                <400>  18
                Arg Ala Ser Glu Asn Val Asp Asn Tyr Gly Ile Ser Phe Met His
                1                    5                    10                   15


                <210>  19
                <211>  7
                <212>  PRT
                <213>  Mus musculus


                <220>
                <221>  DOMAIN
                <223>  m011 LCDR2


                <400>  19
                Arg Ala Ser Asn Leu Glu Ser
                1                    5


                <210>  20
                <211>  9
                <212>  PRT
                <213>  Mus musculus


                <220>
                <221>  DOMAIN
                <223>  m011 LCDR3


                <400>  20
                Gln Gln Ser Asn Lys Asp Pro Leu Thr
                1                    5


                <210>  21
                <211>  17
                <212>  PRT
```

<213> Mus musculus

<220>
<221> DOMAIN
<223> m160 HCDR2

<400> 21
Phe Ile Ser Thr Gly Ser Ser Asn Ile Tyr Tyr Val Asp Lys Val Lys
1               5                   10                  15
Gly

<210> 22
<211> 15
<212> PRT
<213> Mus musculus

<220>
<221> DOMAIN
<223> m160 LCDR1

<400> 22
Arg Ala Ser Glu Ser Val Asp Asn Tyr Gly Ile Ser Phe Met His
1               5                   10                  15

<210> 23
<211> 9
<212> PRT
<213> Mus musculus

<220>
<221> DOMAIN
<223> m160 LCDR3

<400> 23
Gln Gln Thr Asn Lys Asp Pro Leu Thr
1               5

<210> 24
<211> 118
<212> PRT
<213> Artificial Sequence

<220>
<221> DOMAIN
<223> h009 VH1

<400> 24
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Asn Leu Asn Trp Val Arg Gln Ala Pro Gly Gln Arg Leu Glu Trp Met
        35                  40                  45
Gly Val Ile Asn Pro Lys Tyr Asp Ala Ile Asn Tyr Asn Gln Lys Phe
    50                  55                  60
Lys Asp Arg Val Thr Ile Thr Arg Asp Thr Ser Ala Ser Thr Ala Tyr
65                  70                  75                  80
Met Glu Leu Ser Ser Leu Arg Ser Glu Asp Thr Ala Val Tyr Tyr Cys

```
                        85                      90                      95
        Ala Arg Glu Gly Trp Gly Lys Ala Leu Asp Tyr Trp Gly Gln Gly Thr
                        100                     105                     110
        Leu Val Thr Val Ser Ser
                        115


        <210>   25
        <211>   107
        <212>   PRT
        <213>   Artificial Sequence

        <220>
        <221>   DOMAIN
        <223>   h009 VL1


        <400>   25
        Glu Ile Val Leu Thr Gln Ser Pro Ala Thr Leu Ser Leu Ser Pro Gly
        1                   5                   10                      15
        Glu Arg Ala Thr Leu Ser Cys Arg Ala Ser Gln Ser Ile Tyr Thr Asn
                        20                      25                      30
        Leu His Trp Tyr Gln Gln Lys Pro Gly Gln Ala Pro Arg Leu Leu Ile
                        35                      40                      45
        Tyr Tyr Ala Ser Gln Ser Ile Ser Gly Ile Pro Ala Arg Phe Ser Gly
                50                      55                      60
        Ser Gly Ser Gly Thr Asp Phe Thr Leu Thr Ile Ser Ser Leu Glu Pro
        65                      70                      75                      80
        Glu Asp Phe Ala Val Tyr Tyr Cys Gln Gln Ser Asn Ser Trp Pro Leu
                        85                      90                      95
        Thr Phe Gly Gly Gly Thr Lys Val Glu Ile Lys
                        100                     105


        <210>   26
        <211>   118
        <212>   PRT
        <213>   Artificial Sequence

        <220>
        <221>   DOMAIN
        <223>   h009 VH2


        <400>   26
        Glu Phe Gln Leu Val Gln Ser Gly Ala Glu Val Lys Lys Pro Gly Ala
        1                   5                   10                      15
        Ser Val Lys Val Ser Cys Lys Ala Ser Gly Tyr Thr Phe Thr Asp Tyr
                        20                      25                      30
        Asn Leu Asn Trp Val Arg Gln Ala Pro Gly Gln Ser Leu Glu Trp Met
                        35                      40                      45
        Gly Val Ile Asn Pro Lys Tyr Asp Ala Ile Asn Tyr Asn Gln Lys Phe
                50                      55                      60
        Lys Asp Arg Val Thr Ile Thr Val Asp Thr Ser Ala Ser Thr Ala Tyr
        65                      70                      75                      80
        Met Glu Leu Ser Ser Leu Arg Ser Glu Asp Thr Ala Val Tyr Tyr Cys
                        85                      90                      95
        Ala Arg Glu Gly Trp Gly Lys Ala Leu Asp Tyr Trp Gly Gln Gly Thr
                        100                     105                     110
        Leu Val Thr Val Ser Ser
                        115


        <210>   27
        <211>   118
        <212>   PRT
```

<213> Artificial Sequence

<220>
<221> DOMAIN
<223> h009 VH3

<400> 27
Glu Phe Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Asn Leu Asn Trp Val Arg Gln Ala Pro Gly Gln Ser Leu Glu Trp Ile
        35                  40                  45
Gly Val Ile Asn Pro Lys Tyr Asp Ala Ile Asn Tyr Asn Gln Lys Phe
        50                  55                  60
Lys Asp Arg Ala Thr Ile Thr Val Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Glu Gly Trp Gly Lys Ala Leu Asp Tyr Trp Gly Gln Gly Thr
                100                 105                 110
Leu Val Thr Val Ser Ser
                115

<210> 28
<211> 118
<212> PRT
<213> Artificial Sequence

<220>
<221> DOMAIN
<223> h009 VH4

<400> 28
Glu Phe Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Asn Leu Asn Trp Val Lys Gln Ala Pro Gly Gln Ser Leu Glu Trp Met
        35                  40                  45
Gly Val Ile Asn Pro Lys Tyr Asp Ala Ile Asn Tyr Asn Gln Lys Phe
        50                  55                  60
Lys Asp Lys Val Thr Leu Thr Val Asp Gln Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Glu Gly Trp Gly Lys Ala Leu Asp Tyr Trp Gly Gln Gly Thr
                100                 105                 110
Leu Val Thr Val Ser Ser
                115

<210> 29
<211> 118
<212> PRT
<213> Artificial Sequence

<220>
<221> DOMAIN
<223> h009 VH5

<400> 29
Glu Phe Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Asn Leu Asn Trp Val Lys Gln Ala Pro Gly Gln Ser Leu Glu Trp Ile
        35                  40                  45
Gly Val Ile Asn Pro Lys Tyr Asp Ala Ile Asn Tyr Asn Gln Lys Phe
    50                  55                  60
Lys Asp Lys Ala Thr Leu Thr Val Asp Gln Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Glu Gly Trp Gly Lys Ala Leu Asp Tyr Trp Gly Gln Gly Thr
            100                 105                 110
Leu Val Thr Val Ser Ser
            115


<210> 30
<211> 107
<212> PRT
<213> Artificial Sequence

<220>
<221> DOMAIN
<223> h009 VL2


<400> 30
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Ser Ile Tyr Thr Asn
            20                  25                  30
Leu His Trp Tyr Gln Gln Lys Pro Gly Gln Ser Pro Arg Leu Leu Ile
        35                  40                  45
Lys Tyr Ala Ser Gln Ser Ile Ser Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys Gln Gln Ser Asn Ser Trp Pro Leu
                85                  90                  95
Thr Phe Gly Gly Gly Thr Lys Val Glu Ile Lys
            100                 105


<210> 31
<211> 107
<212> PRT
<213> Artificial Sequence

<220>
<221> DOMAIN
<223> h009 VL3


<400> 31
Glu Phe Val Leu Thr Gln Ser Pro Ala Thr Leu Ser Leu Ser Pro Gly
1               5                   10                  15
Glu Arg Ala Thr Leu Ser Cys Arg Ala Ser Gln Ser Ile Tyr Thr Asn
            20                  25                  30
Leu His Trp Tyr Gln Gln Lys Pro Gly Gln Ser Pro Arg Leu Leu Ile
        35                  40                  45
Lys Tyr Ala Ser Gln Ser Ile Ser Gly Ile Pro Ala Arg Phe Ser Gly
    50                  55                  60

Ser Gly Ser Gly Thr Asp Phe Thr Leu Thr Ile Ser Ser Leu Glu Pro
65          70              75              80
Glu Asp Phe Ala Val Tyr Phe Cys Gln Gln Ser Asn Ser Trp Pro Leu
            85              90              95
Thr Phe Gly Gly Gly Thr Lys Val Glu Ile Lys
            100             105

<210> 32
<211> 120
<212> PRT
<213> Artificial Sequence

<220>
<221> DOMAIN
<223> h011 VH1


<400> 32
Glu Val Gln Leu Val Glu Ser Gly Gly Gly Leu Val Gln Pro Gly Gly
1               5               10              15
Ser Leu Arg Leu Ser Cys Ala Ala Ser Gly Phe Thr Phe Ser Asp Tyr
            20              25              30
Gly Met His Trp Val Arg Gln Ala Pro Gly Lys Gly Leu Glu Trp Val
        35              40              45
Ala Phe Ile Ser Ser Gly Ser Ser Ser Ile Tyr Tyr Ala Asp Thr Val
        50              55              60
Lys Gly Arg Phe Thr Ile Ser Arg Asp Asn Ala Lys Ser Ser Leu Tyr
65          70              75              80
Leu Gln Met Thr Ser Leu Arg Ala Glu Asp Thr Ala Val Tyr Tyr Cys
            85              90              95
Ala Arg Asn Tyr Val Ser Ser Tyr Gly Tyr Phe Asp Tyr Trp Gly Gln
            100             105             110
Gly Thr Thr Val Thr Val Ser Ser
        115             120

<210> 33
<211> 111
<212> PRT
<213> Artificial Sequence

<220>
<221> DOMAIN
<223> h011 VL1


<400> 33
Asp Ile Val Met Thr Gln Ser Pro Asp Ser Leu Ala Val Ser Leu Gly
1               5               10              15
Glu Arg Ala Thr Ile Asn Cys Arg Ala Ser Glu Asn Val Asp Asn Tyr
            20              25              30
Gly Ile Ser Phe Met His Trp Tyr Gln Gln Lys Pro Gly Gln Pro Pro
        35              40              45
Lys Leu Leu Ile Tyr Arg Ala Ser Asn Leu Glu Ser Gly Val Pro Asp
        50              55              60
Arg Phe Ser Gly Ser Gly Ser Gly Thr Asp Phe Thr Leu Thr Ile Ser
65          70              75              80
Ser Leu Gln Ala Glu Asp Val Ala Val Tyr Tyr Cys Gln Gln Ser Asn
            85              90              95
Lys Asp Pro Leu Thr Phe Gly Gly Gly Thr Lys Val Glu Ile Lys
            100             105             110

<210> 34
<211> 120

```
<212>    PRT
<213>    Artificial Sequence

<220>
<221>    DOMAIN
<223>    h011 VH2


<400>    34
Glu Val Gln Leu Val Glu Ser Gly Gly Gly Leu Val Gln Pro Gly Gly
1                   5                   10                  15
Ser Leu Arg Leu Ser Cys Ala Ala Ser Gly Phe Thr Phe Ser Asp Tyr
            20                  25                  30
Gly Met His Trp Val Arg Gln Ala Pro Gly Lys Gly Leu Glu Trp Val
        35                  40                  45
Ala Phe Ile Ser Ser Gly Ser Ser Ile Tyr Tyr Ala Asp Thr Val
        50                  55                  60
Lys Gly Arg Phe Thr Ile Ser Arg Asp Asn Ala Lys Ser Ser Leu Phe
65                  70                  75                  80
Leu Gln Met Thr Ser Leu Arg Ala Glu Asp Thr Ala Val Tyr Ser Cys
                85                  90                  95
Ala Arg Asn Tyr Val Ser Ser Tyr Gly Tyr Phe Asp Tyr Trp Gly Gln
            100                 105                 110
Gly Thr Thr Val Thr Val Ser Ser
            115                 120


<210>    35
<211>    111
<212>    PRT
<213>    Artificial Sequence

<220>
<221>    DOMAIN
<223>    h011 VL2


<400>    35
Asp Ile Val Met Thr Gln Ser Pro Asp Ser Leu Ala Val Ser Leu Gly
1                   5                   10                  15
Glu Arg Ala Thr Ile Asn Cys Arg Ala Ser Glu Asn Val Asp Asn Tyr
            20                  25                  30
Gly Ile Ser Phe Met His Trp Tyr Gln Gln Lys Pro Gly Gln Pro Pro
        35                  40                  45
Lys Leu Leu Ile Tyr Arg Ala Ser Asn Leu Glu Ser Gly Val Pro Asp
        50                  55                  60
Arg Phe Ser Gly Ser Gly Ser Arg Thr Asp Phe Thr Leu Thr Ile Ser
65                  70                  75                  80
Ser Leu Gln Ala Glu Asp Val Ala Val Tyr Tyr Cys Gln Gln Ser Asn
                85                  90                  95
Lys Asp Pro Leu Thr Phe Gly Gly Gly Thr Lys Val Glu Ile Lys
            100                 105                 110


<210>    36
<211>    111
<212>    PRT
<213>    Artificial Sequence

<220>
<221>    DOMAIN
<223>    h011VL3


<400>    36
```

```
Asp Ile Val Met Thr Gln Ser Pro Asp Ser Leu Ala Val Ser Leu Gly
1               5                   10                  15
Glu Arg Ala Thr Ile Asn Cys Arg Ala Ser Glu Asn Val Asp Asn Tyr
            20                  25                  30
Gly Ile Ser Phe Met His Trp Tyr Gln Gln Lys Pro Gly Gln Pro Pro
            35                  40                  45
Lys Leu Leu Ile Tyr Arg Ala Ser Asn Leu Glu Ser Gly Ile Pro Asp
    50                  55                  60
Arg Phe Ser Gly Ser Gly Ser Arg Thr Asp Phe Thr Leu Thr Ile Ser
65                  70                  75                  80
Ser Leu Gln Ala Glu Asp Val Ala Thr Tyr Tyr Cys Gln Gln Ser Asn
                85                  90                  95
Lys Asp Pro Leu Thr Phe Gly Gly Gly Thr Lys Val Glu Ile Lys
            100                 105                 110
```

```
<210>  37
<211>  120
<212>  PRT
<213>  Artificial Sequence

<220>
<221>  DOMAIN
<223>  h160 VH1


<400>  37
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
Gly Met His Trp Val Arg Gln Ala Pro Gly Lys Gly Leu Glu Trp Val
            35                  40                  45
Ala Phe Ile Ser Thr Gly Ser Ser Asn Ile Tyr Tyr Val Asp Lys Val
    50                  55                  60
Lys Gly Arg Phe Thr Ile Ser Arg Asp Asn Ala Lys Asn Thr Leu Tyr
65                  70                  75                  80
Leu Gln Met Thr Ser Leu Arg Ala Glu Asp Thr Ala Val Tyr Tyr Cys
                85                  90                  95
Ala Arg Asn Tyr Val Ser Ser Tyr Gly Tyr Phe Asp Tyr Trp Gly Gln
            100                 105                 110
Gly Thr Thr Val Thr Val Ser Ser
            115                 120
```

```
<210>  38
<211>  111
<212>  PRT
<213>  Artificial Sequence

<220>
<221>  DOMAIN
<223>  h160 VL1


<400>  38
Asp Ile Val Met Thr Gln Ser Pro Ala Ser Leu Ala Val Ser Leu Gly
1               5                   10                  15
Glu Arg Ala Thr Ile Asn Cys Arg Ala Ser Glu Ser Val Asp Asn Tyr
            20                  25                  30
Gly Ile Ser Phe Met His Trp Tyr Gln Gln Lys Pro Gly Gln Pro Pro
            35                  40                  45
Lys Leu Leu Ile Tyr Arg Ala Ser Asn Leu Glu Ser Gly Val Pro Asp
    50                  55                  60
Arg Phe Ser Gly Ser Gly Ser Gly Thr Asp Phe Thr Leu Thr Ile Ser
```

```
                        65                      70                      75                      80
                        Ser Leu Gln Ala Glu Asp Val Ala Val Tyr Tyr Cys Gln Gln Thr Asn
                                        85                      90                      95
                        Lys Asp Pro Leu Thr Phe Gly Gly Gly Thr Lys Val Glu Ile Lys
                                        100                     105                     110


<210>   39
<211>   120
<212>   PRT
<213>   Artificial Sequence

<220>
<221>   DOMAIN
<223>   h160 VH2


<400>   39
Glu Val Gln Leu Val Glu Ser Gly Gly Gly Leu Val Gln Pro Gly Gly
1                   5                       10                      15
Ser Leu Arg Leu Ser Cys Ala Ala Ser Gly Phe Thr Phe Ser Asp Tyr
                20                      25                      30
Gly Met His Trp Val Arg Gln Ala Pro Gly Lys Gly Leu Glu Trp Ile
        35                      40                      45
Ala Phe Ile Ser Thr Gly Ser Ser Asn Ile Tyr Tyr Val Asp Lys Val
        50                      55                      60
Lys Gly Arg Phe Thr Ile Ser Arg Asp Asn Ala Lys Asn Thr Leu Tyr
65                      70                      75                      80
Leu Gln Met Thr Ser Leu Arg Ala Glu Asp Thr Ala Val Tyr Tyr Cys
                85                      90                      95
Ala Arg Asn Tyr Val Ser Ser Tyr Gly Tyr Phe Asp Tyr Trp Gly Gln
                100                     105                     110
Gly Thr Thr Val Thr Val Ser Ser
        115                     120

<210>   40
<211>   111
<212>   PRT
<213>   Artificial Sequence

<220>
<221>   DOMAIN
<223>   h160 VL2


<400>   40
Asp Ile Val Leu Thr Gln Ser Pro Ala Ser Leu Ala Val Ser Leu Gly
1                   5                       10                      15
Glu Arg Ala Thr Ile Asn Cys Arg Ala Ser Glu Ser Val Asp Asn Tyr
                20                      25                      30
Gly Ile Ser Phe Met His Trp Tyr Gln Gln Lys Pro Gly Gln Pro Pro
        35                      40                      45
Lys Leu Leu Ile Tyr Arg Ala Ser Asn Leu Glu Ser Gly Val Pro Asp
        50                      55                      60
Arg Phe Ser Gly Ser Gly Ser Gly Thr Asp Phe Thr Leu Thr Ile Ser
65                      70                      75                      80
Ser Leu Gln Ala Glu Asp Val Ala Val Tyr Tyr Cys Gln Gln Thr Asn
                85                      90                      95
Lys Asp Pro Leu Thr Phe Gly Gly Gly Thr Lys Val Glu Ile Lys
                100                     105                     110

<210>   41
<211>   111
<212>   PRT
```

<210> Artificial Sequence

<220>
<221> DOMAIN
<223> h160 VL3

<400> 41

```
Asp Ile Val Leu Thr Gln Ser Pro Ala Ser Leu Ala Val Ser Leu Gly
1               5                   10                  15
Glu Arg Ala Thr Ile Asn Cys Arg Ala Ser Glu Ser Val Asp Asn Tyr
            20                  25                  30
Gly Ile Ser Phe Met His Trp Tyr Gln Gln Lys Pro Gly Gln Pro Pro
            35                  40                  45
Lys Leu Leu Ile Tyr Arg Ala Ser Asn Leu Glu Ser Gly Val Pro Ala
        50                  55                  60
Arg Phe Ser Gly Ser Gly Ser Arg Thr Asp Phe Thr Leu Thr Ile Ser
65                  70                  75                  80
Ser Leu Gln Ala Glu Asp Val Ala Val Tyr Tyr Cys Gln Gln Thr Asn
                85                  90                  95
Lys Asp Pro Leu Thr Phe Gly Gly Gly Thr Lys Val Glu Ile Lys
            100                 105                 110
```

<210> 42
<211> 111
<212> PRT
<213> Artificial Sequence

<220>
<221> DOMAIN
<223> h160 VL4

<400> 42

```
Asp Ile Val Leu Thr Gln Ser Pro Ala Ser Leu Ala Val Ser Leu Gly
1               5                   10                  15
Glu Arg Ala Thr Ile Ser Cys Arg Ala Ser Glu Ser Val Asp Asn Tyr
            20                  25                  30
Gly Ile Ser Phe Met His Trp Tyr Gln Gln Lys Pro Gly Gln Pro Pro
            35                  40                  45
Lys Leu Leu Ile Tyr Arg Ala Ser Asn Leu Glu Ser Gly Ile Pro Ala
        50                  55                  60
Arg Phe Ser Gly Ser Gly Ser Arg Thr Asp Phe Thr Leu Thr Ile Ser
65                  70                  75                  80
Ser Leu Gln Ala Glu Asp Val Ala Val Tyr Tyr Cys Gln Gln Thr Asn
                85                  90                  95
Lys Asp Pro Leu Thr Phe Gly Gly Gly Thr Lys Val Glu Ile Lys
            100                 105                 110
```

<210> 43
<211> 330
<212> PRT
<213> Artificial Sequence

<220>
<221> DOMAIN
<223> IgG1 heavy chain constant region

<400> 43

```
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
```

```
                    20                      25                      30
    Phe Pro Glu Pro Val Thr Val Ser Trp Asn Ser Gly Ala Leu Thr Ser
            35                      40                      45
    Gly Val His Thr Phe Pro Ala Val Leu Gln Ser Ser Gly Leu Tyr Ser
        50                      55                      60
    Leu Ser Ser Val Val Thr Val Pro Ser Ser Ser Leu Gly Thr Gln Thr
    65                      70                      75                      80
    Tyr Ile Cys Asn Val Asn His Lys Pro Ser Asn Thr Lys Val Asp Lys
                    85                      90                      95
    Lys Val Glu Pro Lys Ser Cys Asp Lys Thr His Thr Cys Pro Pro Cys
                100                     105                     110
    Pro Ala Pro Glu Leu Leu Gly Gly Pro Ser Val Phe Leu Phe Pro Pro
            115                     120                     125
    Lys Pro Lys Asp Thr Leu Met Ile Ser Arg Thr Pro Glu Val Thr Cys
        130                     135                     140
    Val Val Val Asp Val Ser His Glu Asp Pro Glu Val Lys Phe Asn Trp
    145                     150                     155                     160
    Tyr Val Asp Gly Val Glu Val His Asn Ala Lys Thr Lys Pro Arg Glu
                    165                     170                     175
    Glu Gln Tyr Asn Ser Thr Tyr Arg Val Val Ser Val Leu Thr Val Leu
                180                     185                     190
    His Gln Asp Trp Leu Asn Gly Lys Glu Tyr Lys Cys Lys Val Ser Asn
                195                     200                     205
    Lys Ala Leu Pro Ala Pro Ile Glu Lys Thr Ile Ser Lys Ala Lys Gly
        210                     215                     220
    Gln Pro Arg Glu Pro Gln Val Tyr Thr Leu Pro Pro Ser Arg Asp Glu
    225                     230                     235                     240
    Leu Thr Lys Asn Gln Val Ser Leu Thr Cys Leu Val Lys Gly Phe Tyr
                    245                     250                     255
    Pro Ser Asp Ile Ala Val Glu Trp Glu Ser Asn Gly Gln Pro Glu Asn
                260                     265                     270
    Asn Tyr Lys Thr Thr Pro Pro Val Leu Asp Ser Asp Gly Ser Phe Phe
                275                     280                     285
    Leu Tyr Ser Lys Leu Thr Val Asp Lys Ser Arg Trp Gln Gln Gly Asn
        290                     295                     300
    Val Phe Ser Cys Ser Val Met His Glu Ala Leu His Asn His Tyr Thr
    305                     310                     315                     320
    Gln Lys Ser Leu Ser Leu Ser Pro Gly Lys
                    325                     330


    <210>   44
    <211>   330
    <212>   PRT
    <213>   Artificial Sequence

    <220>
    <221>   DOMAIN
    <223>   IgG1-E333A heavy chain constant region


    <400>   44
    Ala Ser Thr Lys Gly Pro Ser Val Phe Pro Leu Ala Pro Ser Ser Lys
    1                   5                       10                      15
    Ser Thr Ser Gly Gly Thr Ala Ala Leu Gly Cys Leu Val Lys Asp Tyr
                    20                      25                      30
    Phe Pro Glu Pro Val Thr Val Ser Trp Asn Ser Gly Ala Leu Thr Ser
            35                      40                      45
    Gly Val His Thr Phe Pro Ala Val Leu Gln Ser Ser Gly Leu Tyr Ser
        50                      55                      60
    Leu Ser Ser Val Val Thr Val Pro Ser Ser Ser Leu Gly Thr Gln Thr
    65                      70                      75                      80
    Tyr Ile Cys Asn Val Asn His Lys Pro Ser Asn Thr Lys Val Asp Lys
                    85                      90                      95
```

```
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
            115                 120                 125
Lys Pro Lys Asp Thr Leu Met Ile Ser Arg Thr Pro Glu Val Thr Cys
            130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                    165                 170                 175
Glu Gln Tyr Asn Ser Thr Tyr Arg Val Ser Val Leu Thr Val Leu
            180                 185                 190
His Gln Asp Trp Leu Asn Gly Lys Glu Tyr Lys Cys Lys Val Ser Asn
            195                 200                 205
Lys Ala Leu Pro Ala Pro Ile Ala Lys Thr Ile Ser Lys Ala Lys Gly
    210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                    245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
            275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                    325                 330
```

```
<210>   45
<211>   107
<212>   PRT
<213>   Artificial Sequence

<220>
<221>   DOMAIN
<223>   kappa light chain constant region


<400>   45
Arg Thr Val Ala Ala Pro Ser Val Phe Ile Phe Pro Pro Ser Asp Glu
1                   5                   10                  15
Gln Leu Lys Ser Gly Thr Ala Ser Val Val Cys Leu Leu Asn Asn Phe
            20                  25                  30
Tyr Pro Arg Glu Ala Lys Val Gln Trp Lys Val Asp Asn Ala Leu Gln
            35                  40                  45
Ser Gly Asn Ser Gln Glu Ser Val Thr Glu Gln Asp Ser Lys Asp Ser
            50                  55                  60
Thr Tyr Ser Leu Ser Ser Thr Leu Thr Leu Ser Lys Ala Asp Tyr Glu
65                  70                  75                  80
Lys His Lys Val Tyr Ala Cys Glu Val Thr His Gln Gly Leu Ser Ser
                    85                  90                  95
Pro Val Thr Lys Ser Phe Asn Arg Gly Glu Cys
                    100                 105

<210>   46
<211>   448
<212>   PRT
<213>   Artificial Sequence

<220>
```

```
<221>   CHAIN
<223>   h009-07 antibody heavy chain


<400>   46
Glu Phe Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Asn Leu Asn Trp Val Arg Gln Ala Pro Gly Gln Ser Leu Glu Trp Met
        35                  40                  45
Gly Val Ile Asn Pro Lys Tyr Asp Ala Ile Asn Tyr Asn Gln Lys Phe
    50                  55                  60
Lys Asp Arg Val Thr Ile Thr Val Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Glu Gly Trp Gly Lys Ala Leu Asp Tyr Trp Gly Gln Gly Thr
            100                 105                 110
Leu Val Thr Val Ser Ser Ala Ser Thr Lys Gly Pro Ser Val Phe Pro
        115                 120                 125
Leu Ala Pro Ser Ser Lys Ser Thr Ser Gly Gly Thr Ala Ala Leu Gly
    130                 135                 140
Cys Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu Tyr Ser Leu Ser Ser Val Val Thr Val Pro Ser Ser
            180                 185                 190
Ser Leu Gly Thr Gln Thr Tyr Ile Cys Asn Val Asn His Lys Pro Ser
            195                 200                 205
Asn Thr Lys Val Asp Lys Lys Val Glu Pro Lys Ser Cys Asp Lys Thr
    210                 215                 220
His Thr Cys Pro Pro Cys Pro Ala Pro Glu Leu Leu Gly Gly Pro Ser
225                 230                 235                 240
Val Phe Leu Phe Pro Pro Lys Pro Lys Asp Thr Leu Met Ile Ser Arg
                245                 250                 255
Thr Pro Glu Val Thr Cys Val Val Val Asp Val Ser His Glu Asp Pro
            260                 265                 270
Glu Val Lys Phe Asn Trp Tyr Val Asp Gly Val Glu Val His Asn Ala
            275                 280                 285
Lys Thr Lys Pro Arg Glu Glu Gln Tyr Asn Ser Thr Tyr Arg Val Val
    290                 295                 300
Ser Val Leu Thr Val Leu His Gln Asp Trp Leu Asn Gly Lys Glu Tyr
305                 310                 315                 320
Lys Cys Lys Val Ser Asn Lys Ala Leu Pro Ala Pro Ile Glu Lys Thr
                325                 330                 335
Ile Ser Lys Ala Lys Gly Gln Pro Arg Glu Pro Gln Val Tyr Thr Leu
            340                 345                 350
Pro Pro Ser Arg Asp Glu Leu Thr Lys Asn Gln Val Ser Leu Thr Cys
    355                 360                 365
Leu Val Lys Gly Phe Tyr Pro Ser Asp Ile Ala Val Glu Trp Glu Ser
    370                 375                 380
Asn Gly Gln Pro Glu Asn Asn Tyr Lys Thr Thr Pro Pro Val Leu Asp
385                 390                 395                 400
Ser Asp Gly Ser Phe Phe Leu Tyr Ser Lys Leu Thr Val Asp Lys Ser
                405                 410                 415
Arg Trp Gln Gln Gly Asn Val Phe Ser Cys Ser Val Met His Glu Ala
            420                 425                 430
Leu His Asn His Tyr Thr Gln Lys Ser Leu Ser Leu Ser Pro Gly Lys
        435                 440                 445


<210>   47
```

```
<211>   214
<212>   PRT
<213>   Artificial Sequence

<220>
<221>   CHAIN
<223>   h009-07/hu9E antibody light chain


<400>   47
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Ser Ile Tyr Thr Asn
            20                  25                  30
Leu His Trp Tyr Gln Gln Lys Pro Gly Gln Ser Pro Arg Leu Leu Ile
        35                  40                  45
Lys Tyr Ala Ser Gln Ser Ile Ser Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys Gln Gln Ser Asn Ser Trp Pro Leu
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
            115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
            165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
            195                 200                 205
Phe Asn Arg Gly Glu Cys
            210

<210>   48
<211>   448
<212>   PRT
<213>   Artificial Sequence

<220>
<221>   CHAIN
<223>   hu9E antibody heavy chain

<400>   48
Glu Phe Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Asn Leu Asn Trp Val Arg Gln Ala Pro Gly Gln Ser Leu Glu Trp Met
        35                  40                  45
Gly Val Ile Asn Pro Lys Tyr Asp Ala Ile Asn Tyr Asn Gln Lys Phe
    50                  55                  60
Lys Asp Arg Val Thr Ile Thr Val Asp Thr Ser Ala Ser Thr Ala Tyr
65                  70                  75                  80
Met Glu Leu Ser Ser Leu Arg Ser Glu Asp Thr Ala Val Tyr Tyr Cys
            85                  90                  95
Ala Arg Glu Gly Trp Gly Lys Ala Leu Asp Tyr Trp Gly Gln Gly Thr
            100                 105                 110
```

```
Leu Val Thr Val Ser Ser Ala Ser Thr Lys Gly Pro Ser Val Phe Pro
        115                 120                 125
Leu Ala Pro Ser Ser Lys Ser Thr Ser Gly Gly Thr Ala Ala Leu Gly
        130                 135                 140
Cys Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu Tyr Ser Leu Ser Ser Val Val Thr Val Pro Ser Ser
            180                 185                 190
Ser Leu Gly Thr Gln Thr Tyr Ile Cys Asn Val Asn His Lys Pro Ser
            195                 200                 205
Asn Thr Lys Val Asp Lys Lys Val Glu Pro Lys Ser Cys Asp Lys Thr
        210                 215                 220
His Thr Cys Pro Pro Cys Pro Ala Pro Glu Leu Leu Gly Gly Pro Ser
225                 230                 235                 240
Val Phe Leu Phe Pro Pro Lys Pro Lys Asp Thr Leu Met Ile Ser Arg
                245                 250                 255
Thr Pro Glu Val Thr Cys Val Val Val Asp Val Ser His Glu Asp Pro
            260                 265                 270
Glu Val Lys Phe Asn Trp Tyr Val Asp Gly Val Glu Val His Asn Ala
            275                 280                 285
Lys Thr Lys Pro Arg Glu Glu Gln Tyr Asn Ser Thr Tyr Arg Val Val
        290                 295                 300
Ser Val Leu Thr Val Leu His Gln Asp Trp Leu Asn Gly Lys Glu Tyr
305                 310                 315                 320
Lys Cys Lys Val Ser Asn Lys Ala Leu Pro Ala Pro Ile Ala Lys Thr
                325                 330                 335
Ile Ser Lys Ala Lys Gly Gln Pro Arg Glu Pro Gln Val Tyr Thr Leu
            340                 345                 350
Pro Pro Ser Arg Asp Glu Leu Thr Lys Asn Gln Val Ser Leu Thr Cys
            355                 360                 365
Leu Val Lys Gly Phe Tyr Pro Ser Asp Ile Ala Val Glu Trp Glu Ser
        370                 375                 380
Asn Gly Gln Pro Glu Asn Asn Tyr Lys Thr Thr Pro Pro Val Leu Asp
385                 390                 395                 400
Ser Asp Gly Ser Phe Phe Leu Tyr Ser Lys Leu Thr Val Asp Lys Ser
                405                 410                 415
Arg Trp Gln Gln Gly Asn Val Phe Ser Cys Ser Val Met His Glu Ala
            420                 425                 430
Leu His Asn His Tyr Thr Gln Lys Ser Leu Ser Leu Ser Pro Gly Lys
        435                 440                 445
```

```
<210>  49
<211>  450
<212>  PRT
<213>  Artificial Sequence

<220>
<221>  CHAIN
<223>  h011-01 antibody heavy chain


<400>  49
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
Gly Met His Trp Val Arg Gln Ala Pro Gly Lys Gly Leu Glu Trp Val
        35                  40                  45
Ala Phe Ile Ser Ser Gly Ser Ser Ile Tyr Tyr Ala Asp Thr Val
        50                  55                  60
Lys Gly Arg Phe Thr Ile Ser Arg Asp Asn Ala Lys Ser Ser Leu Tyr
```

```
                        65                      70                      75                      80
        Leu Gln Met Thr Ser Leu Arg Ala Glu Asp Thr Ala Val Tyr Tyr Cys
                            85                      90                      95
        Ala Arg Asn Tyr Val Ser Ser Tyr Gly Tyr Phe Asp Tyr Trp Gly Gln
                        100                     105                     110
        Gly Thr Thr Val Thr Val Ser Ser Ala Ser Thr Lys Gly Pro Ser Val
                    115                     120                     125
        Phe Pro Leu Ala Pro Ser Ser Lys Ser Thr Ser Gly Gly Thr Ala Ala
            130                     135                     140
        Leu Gly Cys Leu Val Lys Asp Tyr Phe Pro Glu Pro Val Thr Val Ser
        145                     150                     155                     160
        Trp Asn Ser Gly Ala Leu Thr Ser Gly Val His Thr Phe Pro Ala Val
                        165                     170                     175
        Leu Gln Ser Ser Gly Leu Tyr Ser Leu Ser Ser Val Val Thr Val Pro
                    180                     185                     190
        Ser Ser Ser Leu Gly Thr Gln Thr Tyr Ile Cys Asn Val Asn His Lys
                195                     200                     205
        Pro Ser Asn Thr Lys Val Asp Lys Lys Val Glu Pro Lys Ser Cys Asp
            210                     215                     220
        Lys Thr His Thr Cys Pro Pro Cys Pro Ala Pro Glu Leu Leu Gly Gly
        225                     230                     235                     240
        Pro Ser Val Phe Leu Phe Pro Pro Lys Pro Lys Asp Thr Leu Met Ile
                        245                     250                     255
        Ser Arg Thr Pro Glu Val Thr Cys Val Val Asp Val Ser His Glu
                    260                     265                     270
        Asp Pro Glu Val Lys Phe Asn Trp Tyr Val Asp Gly Val Glu Val His
                275                     280                     285
        Asn Ala Lys Thr Lys Pro Arg Glu Glu Gln Tyr Asn Ser Thr Tyr Arg
            290                     295                     300
        Val Val Ser Val Leu Thr Val Leu His Gln Asp Trp Leu Asn Gly Lys
        305                     310                     315                     320
        Glu Tyr Lys Cys Lys Val Ser Asn Lys Ala Leu Pro Ala Pro Ile Glu
                        325                     330                     335
        Lys Thr Ile Ser Lys Ala Lys Gly Gln Pro Arg Glu Pro Gln Val Tyr
                    340                     345                     350
        Thr Leu Pro Pro Ser Arg Asp Glu Leu Thr Lys Asn Gln Val Ser Leu
                355                     360                     365
        Thr Cys Leu Val Lys Gly Phe Tyr Pro Ser Asp Ile Ala Val Glu Trp
            370                     375                     380
        Glu Ser Asn Gly Gln Pro Glu Asn Asn Tyr Lys Thr Thr Pro Pro Val
        385                     390                     395                     400
        Leu Asp Ser Asp Gly Ser Phe Phe Leu Tyr Ser Lys Leu Thr Val Asp
                    405                     410                     415
        Lys Ser Arg Trp Gln Gln Gly Asn Val Phe Ser Cys Ser Val Met His
                    420                     425                     430
        Glu Ala Leu His Asn His Tyr Thr Gln Lys Ser Leu Ser Leu Ser Pro
                    435                     440                     445
        Gly Lys
            450
```

<210> 50
<211> 218
<212> PRT
<213> Artificial Sequence

<220>
<221> CHAIN
<223> h011-01/hu11E antibody light chain


<400> 50

```
        Asp Ile Val Met Thr Gln Ser Pro Asp Ser Leu Ala Val Ser Leu Gly
        1                   5                   10                      15
```

```
Glu Arg Ala Thr Ile Asn Cys Arg Ala Ser Glu Asn Val Asp Asn Tyr
            20                  25                  30
Gly Ile Ser Phe Met His Trp Tyr Gln Gln Lys Pro Gly Gln Pro Pro
            35                  40                  45
Lys Leu Leu Ile Tyr Arg Ala Ser Asn Leu Glu Ser Gly Val Pro Asp
            50                  55                  60
Arg Phe Ser Gly Ser Gly Ser Gly Thr Asp Phe Thr Leu Thr Ile Ser
65                  70                  75                  80
Ser Leu Gln Ala Glu Asp Val Ala Val Tyr Tyr Cys Gln Gln Ser Asn
                85                  90                  95
Lys Asp Pro Leu Thr Phe Gly Gly Gly Thr Lys Val Glu Ile Lys Arg
            100                 105                 110
Thr Val Ala Ala Pro Ser Val Phe Ile Phe Pro Pro Ser Asp Glu Gln
            115                 120                 125
Leu Lys Ser Gly Thr Ala Ser Val Val Cys Leu Leu Asn Asn Phe Tyr
            130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
            195                 200                 205
Val Thr Lys Ser Phe Asn Arg Gly Glu Cys
            210                 215
```

```
<210>  51
<211>  450
<212>  PRT
<213>  Artificial Sequence

<220>
<221>  CHAIN
<223>  hu11E antibody heavy chain


<400>  51
Glu Val Gln Leu Val Glu Ser Gly Gly Gly Leu Val Gln Pro Gly Gly
1                   5                   10                  15
Ser Leu Arg Leu Ser Cys Ala Ala Ser Gly Phe Thr Phe Ser Asp Tyr
            20                  25                  30
Gly Met His Trp Val Arg Gln Ala Pro Gly Lys Gly Leu Glu Trp Val
            35                  40                  45
Ala Phe Ile Ser Ser Gly Ser Ser Ile Tyr Tyr Ala Asp Thr Val
            50                  55                  60
Lys Gly Arg Phe Thr Ile Ser Arg Asp Asn Ala Lys Ser Ser Leu Tyr
65                  70                  75                  80
Leu Gln Met Thr Ser Leu Arg Ala Glu Asp Thr Ala Val Tyr Tyr Cys
                85                  90                  95
Ala Arg Asn Tyr Val Ser Ser Tyr Gly Tyr Phe Asp Tyr Trp Gly Gln
            100                 105                 110
Gly Thr Thr Val Thr Val Ser Ser Ala Ser Thr Lys Gly Pro Ser Val
            115                 120                 125
Phe Pro Leu Ala Pro Ser Ser Lys Ser Thr Ser Gly Gly Thr Ala Ala
            130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
```

```
              195                    200                    205
        Pro Ser Asn Thr Lys Val Asp Lys Lys Val Glu Pro Lys Ser Cys Asp
            210                    215                    220
        Lys Thr His Thr Cys Pro Pro Cys Pro Ala Pro Glu Leu Leu Gly Gly
        225                    230                    235                    240
        Pro Ser Val Phe Leu Phe Pro Pro Lys Pro Lys Asp Thr Leu Met Ile
                           245                    250                    255
        Ser Arg Thr Pro Glu Val Thr Cys Val Val Val Asp Val Ser His Glu
                       260                    265                    270
        Asp Pro Glu Val Lys Phe Asn Trp Tyr Val Asp Gly Val Glu Val His
                   275                    280                    285
        Asn Ala Lys Thr Lys Pro Arg Glu Glu Gln Tyr Asn Ser Thr Tyr Arg
                290                    295                    300
        Val Val Ser Val Leu Thr Val Leu His Gln Asp Trp Leu Asn Gly Lys
        305                    310                    315                    320
        Glu Tyr Lys Cys Lys Val Ser Asn Lys Ala Leu Pro Ala Pro Ile Ala
                           325                    330                    335
        Lys Thr Ile Ser Lys Ala Lys Gly Gln Pro Arg Glu Pro Gln Val Tyr
                       340                    345                    350
        Thr Leu Pro Pro Ser Arg Asp Glu Leu Thr Lys Asn Gln Val Ser Leu
                       355                    360                    365
        Thr Cys Leu Val Lys Gly Phe Tyr Pro Ser Asp Ile Ala Val Glu Trp
                       370                    375                    380
        Glu Ser Asn Gly Gln Pro Glu Asn Asn Tyr Lys Thr Thr Pro Pro Val
        385                    390                    395                    400
        Leu Asp Ser Asp Gly Ser Phe Phe Leu Tyr Ser Lys Leu Thr Val Asp
                           405                    410                    415
        Lys Ser Arg Trp Gln Gln Gly Asn Val Phe Ser Cys Ser Val Met His
                       420                    425                    430
        Glu Ala Leu His Asn His Tyr Thr Gln Lys Ser Leu Ser Leu Ser Pro
                       435                    440                    445
        Gly Lys
            450


        <210>  52
        <211>  450
        <212>  PRT
        <213>  Artificial Sequence

        <220>
        <221>  CHAIN
        <223>  h160-01 antibody heavy chain


        <400>  52
        Glu Val Gln Leu Val Glu Ser Gly Gly Gly Leu Val Gln Pro Gly Gly
        1                   5                    10                     15
        Ser Leu Arg Leu Ser Cys Ala Ala Ser Gly Phe Thr Phe Ser Asp Tyr
                       20                    25                     30
        Gly Met His Trp Val Arg Gln Ala Pro Gly Lys Gly Leu Glu Trp Val
                   35                    40                     45
        Ala Phe Ile Ser Thr Gly Ser Ser Asn Ile Tyr Tyr Val Asp Lys Val
            50                    55                     60
        Lys Gly Arg Phe Thr Ile Ser Arg Asp Asn Ala Lys Asn Thr Leu Tyr
        65                    70                     75                     80
        Leu Gln Met Thr Ser Leu Arg Ala Glu Asp Thr Ala Val Tyr Tyr Cys
                           85                    90                     95
        Ala Arg Asn Tyr Val Ser Ser Tyr Gly Tyr Phe Asp Tyr Trp Gly Gln
                       100                   105                    110
        Gly Thr Thr Val Thr Val Ser Ser Ala Ser Thr Lys Gly Pro Ser Val
                   115                   120                    125
        Phe Pro Leu Ala Pro Ser Ser Lys Ser Thr Ser Gly Gly Thr Ala Ala
            130                   135                    140
```

```
Leu Gly Cys Leu Val Lys Asp Tyr Phe Pro Glu Pro Val Thr Val Ser
145             150             155             160
Trp Asn Ser Gly Ala Leu Thr Ser Gly Val His Thr Phe Pro Ala Val
            165             170             175
Leu Gln Ser Ser Gly Leu Tyr Ser Leu Ser Ser Val Val Thr Val Pro
        180             185             190
Ser Ser Ser Leu Gly Thr Gln Thr Tyr Ile Cys Asn Val Asn His Lys
        195             200             205
Pro Ser Asn Thr Lys Val Asp Lys Lys Val Glu Pro Lys Ser Cys Asp
        210             215             220
Lys Thr His Thr Cys Pro Pro Cys Pro Ala Pro Glu Leu Leu Gly Gly
225             230             235             240
Pro Ser Val Phe Leu Phe Pro Pro Lys Pro Lys Asp Thr Leu Met Ile
            245             250             255
Ser Arg Thr Pro Glu Val Thr Cys Val Val Val Asp Val Ser His Glu
            260             265             270
Asp Pro Glu Val Lys Phe Asn Trp Tyr Val Asp Gly Val Glu Val His
        275             280             285
Asn Ala Lys Thr Lys Pro Arg Glu Glu Gln Tyr Asn Ser Thr Tyr Arg
        290             295             300
Val Val Ser Val Leu Thr Val Leu His Gln Asp Trp Leu Asn Gly Lys
305             310             315             320
Glu Tyr Lys Cys Lys Val Ser Asn Lys Ala Leu Pro Ala Pro Ile Glu
            325             330             335
Lys Thr Ile Ser Lys Ala Lys Gly Gln Pro Arg Glu Pro Gln Val Tyr
            340             345             350
Thr Leu Pro Pro Ser Arg Asp Glu Leu Thr Lys Asn Gln Val Ser Leu
        355             360             365
Thr Cys Leu Val Lys Gly Phe Tyr Pro Ser Asp Ile Ala Val Glu Trp
    370             375             380
Glu Ser Asn Gly Gln Pro Glu Asn Asn Tyr Lys Thr Thr Pro Pro Val
385             390             395             400
Leu Asp Ser Asp Gly Ser Phe Phe Leu Tyr Ser Lys Leu Thr Val Asp
            405             410             415
Lys Ser Arg Trp Gln Gln Gly Asn Val Phe Ser Cys Ser Val Met His
            420             425             430
Glu Ala Leu His Asn His Tyr Thr Gln Lys Ser Leu Ser Leu Ser Pro
        435             440             445
Gly Lys
450
```

```
<210>   53
<211>   218
<212>   PRT
<213>   Artificial Sequence

<220>
<221>   CHAIN
<223>   h160-01/hu160E antibody light chain


<400>   53
Asp Ile Val Met Thr Gln Ser Pro Ala Ser Leu Ala Val Ser Leu Gly
1               5               10              15
Glu Arg Ala Thr Ile Asn Cys Arg Ala Ser Glu Ser Val Asp Asn Tyr
            20              25              30
Gly Ile Ser Phe Met His Trp Tyr Gln Gln Lys Pro Gly Gln Pro Pro
        35              40              45
Lys Leu Leu Ile Tyr Arg Ala Ser Asn Leu Glu Ser Gly Val Pro Asp
        50              55              60
Arg Phe Ser Gly Ser Gly Ser Gly Thr Asp Phe Thr Leu Thr Ile Ser
65              70              75              80
Ser Leu Gln Ala Glu Asp Val Ala Val Tyr Tyr Cys Gln Gln Thr Asn
```

```
                        85                      90                      95
        Lys Asp Pro Leu Thr Phe Gly Gly Gly Thr Lys Val Glu Ile Lys Arg
                    100                     105                     110
        Thr Val Ala Ala Pro Ser Val Phe Ile Phe Pro Pro Ser Asp Glu Gln
                    115                     120                     125
        Leu Lys Ser Gly Thr Ala Ser Val Val Cys Leu Leu Asn Asn Phe Tyr
            130                     135                     140
        Pro Arg Glu Ala Lys Val Gln Trp Lys Val Asp Asn Ala Leu Gln Ser
        145                     150                     155                     160
        Gly Asn Ser Gln Glu Ser Val Thr Glu Gln Asp Ser Lys Asp Ser Thr
                        165                     170                     175
        Tyr Ser Leu Ser Ser Thr Leu Thr Leu Ser Lys Ala Asp Tyr Glu Lys
                    180                     185                     190
        His Lys Val Tyr Ala Cys Glu Val Thr His Gln Gly Leu Ser Ser Pro
                    195                     200                     205
        Val Thr Lys Ser Phe Asn Arg Gly Glu Cys
            210                     215

        <210>   54
        <211>   450
        <212>   PRT
        <213>   Artificial Sequence

        <220>
        <221>   CHAIN
        <223>   hu160E antibody heavy chain


        <400>   54
        Glu Val Gln Leu Val Glu Ser Gly Gly Gly Leu Val Gln Pro Gly Gly
        1                   5                       10                      15
        Ser Leu Arg Leu Ser Cys Ala Ala Ser Gly Phe Thr Phe Ser Asp Tyr
                    20                      25                      30
        Gly Met His Trp Val Arg Gln Ala Pro Gly Lys Gly Leu Glu Trp Val
                35                      40                      45
        Ala Phe Ile Ser Thr Gly Ser Ser Asn Ile Tyr Tyr Val Asp Lys Val
            50                      55                      60
        Lys Gly Arg Phe Thr Ile Ser Arg Asp Asn Ala Lys Asn Thr Leu Tyr
        65                      70                      75                      80
        Leu Gln Met Thr Ser Leu Arg Ala Glu Asp Thr Ala Val Tyr Tyr Cys
                        85                      90                      95
        Ala Arg Asn Tyr Val Ser Ser Tyr Gly Tyr Phe Asp Tyr Trp Gly Gln
                    100                     105                     110
        Gly Thr Thr Val Thr Val Ser Ser Ala Ser Thr Lys Gly Pro Ser Val
                    115                     120                     125
        Phe Pro Leu Ala Pro Ser Ser Lys Ser Thr Ser Gly Gly Thr Ala Ala
            130                     135                     140
        Leu Gly Cys Leu Val Lys Asp Tyr Phe Pro Glu Pro Val Thr Val Ser
        145                     150                     155                     160
        Trp Asn Ser Gly Ala Leu Thr Ser Gly Val His Thr Phe Pro Ala Val
                        165                     170                     175
        Leu Gln Ser Ser Gly Leu Tyr Ser Leu Ser Ser Val Val Thr Val Pro
                    180                     185                     190
        Ser Ser Ser Leu Gly Thr Gln Thr Tyr Ile Cys Asn Val Asn His Lys
                    195                     200                     205
        Pro Ser Asn Thr Lys Val Asp Lys Lys Val Glu Pro Lys Ser Cys Asp
            210                     215                     220
        Lys Thr His Thr Cys Pro Pro Cys Pro Ala Pro Glu Leu Leu Gly Gly
        225                     230                     235                     240
        Pro Ser Val Phe Leu Phe Pro Pro Lys Pro Lys Asp Thr Leu Met Ile
                        245                     250                     255
        Ser Arg Thr Pro Glu Val Thr Cys Val Val Val Asp Val Ser His Glu
                    260                     265                     270
```

```
Asp Pro Glu Val Lys Phe Asn Trp Tyr Val Asp Gly Val Glu Val His
    275                     280                 285
Asn Ala Lys Thr Lys Pro Arg Glu Glu Gln Tyr Asn Ser Thr Tyr Arg
    290                     295                 300
Val Val Ser Val Leu Thr Val Leu His Gln Asp Trp Leu Asn Gly Lys
305                     310                 315                 320
Glu Tyr Lys Cys Lys Val Ser Asn Lys Ala Leu Pro Ala Pro Ile Ala
                325                 330                 335
Lys Thr Ile Ser Lys Ala Lys Gly Gln Pro Arg Glu Pro Gln Val Tyr
                340                 345                 350
Thr Leu Pro Pro Ser Arg Asp Glu Leu Thr Lys Asn Gln Val Ser Leu
            355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450


<210>    55
<211>    452
<212>    PRT
<213>    Artificial Sequence


<220>
<221>    CHAIN
<223>    Dara heavy chain sequence


<400>    55
Glu Val Gln Leu Leu Glu Ser Gly Gly Gly Leu Val Gln Pro Gly Gly
1               5                   10                  15
Ser Leu Arg Leu Ser Cys Ala Val Ser Gly Phe Thr Phe Asn Ser Phe
            20                  25                  30
Ala Met Ser Trp Val Arg Gln Ala Pro Gly Lys Gly Leu Glu Trp Val
        35                  40                  45
Ser Ala Ile Ser Gly Ser Gly Gly Gly Thr Tyr Tyr Ala Asp Ser Val
        50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Phe Cys
                85                  90                  95
Ala Lys Asp Lys Ile Leu Trp Phe Gly Glu Pro Val Phe Asp Tyr Trp
            100                 105                 110
Gly Gln Gly Thr Leu Val Thr Val Ser Ser Ala Ser Thr Lys Gly Pro
        115                 120                 125
Ser Val Phe Pro Leu Ala Pro Ser Ser Lys Ser Thr Ser Gly Gly Thr
    130                 135                 140
Ala Ala Leu Gly Cys Leu Val Lys Asp Tyr Phe Pro Glu Pro Val Thr
145                 150                 155                 160
Val Ser Trp Asn Ser Gly Ala Leu Thr Ser Gly Val His Thr Phe Pro
                165                 170                 175
Ala Val Leu Gln Ser Ser Gly Leu Tyr Ser Leu Ser Ser Val Val Thr
            180                 185                 190
Val Pro Ser Ser Ser Leu Gly Thr Gln Thr Tyr Ile Cys Asn Val Asn
        195                 200                 205
His Lys Pro Ser Asn Thr Lys Val Asp Lys Arg Val Glu Pro Lys Ser
```

```
          210                     215                     220
Cys Asp Lys Thr His Thr Cys Pro Pro Cys Pro Ala Pro Glu Leu Leu
225                     230                     235                     240
Gly Gly Pro Ser Val Phe Leu Phe Pro Pro Lys Pro Lys Asp Thr Leu
                    245                     250                     255
Met Ile Ser Arg Thr Pro Glu Val Thr Cys Val Val Val Asp Val Ser
                260                     265                     270
His Glu Asp Pro Glu Val Lys Phe Asn Trp Tyr Val Asp Gly Val Glu
            275                     280                     285
Val His Asn Ala Lys Thr Lys Pro Arg Glu Glu Gln Tyr Asn Ser Thr
    290                     295                     300
Tyr Arg Val Val Ser Val Leu Thr Val Leu His Gln Asp Trp Leu Asn
305                     310                     315                     320
Gly Lys Glu Tyr Lys Cys Lys Val Ser Asn Lys Ala Leu Pro Ala Pro
                325                     330                     335
Ile Glu Lys Thr Ile Ser Lys Ala Lys Gly Gln Pro Arg Glu Pro Gln
            340                     345                     350
Val Tyr Thr Leu Pro Pro Ser Arg Glu Glu Met Thr Lys Asn Gln Val
        355                     360                     365
Ser Leu Thr Cys Leu Val Lys Gly Phe Tyr Pro Ser Asp Ile Ala Val
    370                     375                     380
Glu Trp Glu Ser Asn Gly Gln Pro Glu Asn Asn Tyr Lys Thr Thr Pro
385                     390                     395                     400
Pro Val Leu Asp Ser Asp Gly Ser Phe Phe Leu Tyr Ser Lys Leu Thr
                405                     410                     415
Val Asp Lys Ser Arg Trp Gln Gln Gly Asn Val Phe Ser Cys Ser Val
            420                     425                     430
Met His Glu Ala Leu His Asn His Tyr Thr Gln Lys Ser Leu Ser Leu
        435                     440                     445
Ser Pro Gly Lys
        450


<210>   56
<211>   214
<212>   PRT
<213>   Artificial Sequence

<220>
<221>   CHAIN
<223>   Dara light chain sequence

<400>   56
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Ser Val Ser Ser Tyr
                20                  25                  30
Leu Ala Trp Tyr Gln Gln Lys Pro Gly Gln Ala Pro Arg Leu Leu Ile
            35                  40                  45
Tyr Asp Ala Ser Asn Arg Ala Thr Gly Ile Pro Ala Arg Phe Ser Gly
        50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys Gln Gln Arg Ser Asn Trp Pro Pro
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
        130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
```

```
                        165                 170                 175
        Ser Thr Leu Thr Leu Ser Lys Ala Asp Tyr Glu Lys His Lys Val Tyr
                    180                 185                 190
        Ala Cys Glu Val Thr His Gln Gly Leu Ser Ser Pro Val Thr Lys Ser
                    195                 200                 205
        Phe Asn Arg Gly Glu Cys
                    210


        <210>   57
        <211>   264
        <212>   PRT
        <213>   Artificial Sequence


        <220>
        <221>   PEPTIDE
        <223>   Fusion protein of CD38 extracellular domain with His tag:
         CD38-ECD-His


        <400>   57
        Val Pro Arg Trp Arg Gln Gln Trp Ser Gly Pro Gly Thr Thr Lys Arg
        1               5                   10                  15
        Phe Pro Glu Thr Val Leu Ala Arg Cys Val Lys Tyr Thr Glu Ile His
                    20                  25                  30
        Pro Glu Met Arg His Val Asp Cys Gln Ser Val Trp Asp Ala Phe Lys
                    35                  40                  45
        Gly Ala Phe Ile Ser Lys His Pro Cys Asn Ile Thr Glu Glu Asp Tyr
                    50                  55                  60
        Gln Pro Leu Met Lys Leu Gly Thr Gln Thr Val Pro Cys Asn Lys Ile
        65                  70                  75                  80
        Leu Leu Trp Ser Arg Ile Lys Asp Leu Ala His Gln Phe Thr Gln Val
                        85                  90                  95
        Gln Arg Asp Met Phe Thr Leu Glu Asp Thr Leu Leu Gly Tyr Leu Ala
                    100                 105                 110
        Asp Asp Leu Thr Trp Cys Gly Glu Phe Asn Thr Ser Lys Ile Asn Tyr
                    115                 120                 125
        Gln Ser Cys Pro Asp Trp Arg Lys Asp Cys Ser Asn Asn Pro Val Ser
            130                 135                 140
        Val Phe Trp Lys Thr Val Ser Arg Arg Phe Ala Glu Ala Ala Cys Asp
        145                 150                 155                 160
        Val Val His Val Met Leu Asn Gly Ser Arg Ser Lys Ile Phe Asp Lys
                        165                 170                 175
        Asn Ser Thr Phe Gly Ser Val Glu Val His Asn Leu Gln Pro Glu Lys
                    180                 185                 190
        Val Gln Thr Leu Glu Ala Trp Val Ile His Gly Gly Arg Glu Asp Ser
                    195                 200                 205
        Arg Asp Leu Cys Gln Asp Pro Thr Ile Lys Glu Leu Glu Ser Ile Ile
            210                 215                 220
        Ser Lys Arg Asn Ile Gln Phe Ser Cys Lys Asn Ile Tyr Arg Pro Asp
        225                 230                 235                 240
        Lys Phe Leu Gln Cys Val Lys Asn Pro Glu Asp Ser Ser Cys Thr Ser
                        245                 250                 255
        Glu Ile His His His His His His
                    260
```